(19) Europäisches Patentamt
European Patent Office
Office européen des brevets

(11) EP 4 389 752 A1

(12) EUROPEAN PATENT APPLICATION
published in accordance with Art. 153(4) EPC

(43) Date of publication:
**26.06.2024 Bulletin 2024/26**

(21) Application number: **22857914.0**

(22) Date of filing: **19.08.2022**

(51) International Patent Classification (IPC):
***C07D 491/147*** *(2006.01)* ***C07D 491/22*** *(2006.01)*
***A61K 31/436*** *(2006.01)* ***A61P 35/00*** *(2006.01)*

(52) Cooperative Patent Classification (CPC):
**A61K 31/436; A61P 35/00; C07D 491/147; C07D 491/22**

(86) International application number:
**PCT/CN2022/113499**

(87) International publication number:
**WO 2023/020605 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.08.2021 CN 202110955364**
**13.12.2021 CN 202111515247**
**12.05.2022 CN 202210515797**

(71) Applicant: **Simcere Zaiming Pharmaceutical Co., Ltd.**
**Hainan 570311 (CN)**

(72) Inventors:
- **LI, Zhen**
  **Shanghai 201318 (CN)**
- **TANG, Feng**
  **Shanghai 201318 (CN)**
- **FU, Yayuan**
  **Shanghai 201318 (CN)**
- **LIU, Lifeng**
  **Shanghai 201318 (CN)**
- **ZHAO, Chunyan**
  **Shanghai 201318 (CN)**
- **TANG, Renhong**
  **Shanghai 201318 (CN)**
- **REN, Jinsheng**
  **Nanjing, Jiangsu 210042 (CN)**

(74) Representative: **V.O.**
**P.O. Box 87930**
**2508 DH Den Haag (NL)**

(54) **CAMPTOTHECIN DERIVATIVE, AND PHARMACEUTICAL COMPOSITION AND USE THEREOF**

(57) Provided are a compound of formula (I), or a stereoisomer or pharmaceutically acceptable salt thereof, a pharmaceutical composition thereof, and the anti-tumor use thereof.

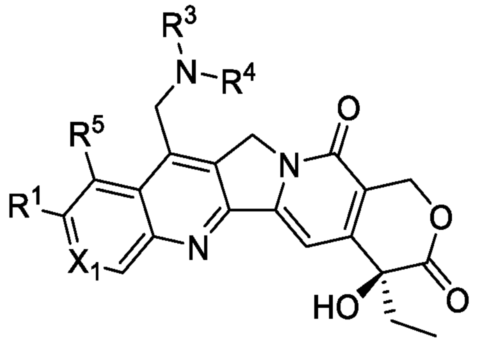

(I)

EP 4 389 752 A1

## Description

The present disclosure claims the rights of priority for Chinese patent application no. CN 202110955364.5, entitled "CAMPTOTHECIN DERIVATIVE AND USE THEREOF" and filed to the China National Intellectual Property Administration on August 19, 2021, Chinese patent application no. CN 202111515247.3, entitled "CAMPTOTHECIN DERIVATIVE AND USE THEREOF" and filed to the China National Intellectual Property Administration on December 13, 2021, and China patent application no. CN 202210515797.3, entitled "CAMPTOTHECIN DERIVATIVE AND USE THEREOF" and filed to the China National Intellectual Property Administration on May 12, 2022. The entirety of the above-referenced prior applications is incorporated herein by reference in their entireties.

## Technical Field

The present disclosure relates to new camptothecin compounds, or stereoisomers or pharmaceutically acceptable salts thereof, pharmaceutical compositions containing same and the use thereof as anti-tumor drugs.

## Background

Camptothecin is a quinoline alkaloid extracted from Camptotheca acuminata (family Nyssaceae). Because of its remarkable cytotoxic activity, it is often used in a cancer treatment related research. Subsequent studies on related mechanisms have shown that camptothecin has the activity of inhibiting topoisomerase I. It can bind to a complex of topoisomerase I and DNA to form a stable ternary complex, thereby hindering topoisomerase I-mediated DNA break, and further blocking DNA replication, transcription and repair processes, ultimately leading to cell cycle arrest and apoptosis.

Early clinical studies have shown that camptothecin suffers from problems such as low solubility, poor chemical stability and obvious toxic and side effects, ultimately hindering its clinical application. Subsequently, camptothecin derivatives, such as topotecan and irinotecan, obtained by modifying camptothecin, have been successfully used in the treatment of malignant tumors such as ovarian cancer, lung cancer and colorectal cancer. Nevertheless, these clinical drugs still have the problems of low solubility and poor chemical stability, which lead to low oral bioavailability, as well as attendant occurrence of serious clinical adverse reactions such as vomiting and bone marrow suppression. Therefore, improving the physicochemical properties, activity and safety of camptothecin drugs is still an urgent problem to be solved.

## Summary of the Invention

The present disclosure provides a compound of formula (I), or a stereoisomer or pharmaceutically acceptable salt thereof:

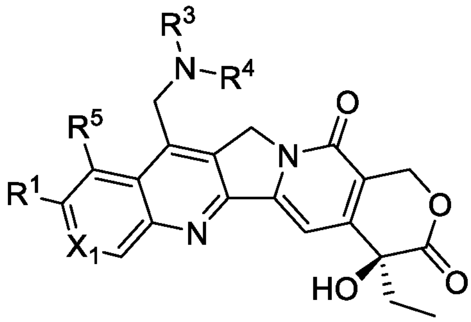

(I)

wherein

- $R^1$ is selected from halogen, CN, $C_1$-$C_6$ alkyl, $C_3$-$C_6$ cycloalkyl or $C_2$-$C_6$ alkynyl, and the $C_1$-$C_6$ alkyl, $C_3$-$C_6$ cycloalkyl or $C_2$-$C_6$ alkynyl is optionally substituted with $R^{a1}$;
- $X_1$ is selected from $CR^2$ or N;
- $R^2$ is selected from H, halogen or CN, or $R^1$ and $R^2$ together with the atoms to which they are attached form a 5- to 6-membered heterocyclyl, and the 5- to 6-membered heterocyclyl is optionally substituted with $R^{a2}$;
- $R^5$ is selected from H, halogen, CN, $NH_2$ or $NO_2$, or $R^1$ and $R^5$ together with the atoms to which they are attached

form a 5- to 6-membered heterocyclyl, a 5- to 6-membered heteroaryl or $C_5$-$C_7$ cycloalkenyl, and the 5- to 6-membered heterocyclyl, 5-to 6-membered heteroaryl or $C_5$-$C_7$ cycloalkenyl is optionally substituted with $R^{a5}$;
$R^3$ is selected from H,

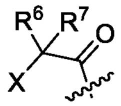,
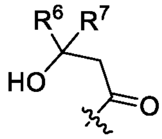 or
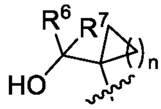

, X is selected from $NH_2$ or OH, $R^6$ is selected from H or $C_1$-$C_3$ alkyl, $R^7$ is selected from H, $C_1$-$C_3$ alkyl or $C_3$-$C_6$ cycloalkyl, or $R^6$ and $R^7$ together with the C atom to which they are attached form $C_3$-$C_6$ cycloalkyl, and the $C_3$-$C_6$ cycloalkyl is optionally substituted with $R^{a3}$;
n is selected from 1, 2, 3 or 4;
$R^4$ is selected from H, or $R^4$ and $R^7$ together with the atoms to which they are attached form a 5- to 6-membered heterocyclyl, and the 5- to 6-membered heterocyclyl is optionally substituted with $R^{a4}$;
each of $R^{a1}$; $R^{a2}$, $R^{a3}$, $R^{a4}$, and $R^{a5}$ is independently selected from D, halogen, CN, =O, OH, $NH_2$, $C_1$-$C_3$ alkyl, $C_3$-$C_6$ cycloalkyl or 4- to 7-membered heterocyclyl, and the OH, $NH_2$, $C_1$-$C_3$ alkyl, $C_3$-$C_6$ cycloalkyl or 4- to 7-membered heterocyclyl is optionally substituted with $R^b$;
each $R^b$ is independently selected from halogen, CN, =O, $C_1$-$C_3$ alkyl, OH, O($C_1$-$C_3$ alkyl), $NH_2$, NH($C_1$-$C_3$ alkyl) or N($C_1$-$C_3$ alkyl)$_2$;
provided that: i) when $R^1$ is selected from methyl and $R^2$ is selected from F, $R^3$ is selected from

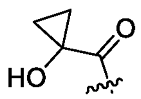,
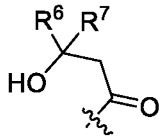 or
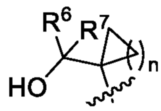,

wherein $R^6$ is selected from H or $C_1$-$C_3$ alkyl, $R^7$ is selected from H, $C_1$-$C_3$ alkyl or $C_3$-$C_6$ cycloalkyl, and n is selected from 1, 2, 3 or 4; ii) when X is selected from $NH_2$, $R^5$ is not selected from H; and iii) the compound of formula (I) does not comprise the following compounds:

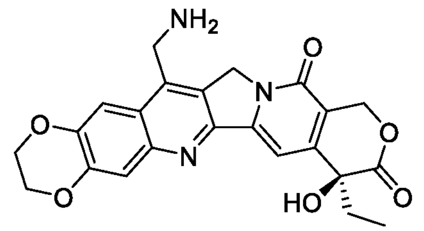,
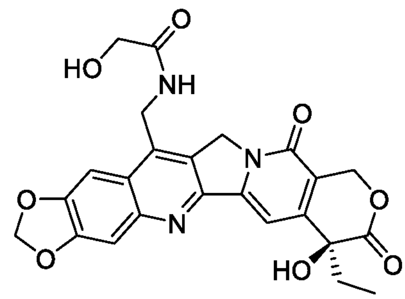,
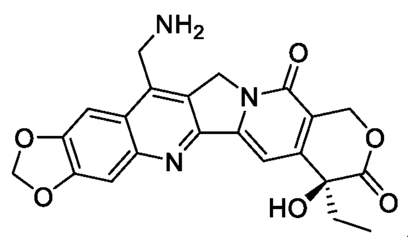.

In some embodiments, provided is the aforementioned compound of formula (I), or the stereoisomer or pharmaceutically acceptable salt thereof, wherein $R^1$ is selected from halogen, CN, $C_1$-$C_6$ alkyl, $C_3$-$C_6$ cycloalkyl or $C_2$-$C_6$ alkynyl, and the $C_1$-$C_6$ alkyl, $C_3$-$C_6$ cycloalkyl or $C_2$-$C_6$ alkynyl is optionally substituted with $R^{a1}$;

$X_1$ is selected from $CR^2$ or N;

$R^2$ is selected from H, halogen or CN, or $R^1$ and $R^2$ together with the atoms to which they are attached form a 5- to 6-membered heterocyclyl, and the 5- to 6-membered heterocyclyl is optionally substituted with $R^{a2}$;

$R^5$ is selected from H, halogen, CN or $NO_2$, or $R^1$ and $R^5$ together with the atoms to which they are attached form a 5- to 6-membered heteroaryl or $C_5$-$C_7$ cycloalkenyl, and the 5- to 6-membered heteroaryl or $C_5$-$C_7$ cycloalkenyl is optionally substituted with $R^{a5}$;

$R^3$ is selected from H,

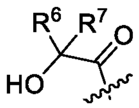
,
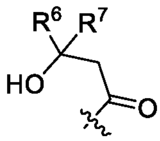
or
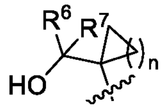
,

$R^6$ is selected from H or $C_1$-$C_3$ alkyl, $R^7$ is selected from H, $C_1$-$C_3$ alkyl or $C_3$-$C_6$ cycloalkyl, or $R^6$ and $R^7$ together with the C atom to which they are attached form $C_3$-$C_6$ cycloalkyl, and the $C_3$-$C_6$ cycloalkyl is optionally substituted with $R^{a3}$;

n is selected from 1, 2, 3 or 4;

$R^4$ is selected from H, or $R^4$ and $R^7$ together with the atoms to which they are attached form a 5- to 6-membered heterocyclyl, and the 5- to 6-membered heterocyclyl is optionally substituted with $R^{a4}$;

each of $R^{a1}$, $R^{a2}$, $R^{a3}$, $R^{a4}$, and $R^{a5}$ is independently selected from halogen, CN, =O, OH, $NH_2$, $C_1$-$C_3$ alkyl, $C_3$-$C_6$ cycloalkyl or 4- to 7-membered heterocyclyl, and the OH, $NH_2$, $C_1$-$C_3$ alkyl, $C_3$-$C_6$ cycloalkyl or 4- to 7-membered heterocyclyl is optionally substituted with $R^b$;

each $R^b$ is independently selected from halogen, CN, =O, $C_1$-$C_3$ alkyl, OH, O($C_1$-$C_3$ alkyl), $NH_2$, NH($C_1$-$C_3$ alkyl) or N($C_1$-$C_3$ alkyl)$_2$;

provided that: i) when $R^1$ is selected from methyl and $R^2$ is selected from F, $R^3$ is selected from

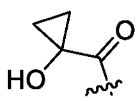
,
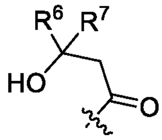
or
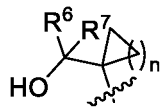
,

wherein $R^6$ is selected from H or $C_1$-$C_3$ alkyl, $R^7$ is selected from H, $C_1$-$C_3$ alkyl or $C_3$-$C_6$ cycloalkyl, and n is selected from 1, 2, 3 or 4; and ii) the compound of formula (I) does not comprise the following compounds:

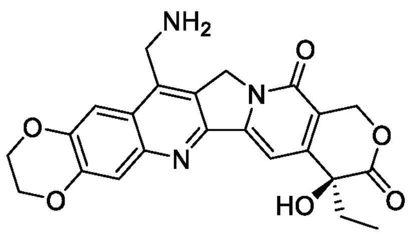
,
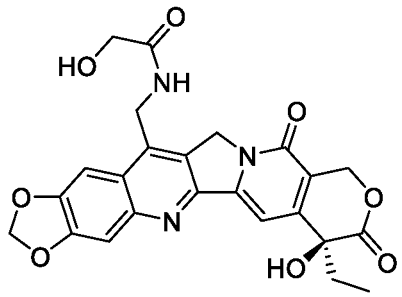
,
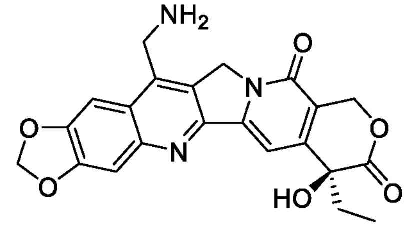
.

In some embodiments, provided is the aforementioned compound of formula (I), or the stereoisomer or pharmaceutically acceptable salt thereof, wherein

$R^1$ is selected from halogen, CN, $C_1$-$C_6$ alkyl, $C_3$-$C_6$ cycloalkyl or $C_2$-$C_6$ alkynyl, and the $C_1$-$C_6$ alkyl, $C_3$-$C_6$ cycloalkyl or $C_2$-$C_6$ alkynyl is optionally substituted with $R^{a1}$;

$X_1$ is selected from $CR^2$ or N;

$R^2$ is selected from H, halogen or CN, or $R^1$ and $R^2$ together with the atoms to which they are attached form a 5- to 6-membered heterocyclyl, and the 5- to 6-membered heterocyclyl is optionally substituted with $R^{a2}$;

$R^5$ is selected from H, halogen or CN, or $R^1$ and $R^5$ together with the atoms to which they are attached form a 5- to 6-membered heteroaryl, and the 5- to 6-membered heteroaryl is optionally substituted with $R^{a5}$;

$R^3$ is selected from H,

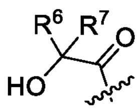 or 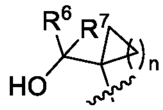,

$R^6$ is selected from H or $C_1$-$C_3$ alkyl, $R^7$ is selected from H, $C_1$-$C_3$ alkyl or $C_3$-$C_6$ cycloalkyl, or $R^6$ and $R^7$ together with the C atom to which they are attached form $C_3$-$C_6$ cycloalkyl, and the $C_3$-$C_6$ cycloalkyl is optionally substituted with $R^{a3}$;

n is selected from 1, 2, 3 or 4;

$R^4$ is selected from H, or $R^4$ and $R^7$ together with the atoms to which they are attached form a 5- to 6-membered heterocyclyl, and the 5- to 6-membered heterocyclyl is optionally substituted with $R^{a4}$;

each of $R^{a1}$, $R^{a2}$, $R^{a3}$, $R^{a4}$, and $R^{a5}$ is independently selected from halogen, CN, =O, OH, $NH_2$, $C_1$-$C_3$ alkyl, $C_3$-$C_6$ cycloalkyl or 4- to 7-membered heterocyclyl, and the OH, $NH_2$, $C_1$-$C_3$ alkyl, $C_3$-$C_6$ cycloalkyl or 4- to 7-membered heterocyclyl is optionally substituted with $R^b$;

each $R^b$ is independently selected from halogen, CN, =O, $C_1$-$C_3$ alkyl, OH, O($C_1$-$C_3$ alkyl), $NH_2$, NH($C_1$-$C_3$ alkyl) or N($C_1$-$C_3$ alkyl)$_2$;

provided that: i) when $R^1$ is selected from methyl and $R^2$ is selected from F, $R^3$ is selected from

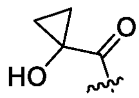 or 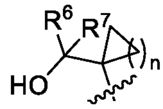,

wherein $R^6$ is selected from H or $C_1$-$C_3$ alkyl, $R^7$ is selected from H, $C_1$-$C_3$ alkyl or $C_3$-$C_6$ cycloalkyl, and n is selected from 1, 2, 3 or 4; and ii) the compound of formula (I) does not comprise the following compounds:

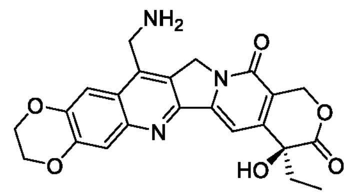,

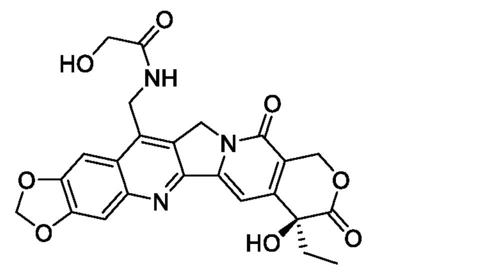,

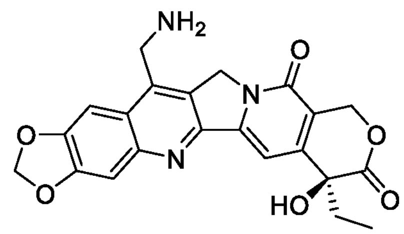.

In some embodiments, provided is the aforementioned compound of formula (I), or the stereoisomer or pharmaceutically acceptable salt thereof, wherein

$R^1$ is selected from halogen, CN, $C_1$-$C_6$ alkyl, $C_3$-$C_6$ cycloalkyl or $C_2$-$C_6$ alkynyl, and the $C_1$-$C_6$ alkyl, $C_3$-$C_6$ cycloalkyl

or $C_2$-$C_6$ alkynyl is optionally substituted with $R^{a1}$;

$X_1$ is selected from $CR^2$ or N;

$R^2$ is selected from halogen or CN, or $R^1$ and $R^2$ together with the atoms to which they are attached form a 5- to 6-membered heterocyclyl, and the 5- to 6-membered heterocyclyl is optionally substituted with $R^{a2}$;

$R^5$ is selected from H, halogen or CN;

$R^3$ is selected from H or

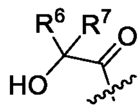

,

$R^6$ is selected from H, $R^7$ is selected from H or $C_3$-$C_6$ cycloalkyl, or $R^6$ and $R^7$ together with the C atom to which they are attached form $C_3$-$C_6$ cycloalkyl, and the $C_3$-$C_6$ cycloalkyl is optionally substituted with $R^{a3}$;

$R^4$ is selected from H, or $R^4$ and $R^7$ together with the atoms to which they are attached form a 5- to 6-membered heterocyclyl, and the 5- to 6-membered heterocyclyl is optionally substituted with $R^{a4}$;

each of $R^{a1}$, $R^{a2}$, $R^{a3}$, and $R^{a4}$ is independently selected from halogen, CN, =O, OH, $NH_2$, $C_1$-$C_3$ alkyl, $C_3$-$C_6$ cycloalkyl or 4- to 7-membered heterocyclyl, and the OH, $NH_2$, $C_1$-$C_3$ alkyl, $C_3$-$C_6$ cycloalkyl or 4- to 7-membered heterocyclyl is optionally substituted with $R^b$;

each $R^b$ is independently selected from halogen, CN, =O, $C_1$-$C_3$ alkyl, OH, O($C_1$-$C_3$ alkyl), $NH_2$, NH($C_1$-$C_3$ alkyl) or N($C_1$-$C_3$ alkyl)$_2$;

provided that: i) when $R^1$ is selected from methyl and $R^2$ is selected from F, $R^3$ is selected from

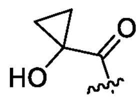

;

and ii) the compound of formula (I) does not comprise the following compounds:

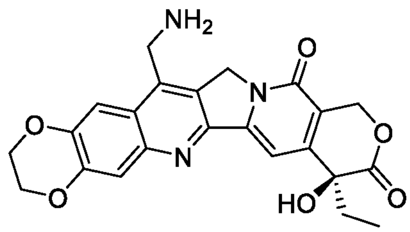

,

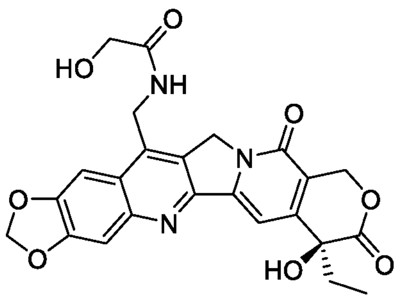

,

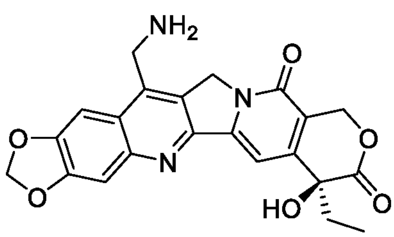

.

In some embodiments, $R^{a1}$; $R^{a2}$, $R^{a3}$, $R^{a4}$, $R^{a5}$ are independently selected from D, halogen, CN, =O or OH.

In some embodiments, $R^{a1}$; $R^{a2}$, $R^{a3}$, $R^{a4}$, $R^{a5}$ are independently selected from halogen, CN, =O or OH.

In some embodiments, $R^{a1}$; $R^{a2}$, $R^{a3}$, $R^{a4}$ are independently selected from halogen, CN, =O or OH.

In some embodiments, $R^{a2}$ and $R^{a3}$ are independently selected from D.

In some embodiments, $R^1$ is selected from halogen, $C_1$-$C_3$ alkyl, $C_3$-$C_6$ cycloalkyl or $C_2$-$C_3$ alkynyl.

In some embodiments, $R^1$ is selected from Cl, Br, methyl, cyclopropyl or ethynyl.

In some embodiments, $R^1$ is selected from Cl, Br or methyl.

In some embodiments, $X_1$ is selected from N.

In some embodiments, $X_1$ is selected from $CR^2$.

In some embodiments, $R^2$ is selected from H, halogen or CN, or $R^1$ and $R^2$ together with the atoms to which they are attached form a 5- to 6-membered heterocyclyl, the 5- to 6-membered heterocyclyl contains 1 or 2 oxygen atoms as ring atoms, and the 5- to 6-membered heterocyclyl is optionally substituted with D atom.

In some embodiments, $R^2$ is selected from H, halogen or CN, or $R^1$ and $R^2$ together with the atoms to which they are attached form a 5- to 6-membered heterocyclyl, and the 5- to 6-membered heterocyclyl contains 1 or 2 oxygen atoms as ring atoms.

In some embodiments, $R^2$ is selected from halogen or CN, or $R^1$ and $R^2$ together with the atoms to which they are attached form a 5- to 6-membered heterocyclyl, and the 5- to 6-membered heterocyclyl contains 1 or 2 oxygen atoms as ring atoms.

In some embodiments, $R^2$ is selected from H or halogen, or $R^1$ and $R^2$ together with the atoms to which they are attached form a 5- to 6-membered heterocyclyl, and the 5- to 6-membered heterocyclyl contains 1 or 2 oxygen atoms as ring atoms.

In some embodiments, $R^2$ is selected from H, F or Cl, or $R^1$ and $R^2$ together with the atoms to which they are attached form

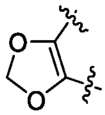, 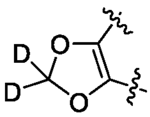 or 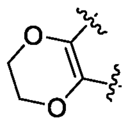.

In some embodiments, $R^2$ is selected from H, F or Cl, or $R^1$ and $R^2$ together with the atoms to which they are attached form

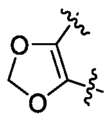 or 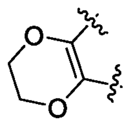.

In some embodiments, $R^2$ is selected from F or Cl, or $R^1$ and $R^2$ together with the atoms to which they are attached form

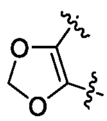 or 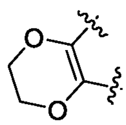.

In some embodiments, $R^1$ is selected from methyl, and $R^2$ is selected from F or Cl.

In some embodiments, $R^1$ is selected from methyl, and $R^2$ is selected from Cl.

In some embodiments, $R^1$ is selected from Cl, Br, cyclopropyl or ethynyl, and $R^2$ is selected from F.

In some embodiments, $R^5$ is selected from H, halogen, $NH_2$ or $NO_2$, or $R^1$ and $R^5$ together with the atoms to which they are attached form a 5- to 6-membered heteroaryl or $C_5$-$C_6$ cycloalkenyl, and the 5- to 6-membered heteroaryl or $C_5$-$C_6$ cycloalkenyl is optionally substituted with $R^{a5}$.

In some embodiments, $R^5$ is selected from H, halogen or $NO_2$, or $R^1$ and $R^5$ together with the atoms to which they are attached form a 5- to 6-membered heteroaryl or $C_5$-$C_6$ cycloalkenyl, and the 5- to 6-membered heteroaryl or $C_5$-$C_6$ cycloalkenyl is optionally substituted with $R^{a5}$.

In some embodiments, $R^5$ is selected from H or halogen, or $R^1$ and $R^5$ together with the atoms to which they are attached form a 5- to 6-membered heteroaryl.

In some embodiments, $R^5$ is selected from H, halogen or $NO_2$.

In some embodiments, $R^5$ is selected from H or halogen.

In some embodiments, $R^5$ is selected from H, Cl, F, $NH_2$ or $NO_2$, or $R^1$ and $R^5$ together with the atoms to which they are attached form

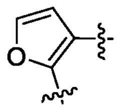 or 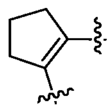 .

In some embodiments, $R^5$ is selected from H, Cl, F or $NO_2$, or $R^1$ and $R^5$ together with the atoms to which they are attached form

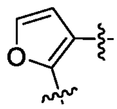 or 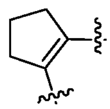 .

In some embodiments, $R^5$ is selected from H or F, or $R^1$ and $R^5$ together with the atoms to which they are attached form

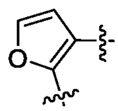 .

In some embodiments, $R^5$ is selected from H, Cl, F or $NO_2$.

In some embodiments, $R^5$ is selected from H or F.

In some embodiments, $R^3$ is selected from H.

In some embodiments, $R^3$ is selected from

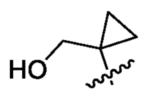 .

In some embodiments, $R^3$ is selected from

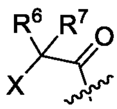 or 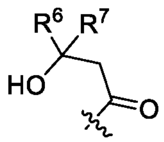 ,

X is selected from $NH_2$ or OH, $R^6$ is selected from H or methyl, and $R^7$ is selected from H, methyl, isopropyl, or cyclopropyl optionally substituted with D atom.

In some embodiments, $R^3$ is selected from

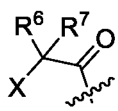 or 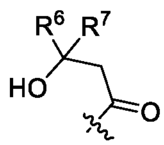 ,

X is selected from $NH_2$ or OH, $R^6$ is selected from H or methyl, and $R^7$ is selected from H, methyl, isopropyl or cyclopropyl.

In some embodiments, $R^3$ is selected from

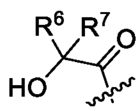 or 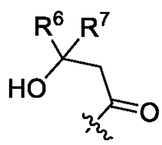 ,

$R^6$ is selected from H or methyl, and $R^7$ is selected from H, methyl, cyclopropyl or isopropyl.

In some embodiments, $R^3$ is selected from

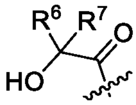

, $R^6$ is selected from H or methyl, and $R^7$ is selected from H, methyl, cyclopropyl or isopropyl.

In some embodiments, $R^3$ is selected from

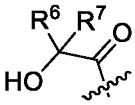 or 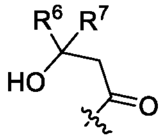,

$R^6$ is selected from H, and $R^7$ is selected from H or cyclopropyl.

In some embodiments, $R^3$ is selected from

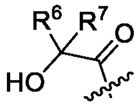,

$R^6$ is selected from H, and $R^7$ is selected from H or cyclopropyl.

In some embodiments, $R^3$ is selected from

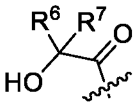,

and $R^6$ and $R^7$ together with the C atom to which they are attached form $C_3$-$C_6$ cycloalkyl.

In some embodiments, $R^3$ is selected from

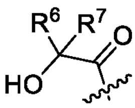,

and $R^6$ and $R^7$ together with the C atom to which they are attached form cyclopropyl.

In some embodiments, $R^1$ is selected from methyl, $X_1$ is selected from CF or CCl, $R^3$ is selected from

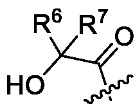,

and $R^6$ and $R^7$ together with the C atom to which they are attached form $C_3$-$C_6$ cycloalkyl.

In some embodiments, $R^1$ is selected from methyl, $X_1$ is selected from CF, $R^3$ is selected from

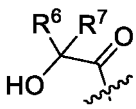,

and $R^6$ and $R^7$ together with the C atom to which they are attached form $C_3$-$C_6$ cycloalkyl.

In some embodiments, $R^1$ is selected from methyl, $R^2$ is selected from F or Cl, and $R^3$ is selected from

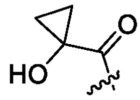

.

In some embodiments, $R^1$ is selected from methyl, $R^2$ is selected from F, and $R^3$ is selected from

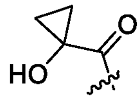

.

In some embodiments, $R^4$ is selected from H.

In some embodiments, $R^4$ and $R^7$ together with the atoms to which they are each attached form a 5- to 6-membered heterocyclyl.

In some embodiments, $R^4$ and $R^7$ together with the atoms to which they are each attached form a 5-membered heterocyclyl.

In some embodiments, $R^3$ is selected from H or

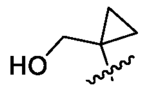

,

and $R^4$ is selected from H.

In some embodiments, $R^3$ and $R^4$ are both selected from H.

In some embodiments, $R^4$ is selected from H, $R^3$ is selected from

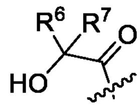

, $R^6$ is selected from H, $R^7$ is selected from H or cyclopropyl, or $R^6$ and $R^7$ together with the C atom to which they are attached form cyclopropyl.

In some embodiments, $R^3$ is selected from

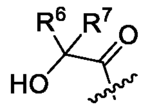

,

$R^6$ is selected from H, and $R^4$ and $R^7$ together with the atoms to which they are each attached form a 5- to 6-membered heterocyclyl.

In some embodiments, $R^3$ is selected from

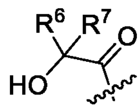

,

$R^6$ is selected from H, and $R^4$ and $R^7$ together with the atoms to which they are each attached form a 5-membered heterocyclyl.

In some embodiments, $R^1$ and $R^2$ together with the atoms to which they are each attached form

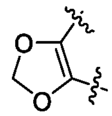 or 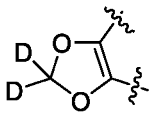,

$R^3$ is selected from H,

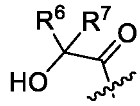

or

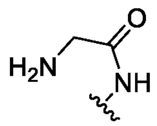,

$R^4$ is selected from H, $R^6$ is selected from H, $R^7$ is selected from H, or cyclopropyl optionally substituted with D atom, or $R^6$ and $R^7$ together with the C atom to which they are attached form $C_3$-$C_6$ cycloalkyl.

In some embodiments, $R^1$ and $R^2$ together with the atoms to which they are each attached form

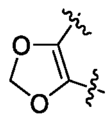 or 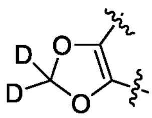,

$R^3$ is selected from

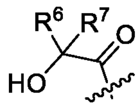,

$R^4$ is selected from H, $R^6$ is selected from H, $R^7$ is selected from cyclopropyl optionally substituted with D atom, or $R^6$ and $R^7$ together with the C atom to which they are attached form cyclopropyl.

In some embodiments, $R^1$ and $R^2$ together with the atom to which they are each attached form

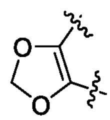,

$R^3$ is selected from

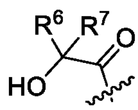,

$R^4$ is selected from H, $R^6$ is selected from H, $R^7$ is selected from cyclopropyl, or $R^6$ and $R^7$ together with the C atom to which they are attached form $C_3$-$C_6$ cycloalkyl.

In some embodiments, $R^1$ and $R^2$ together with the atoms to which they are each attached form

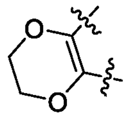,

$R^3$ is selected from H,

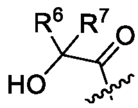 or 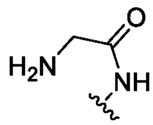,

$R^4$ is selected from H, $R^6$ is selected from H, $R^7$ is selected from H or cyclopropyl, or $R^6$ and $R^7$ together with the C atom to which they are attached form $C_3$-$C_6$ cycloalkyl.

In some embodiments, $R^1$ and $R^2$ together with the atom to which they are each attached form

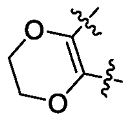,

$R^3$ is selected from

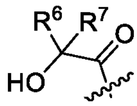,

$R^4$ is selected from H, $R^6$ is selected from H, $R^7$ is selected from H or cyclopropyl, or $R^6$ and $R^7$ together with the C atom to which they are attached form $C_3$-$C_6$ cycloalkyl.

In some embodiments, $R^1$ and $R^5$ together with the atoms to which they are attached form a 5- to 6-membered heteroaryl or $C_5$-$C_6$ cycloalkenyl, and $R^3$ is selected from

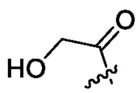 or 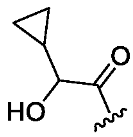.

In some embodiments, $R^1$ and $R^5$ together with the atoms to which they are attached form a 5- to 6-membered heteroaryl, and $R^3$ is selected from

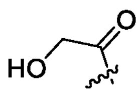

or

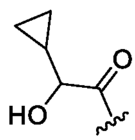.

In some embodiments, $R^1$ and $R^5$ together with the atoms to which they are attached form a 5- to 6-membered

heteroaryl, and $R^3$ is selected from

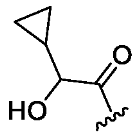

.

In some embodiments, $R^1$ and $R^5$ together with the atoms to which they are attached form a 5- to 6-membered heteroaryl, and $R^3$ is selected from

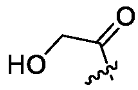

.

In some embodiments, $R^2$ is selected from H, and $R^1$ and $R^5$ together with the atoms to which they are attached form a 5- to 6-membered heteroaryl or $C_5$-$C_6$ cycloalkenyl.

In some embodiments, $R^2$ is selected from H, and $R^1$ and $R^5$ together with the atoms to which they are attached form $C_5$-$C_6$ cycloalkenyl.

In some embodiments, $R^2$ is selected from H, and $R^1$ and $R^5$ together with the atoms to which they are attached form a 5- to 6-membered heteroaryl.

In some embodiments, $R^2$ is selected from H, $R^1$ and $R^5$ together with the atom to which they are attached form a 5- to 6-membered heteroaryl, and $R^3$ is selected from

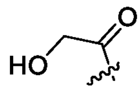

.

In some embodiments, $R^3$ is selected from H,

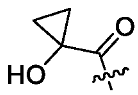, 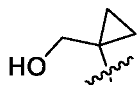,

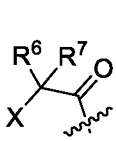 or 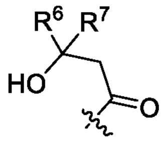,

wherein X is selected from $NH_2$ or OH, $R^6$ is selected from H or methyl, and $R^7$ is selected from H, methyl, isopropyl, or cyclopropyl optionally substituted with D atom; $R^4$ is selected from H, or $R^4$ and $R^7$ together with the atoms to which they are each attached form a 5-membered heterocyclyl.

In some embodiments, $R^3$ is selected from H,

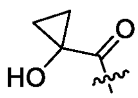, 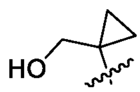,

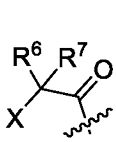 or 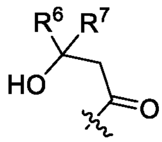,

wherein X is selected from $NH_2$ or OH, $R^6$ is selected from H or methyl, and $R^7$ is selected from H, methyl, isopropyl or cyclopropyl; $R^4$ is selected from H.

In some embodiments, the structural unit

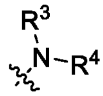

is selected from

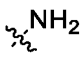,

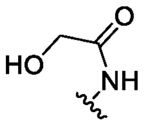, 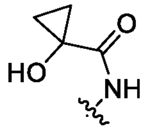, 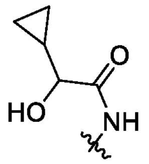, 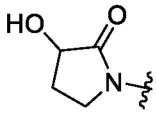, 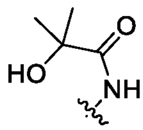, 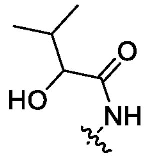,

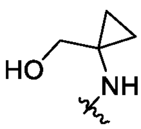, 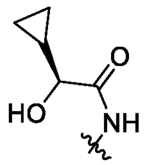, 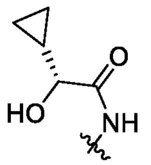, 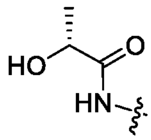, 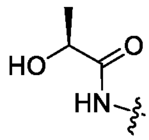, 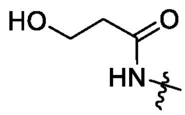,

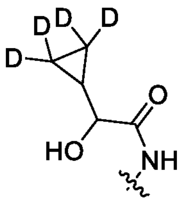 or 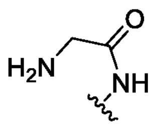.

In some embodiments, the structural unit

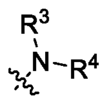

is selected from

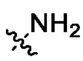,

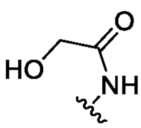, 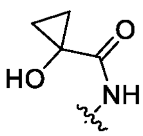, 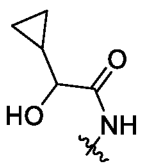, 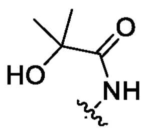, 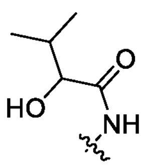, 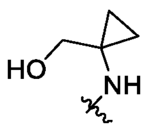, 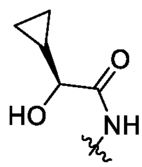,

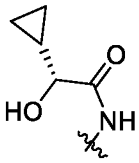, 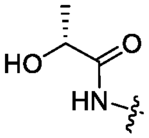, 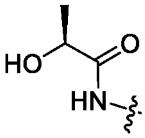, 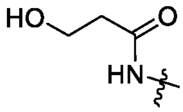 or 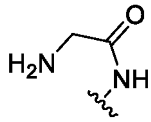.

In some embodiments, the structural unit

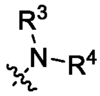

is selected from

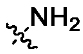,

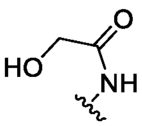, 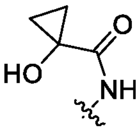, 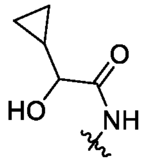, 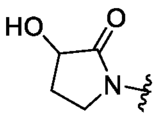, 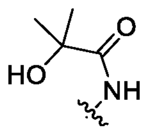, 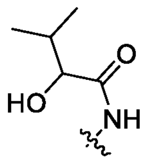,

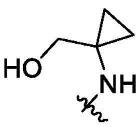, 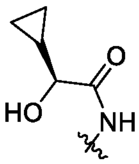, 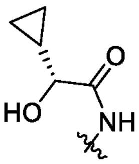, 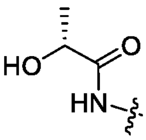, 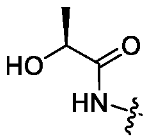 or 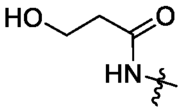.

In some embodiments, the structural unit

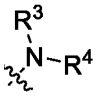

is selected from

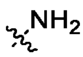,

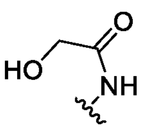, 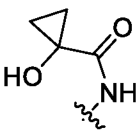, 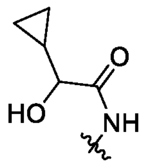, 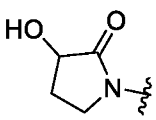, 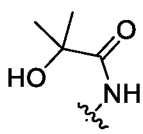, 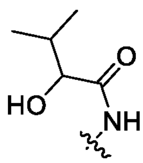,

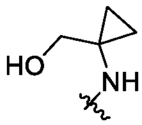, 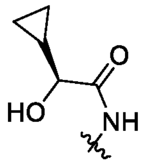, 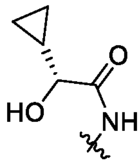, 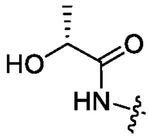 or 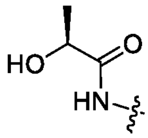.

In some embodiments, the structural unit

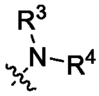

is selected from

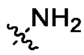,

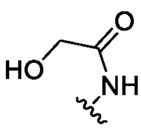, 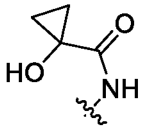, 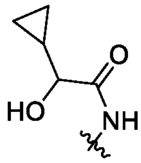 or 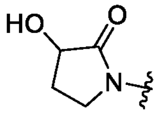.

In some embodiments, $R^1$ is selected from methyl, $X_1$ is selected from CF, and the structural unit

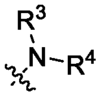

is selected from

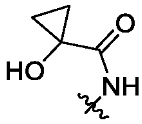.

In some embodiments, $R^1$ and $R^2$ together with the atoms to which they are attached form

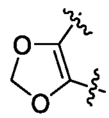 or 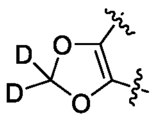,

and the structural unit

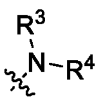

is selected from

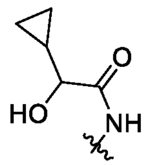
.

In some embodiments, $R^1$ and $R^2$ together with the atoms to which they are each attached form

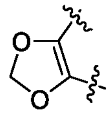
,

and the structural unit

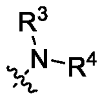

is selected from

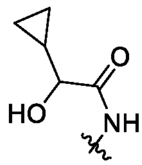

In some embodiments, $R^1$ and $R^2$ together with the atoms to which they are each attached form

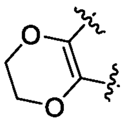
,

and the structural unit

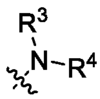

is selected from

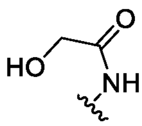
.

In some embodiments, $R^1$ and $R^5$ together with the atoms to which they are attached form

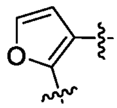 or 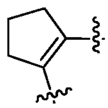 ,

and the structural unit

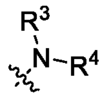

is selected from

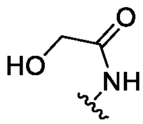 or 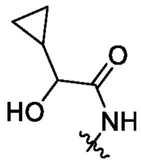 .

In some embodiments, $R^1$ and $R^5$ together with the atoms to which they are attached form

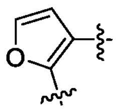 ,

and the structural unit

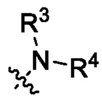

is selected from

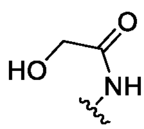 .

In some embodiments, the compound of formula (I), or the stereoisomer or pharmaceutically acceptable salt thereof, is selected from a compound of formula (Ia), or a stereoisomer or pharmaceutically acceptable salt thereof:

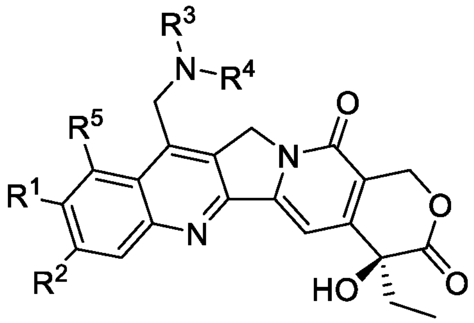

(Ia)

wherein $R^1$, $R^2$, $R^3$, $R^4$ and $R^5$ are as defined above.

It is to be understood that in claim 11 relating to formula (Ia), when claim 11 refers to the preceding claim x, $R^1$, $R^2$, $R^3$, $R^4$ and $R^5$ in the formula (Ia) are as defined in claim x. For example, when claim 11 refers to the preceding claim 1, $R^1$, $R^2$, $R^3$, $R^4$ and $R^5$ in the formula (Ia) are as defined in claim 1; when claim 11 refers to the preceding claim 2, $R^1$, $R^2$, $R^3$, $R^4$ and $R^5$ in the formula (Ia) are as defined in claim 2, and so on.

In some embodiments, the compound of formula (I), or the stereoisomer or pharmaceutically acceptable salt thereof, is selected from a compound of formula (Ib), or a stereoisomer or pharmaceutically acceptable salt thereof:

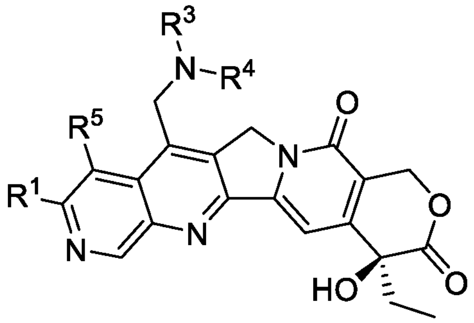

(Ib)

wherein $R^1$, $R^3$, $R^4$ and $R^5$ are as defined above.

It is to be understood that in claim 12 relating to formula (Ib), when claim 12 refers to the preceding claim x, $R^1$, $R^3$, $R^4$ and $R^5$ in the formula (Ib) are as defined in claim x. For example, when claim 12 refers to the preceding claim 1, $R^1$, $R^3$, $R^4$ and $R^5$ in the formula (Ib) are as defined in claim 1; when claim 12 refers to the preceding claim 2, $R^1$, $R^3$, $R^4$ and $R^5$ in the formula (Ib) are as defined in claim 2, and so on.

The present disclosure provides a compound of formula (II), or a stereoisomer or pharmaceutically acceptable salt thereof:

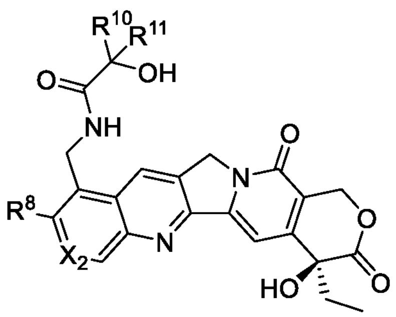

(II)

wherein

$R^8$ is selected from hydroxyl, halogen, CN, $C_1$-$C_6$ alkyl, $C_3$-$C_6$ cycloalkyl or $C_2$-$C_6$ alkynyl;
$X_2$ is selected from $CR^9$ or N;
$R^9$ is selected from H, halogen or CN, or $R^8$ and $R^9$ together with the atoms to which they are each attached form a 5- to 6-membered heterocyclyl;
$R^{10}$ and $R^{11}$ are independently selected from H, $C_3$-$C_6$ cycloalkyl, or $R^{10}$ and $R^{11}$ together with the C atom to which they are attached form $C_3$-$C_6$ cycloalkyl.

In some embodiments, $R^8$ is selected from hydroxyl, halogen or CN.
In some embodiments, $R^8$ is selected from hydroxyl.
In some embodiments, $X_2$ is selected from CH.
In some embodiments, $R^{10}$ and $R^{11}$ are both selected from H.
In case of no conflict, it is to be understood that the above-described embodiments can be arbitrarily combined to form technical solutions comprising the features of the combined embodiments. Such combined technical solutions are within the scope of the present invention. In some embodiments, the compound of formula (I) or formula (II), or the stereoisomer or pharmaceutically acceptable salt thereof, is selected from the following compounds, or stereoisomers or pharmaceutically acceptable salts thereof:

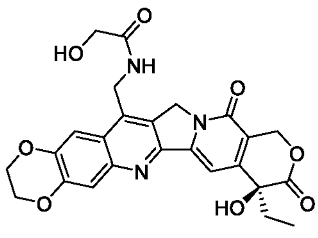
1

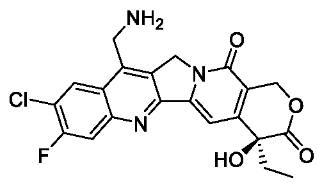
2

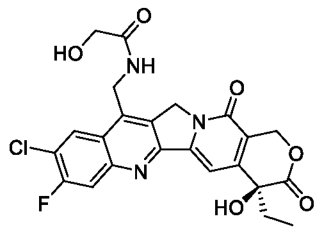
3

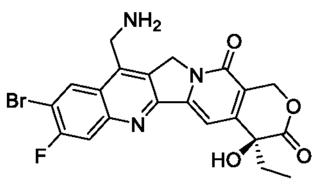
4

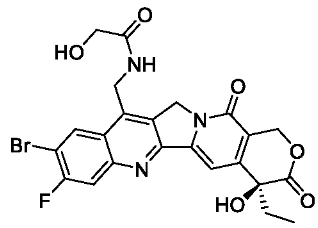
5

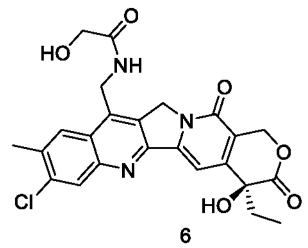
6

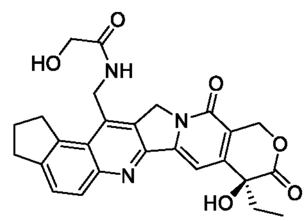
7

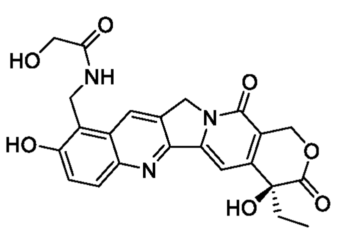
8

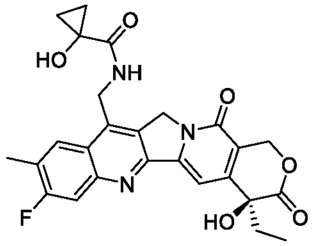
9

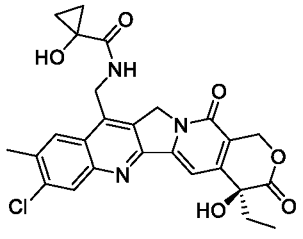
10

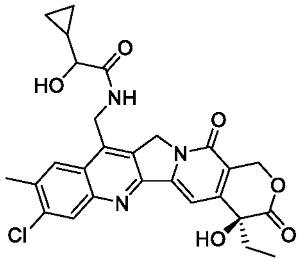
11

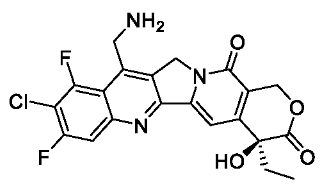
12

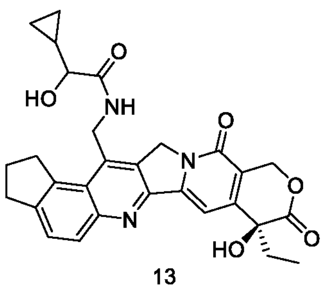
13

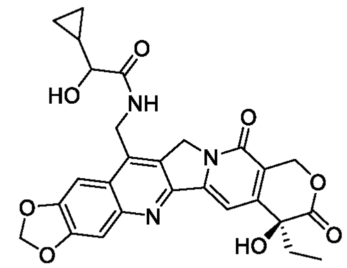
14

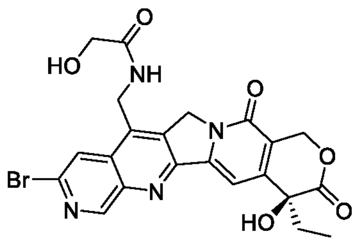
15

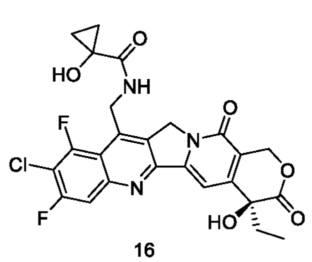
16

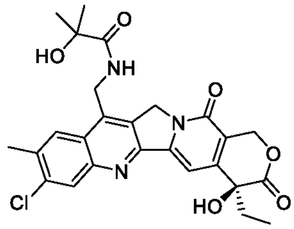
17

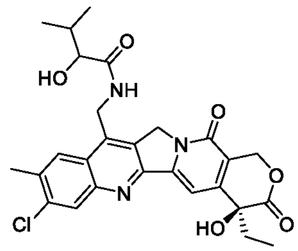
18

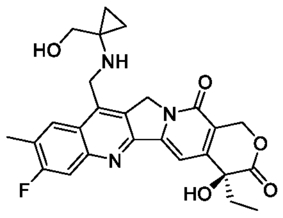
19

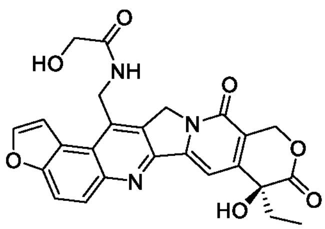
20

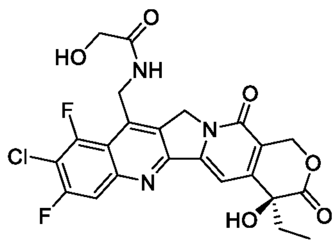
21

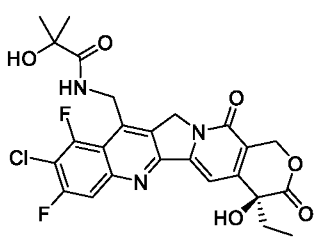
22

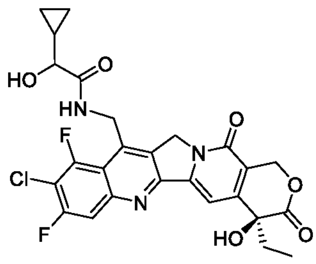
23

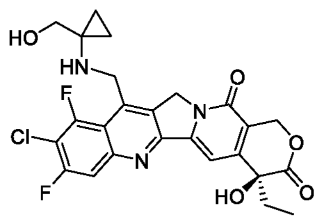
24

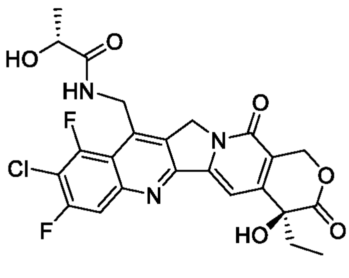
25

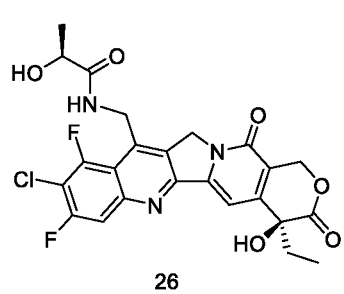
26

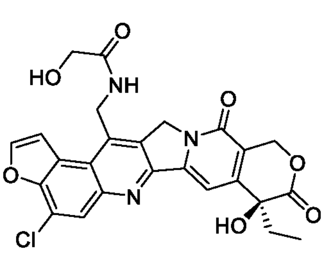
27

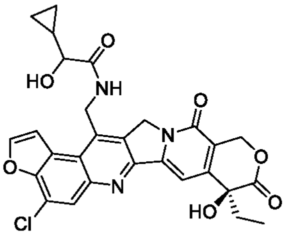
28

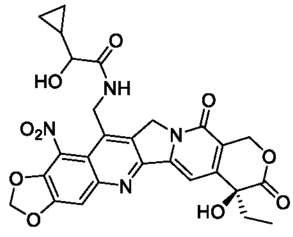
29

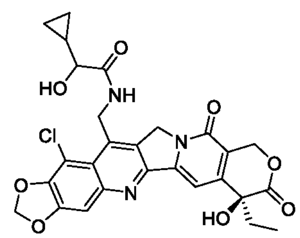
30

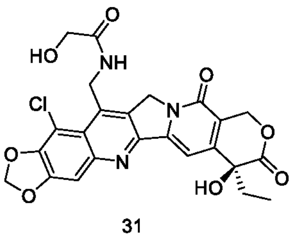
31

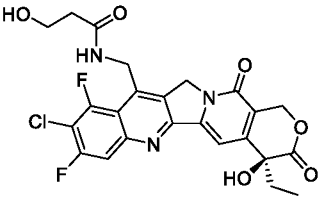
32

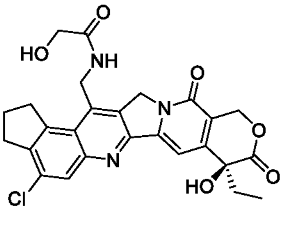
33

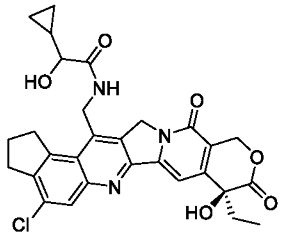
34

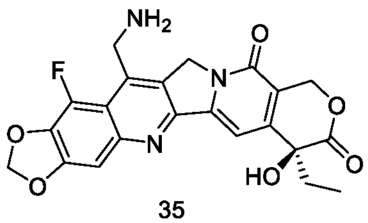
35

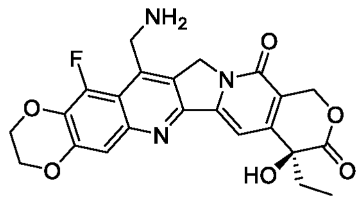
36

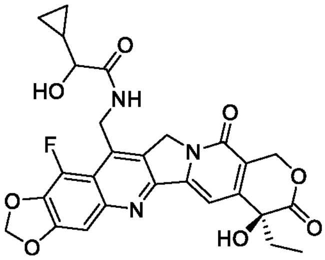
37

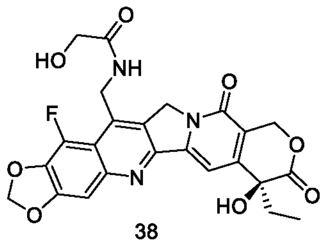
38

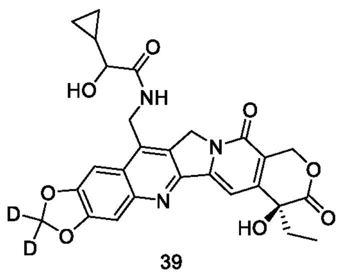
39

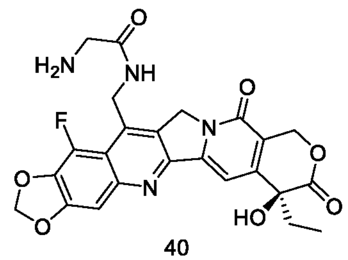
40

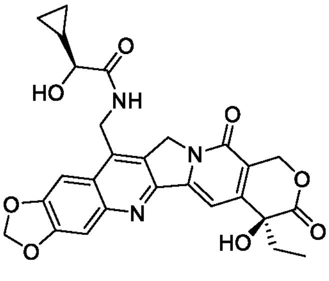

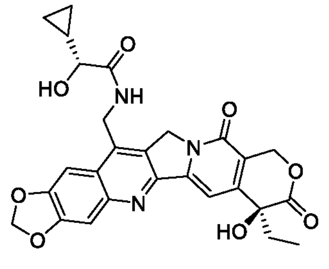

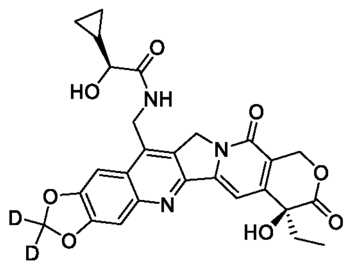
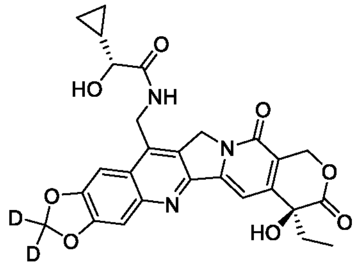
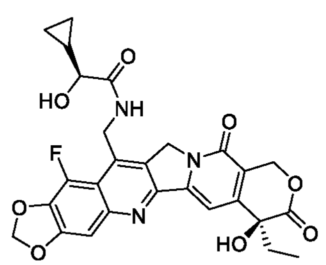

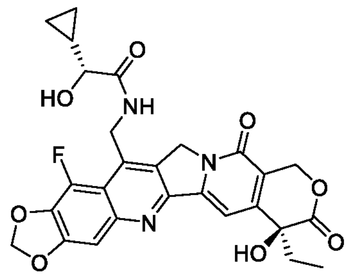
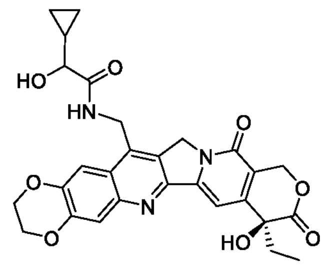
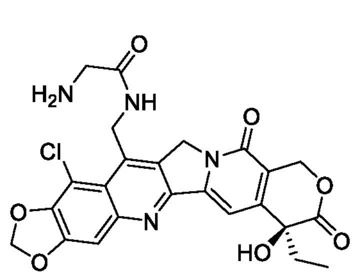

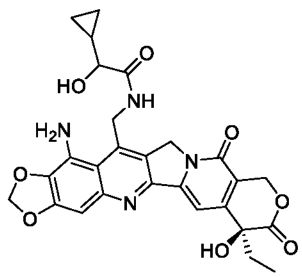
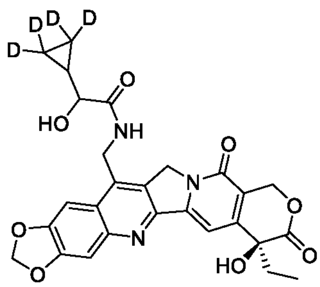
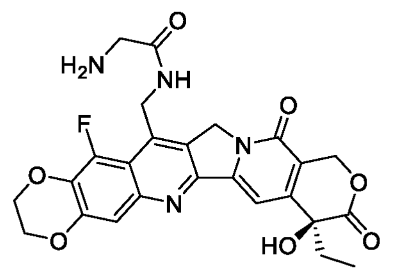

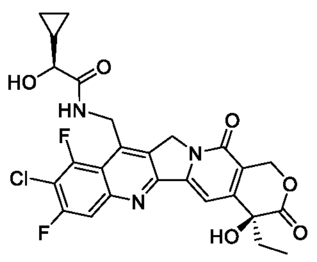
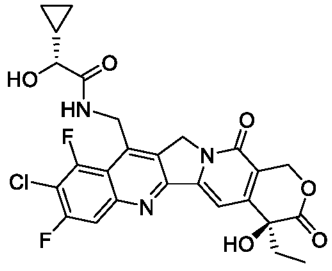
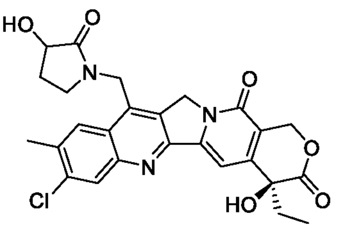

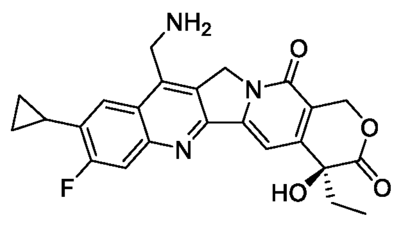

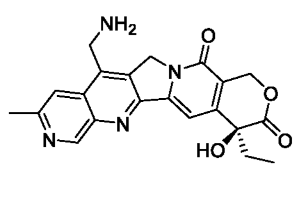
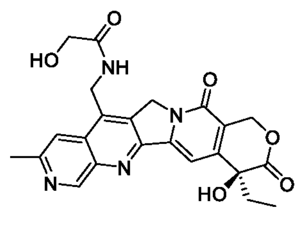
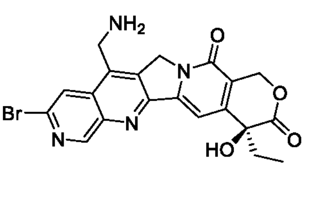

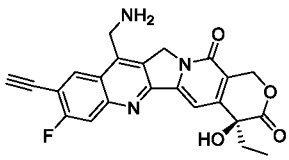 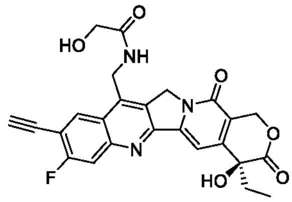 or 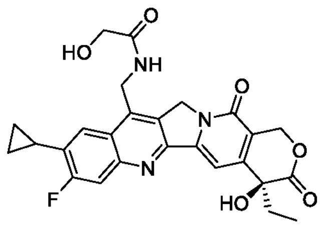 .

The present disclosure further provides a pharmaceutical composition, comprising a compound of formula (I) or formula (II), or a stereoisomer or pharmaceutically acceptable salt thereof, and a pharmaceutically acceptable adjuvant.

Further, the present disclosure provides the use of a compound of formula (I) or formula (II), or a stereoisomer or pharmaceutically acceptable salt thereof, or a pharmaceutical composition thereof, in the preparation of a medicament for preventing or treating a topoisomerase I-related disease.

Further, the present disclosure provides the use of a compound of formula (I) or formula (II), or a stereoisomer or pharmaceutically acceptable salt thereof, or a pharmaceutical composition thereof, in the prevention or treatment of a topoisomerase I-related disease.

Further, the present disclosure provides a compound of formula (I) or formula (II), or a stereoisomer or pharmaceutically acceptable salt thereof, or a pharmaceutical composition thereof, for use in the prevention or treatment of a topoisomerase I-related disease.

The present disclosure further provides a method for treating a topoisomerase I-related disease, comprising administering to a patient a therapeutically effective dose of a compound of formula (I) or formula (II), or a stereoisomer or pharmaceutically acceptable salt thereof, or a pharmaceutical composition thereof, or a pharmaceutical preparation comprising the compound of formula (I) or formula (II), or the stereoisomer or pharmaceutically acceptable salt thereof of the present disclosure.

In some embodiments, the topoisomerase I-related disease includes, but is not limited to, cancer.

Further, the present disclosure provides the use of a compound of formula (I) or formula (II), or a stereoisomer or pharmaceutically acceptable salt thereof, or a pharmaceutical composition thereof, in the preparation of an anti-tumor drug.

Further, the present disclosure provides the anti-tumor use of a compound of formula (I) or formula (II), or a stereoisomer or pharmaceutically acceptable salt thereof, or a pharmaceutical composition thereof.

Further, the present disclosure provides a compound of formula (I) or formula (II), or a stereoisomer or pharmaceutically acceptable salt thereof, or a pharmaceutical composition thereof, for use in an anti-tumor application.

The present disclosure also provides a method for treating a tumor, comprising administering to a patient a therapeutically effective dose of a compound of formula (I) or formula (II), or a stereoisomer or pharmaceutically acceptable salt thereof, or a pharmaceutical composition thereof, or a pharmaceutical preparation comprising the compound of formula (I) or formula (II), or the stereoisomer or pharmaceutically acceptable salt thereof of the present disclosure.

## Definition and Description of Terminology

Unless otherwise stated, the terms used in the present disclosure have the following meanings; the definitions of groups and terms described in the present disclosure, including their definitions as examples, exemplary definitions, preferred definitions, definitions listed in tables, definitions of specific compounds in the examples, etc., may be arbitrarily combined or incorporated with one another. A specific term should not be considered uncertain or unclear unless specifically defined, but should be understood in its ordinary meaning in the art. When a trade name appears herein, it is intended to refer to the corresponding commodity or an active ingredient thereof.

“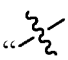”

herein indicates a linking site.

The diagrammatic presentation of the racemate or enantiomerically pure compound herein is from Maehr, J.Chem.Ed. 1985, 62:114-120. Unless otherwise stated, the wedged solid bond and wedged dashed bond (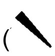 and 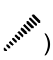) are used to represent the absolute configuration of a stereocenter, and the straight solid bond and straight dashed bond (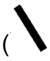 and 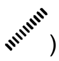) are used to represent the relative configuration of a stereocenter (such as cis or trans configuration

of alicyclic compounds).

The term "tautomer" refers to a functional group isomer resulting from the rapid movement of an atom in two positions in a molecule. The compounds of the present disclosure may exhibit tautomerism. Tautomeric compounds can exist as two or more interconvertible forms. Tautomers generally exist in equilibrium form, so that the attempts to separate a single tautomer usually result in the formation of a mixture, whose chemical and physical properties are consistent with a mixture of the compounds. The position of equilibrium depends on the chemical properties within the molecule. For example, in many aliphatic aldehydes and ketones such as acetaldehyde, the ketone form is dominant; and in phenols, the enol form is dominant. The present disclosure encompasses all tautomeric forms of the compounds.

The term "stereoisomer" refers to an isomer created as a result of different spatial arrangement of atoms in molecules, including cis and trans isomers, enantiomers and diastereomers.

The compounds of the present disclosure may have asymmetric atoms, such as carbon atoms, sulfur atoms, nitrogen atoms, phosphorus atoms or asymmetric double bonds, and therefore the compounds of the present disclosure may exist in specific geometric or stereoisomeric forms. Specific geometric or stereoisomeric forms may be cis and trans isomers, E- and Z-geometric isomers, (-)- and (+)-enantiomers, (R)- and (S)-enantiomers, diastereomers, (D)-isomers, (L)-isomers, and racemic mixtures or other mixtures thereof, such as enantiomerically or diastereomerically enriched mixtures, and all of the above isomers and mixtures thereof fall within the scope of the definition of the compounds of the present disclosure. Additional asymmetric carbon atoms, asymmetric sulfur atoms, asymmetric nitrogen atoms, or asymmetric phosphorus atoms may be present in substituents such as an alkyl group, and these isomers and mixtures thereof involved in all substituents are also included in the scope of the definition of the compounds of the present disclosure. The compounds containing asymmetric atoms of the present disclosure can be separated in optically active-pure or racemic forms, and the optically active-pure forms can be resolved from the racemic mixture or synthesized by utilizing chiral raw materials or chiral reagents.

The term "substituted" means that any one or more hydrogen atoms on the designated atom are substituted with a substituent, provided that the valence state of the designated atom is normal, and the substituted compound is stable. When the substituent is oxo (i.e., =O), it means that two hydrogen atoms are substituted, which would not occur on aromatic groups.

The term "optional" or "optionally" means that the subsequently described event or circumstance may or may not occur, and the description includes the occurrence and the non-occurrence of the event or circumstance. For example, the expression "ethyl is "optionally" substituted with halogen" means that ethyl may be unsubstituted ($CH_2CH_3$), mono-substituted (e.g., $CH_2CH_2F$, $CH_2CH_2Cl$, etc.), poly-substituted (e.g., $CHFCH_2F$, $CH_2CHF_2$, $CHFCH_2Cl$, $CH_2CHCl_2$, etc.) or completely substituted ($CF_2CF_3$, $CF_2CCl_3$, $CCl_2CCl_3$, etc.). With respect to any group containing one or more substituents, it will be understood by those skilled in the art that any substitution or substitution patterns that are sterically impractical and/or synthetically non-feasible are not intended to be introduced into such group.

Where any variable (such as $R^a$, $R^b$) appears more than once in the constitution or structure of a compound, its definition in each case is independent. For example, if a group is substituted with two $R^b$, then each $R^b$ has an independent option.

When the number of a linking group is 0, such as $-(CH_2)_0-$, it means that the linking group is a bond.

When one of the variables is selected from a chemical bond or absent, it means that the two groups to which it is attached are directly connected. For example, when L represents a bond in A-L-Z, it means that the structure is actually A-Z.

When the linking direction of the linking group referred to herein is not indicated, the linking direction is arbitrary. For example, in the case where $L^1$ in the structural unit

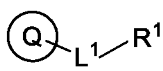

is selected from "$C_1$-$C_3$alkylene-O", $L^1$ can connect ring Q and $R^1$ in the same direction as the reading order from left to right to form "ring Q-$C_1$-$C_3$alkylene-O-$R^1$", and can also connect ring Q and $R^1$ in the opposite direction as the reading order from left to right to form "ring Q-O-$C_1$-$C_3$alkylene-$R^1$".

$C_m$-$C_n$ herein means that it has an integer number of carbon atoms, wherein the integer number is within the range of m-n. For example, "$C_1$-$C_{10}$" means that the group may have 1, 2, 3, 4, 5, 6, 7, 8, 9 or 10 carbon atoms.

The term "alkyl" refers to a hydrocarbon group of general formula $C_nH_{2n+1}$, which may be linear or branched. The term "$C_1$-$C_6$ alkyl" is to be understood as denoting a linear or branched, saturated hydrocarbon group having 1, 2, 3, 4, 5 or 6 carbon atoms. The alkyl includes, but is not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl. The term "$C_1$-$C_3$

alkyl" refers to an alkyl containing 1 to 3 carbon atoms, such as methyl, ethyl, n-propyl, or isopropyl.

The "$C_1$-$C_6$ alkyl" described herein may further comprise "$C_1$-$C_3$ alkyl".

The term "alkynyl" refers to a linear or branched, unsaturated aliphatic hydrocarbon group consisting of carbon atoms and hydrogen atoms and having at least one triple bond. For example, the term "$C_2$-$C_6$ alkynyl" is to be understood as a linear or branched hydrocarbon group, comprising one or more triple bonds and having 2, 3, 4, 5 or 6 carbon atoms. Examples of the "$C_2$-$C_6$ alkynyl" include, but are not limited to, ethynyl (-C=CH), prop-1-ynyl (1-propynyl, $-C\equiv CCH_3$), prop-2-ynyl ($-CH_2C\equiv CH$), but-1-ynyl, but-2-ynyl or but-3-ynyl. The "$C_2$-$C_6$ alkynyl" may include "$C_2$-$C_3$ alkynyl", and examples of the "$C_2$-$C_3$ alkynyl" include ethynyl (-C=CH), prop-1-ynyl (1-propynyl, - $C\equiv CCH_3$), or prop-2-ynyl ($-CH_2C\equiv CH$).

The term "cycloalkyl" refers to a carbocyclic group that is fully saturated and exists as a monocyclic ring, fused ring, bridged ring or spirocyclic ring and other forms. The term "$C_3$-$C_6$ cycloalkyl" refers to a cycloalkyl group having 3, 4, 5 or 6 ring carbon atoms. Specific examples include, but are not limited to, cyclopropyl, cyclobutyl, cyclopentyl, or cyclohexyl.

The term "cycloalkenyl" refers to a non-aromatic carbocyclic group that is not fully saturated, has at least one carbon-carbon double bond and exists as a monocyclic ring, fused ring, bridged ring or spirocyclic ring and other forms. Unless otherwise indicated, the carbocyclic group is typically a 5- to 8-membered ring. The term "$C_5$-$C_7$ cycloalkenyl" refers to a cycloalkenyl group having 5, 6 or 7 ring atoms. Specific examples include, but are not limited to, cyclopentenyl, cyclopentadienyl, cyclohexenyl, cyclohexadienyl, cycloheptenyl, or cycloheptadienyl. The term "$C_5$-$C_7$ cycloalkenyl" may include ranges of such as "$C_5$-$C_6$ cycloalkenyl". The term "$C_5$-$C_6$ cycloalkenyl" refers to a cycloalkenyl group having 5 or 6 ring atoms. Specific examples include, but are not limited to, cyclopentenyl, cyclopentadienyl, cyclohexenyl, or cyclohexadienyl.

The term "heterocyclyl" refers to a fully saturated or partially saturated monocyclic ring, fused ring, spirocyclic ring or bridged ring group, the ring atoms therein containing 1-5 (e.g., 1-3 or 1-2) heteroatoms or heteroatomic groups (namely, atomic groups containing heteroatoms). The "heteroatoms or heteroatomic groups" include, but are not limited to, nitrogen atoms (N), oxygen atoms (O), sulfur atoms (S), phosphorus atoms (P), boron atoms (B), $-S(=O)_2-$, -S(=O)-, $-P(=O)_2-$, -P(=O)-, -NH-, -S(=O)(=NH)-, -C(=O)NH-, or -NHC(=O)NH-. The term "4- to 7-membered heterocyclyl" refers to a heterocyclyl group having 4, 5, 6 or 7 ring atoms, wherein the ring atoms contain 1-3 heteroatoms or heteroatomic groups which are independently selected from the above-mentioned heteroatoms or heteroatomic groups. Examples of 4-membered heterocyclyl include, but are not limited to azetidinyl, oxetanyl; examples of 5-membered heterocyclyl include, but are not limited to tetrahydrofuryl, dioxolyl, pyrrolidyl, imidazolidinyl, pyrazolidinyl, pyrrolinyl, 4,5-dihydrooxazole or 2,5-dihydro-1H-pyrrolyl; examples of 6-membered heterocyclyl include, but are not limited to tetrahydropyranyl, piperidyl, morpholinyl, dithianyl, thiomorpholinyl, piperazinyl, trithianyl, tetrahydropyridyl or 4H-[1,3,4]thiadiazinyl; examples of 7-membered heterocyclyl include, but are not limited to diazepanyl. The "4- to 7-membered heterocyclyl" may include ranges of such as "4- to 7-membered heterocycloalkyl", "5- to 6-membered heterocyclyl" and "5- to 6-membered heterocycloalkyl".

The term "5- to 6-membered heteroaryl" refers to an aromatic ring group having 5 or 6 ring atoms, and containing 1-3 heteroatoms, such as 1-2 heteroatoms independently selected from N, O and S. Examples of 5- to 6-membered heteroaryl include, but are not limited to thienyl, furyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidyl, pyrazinyl, or triazinyl.

The term "halo" or "halogen" refers to fluoro, chloro, bromo and iodo.

The term "therapeutically effective amount" means an amount of a compound of the present disclosure that (i) treats a particular disease, condition, or disorder, (ii) attenuates, ameliorates, or eliminates one or more symptoms of a particular disease, condition, or disorder, or (iii) delays the onset of one or more symptoms of a particular disease, condition, or disorder described herein. The amount of the compound of the present disclosure which constitutes a "therapeutically effective amount" will vary depending on the compound, the disease states and the severity thereof, the manner of administration, and the age of the mammal to be treated, but can be determined routinely by those skilled in the art according to their own knowledge and the present disclosure.

The term "preventing" refers to the administration of the compound or preparation of the present disclosure for preventing diseases or one or more symptoms associated with the diseases and comprises the prevention of the occurrence of diseases or conditions in individuals (e.g., mammals), particularly when such individuals (e.g., mammals) are susceptible to the conditions, but have not been diagnosed with the conditions.

The term "pharmaceutically acceptable" refers to compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The term "pharmaceutically acceptable salt" refers to salts of pharmaceutically acceptable acids or bases, including salts formed between compounds and inorganic or organic acids, and salts formed between compounds and inorganic or organic bases.

The term "pharmaceutical composition" refers to a mixture of one or more of the compounds or the salts thereof according to the present disclosure and a pharmaceutically acceptable adjuvant. The pharmaceutical composition is intended to facilitate administering the compound of the present disclosure to an organism.

The term "pharmaceutically acceptable adjuvant" refers to adjuvants which have no significant irritating effect on the organism and do not impair the bioactivity and properties of the active compound. Suitable adjuvants are well known to those skilled in the art, such as a carbohydrate, a wax, a water-soluble and/or water-swellable polymer, a hydrophilic or hydrophobic material, gelatin, an oil, a solvent, and water.

The term "patient" includes mammals and non-mammals. Examples of mammals include, but are not limited to any member of the class Mammalia: humans, non-human primates (such as chimpanzees and other apes and monkeys); livestock, such as cattle, horses, sheep, goats and pigs; domestic animals, such as rabbits, dogs and cats; laboratory animals, including rodents, such as rats, mice and guinea pigs. Examples of non-human mammals include, but are not limited to birds and fishes. In one embodiment of the method and composition provided herein, the mammal is a human. The terms "patient" and "individual" are used interchangeably.

The word "comprise" and its variants such as "comprises" or "comprising" are to be understood as an open, non-exclusive meaning, i.e., "including but not limited to".

The present disclosure also includes isotopically-labeled compounds of the present disclosure which are identical to those recited herein, but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, respectively.

Certain isotopically-labeled compounds of the present disclosure (e.g., those labeled with $^{3}H$ and $^{14}C$) are useful in tissue distribution assays of compounds and/or substrates. Tritiated (i.e., $^{3}H$) and carbon-14 (i.e., $^{14}C$) isotopes are particularly preferred for their ease of preparation and detectability. Positron emitting isotopes such as $^{15}O$, $^{13}N$, $^{11}C$, and $^{18}F$ are useful for positron emission tomography (PET) studies to examine substrate occupancy. The isotopically-labeled compounds of the present disclosure can generally be prepared according to following procedures analogous to those disclosed in the Schemes and/or in the Examples herein below by substituting a non-isotopically-labeled reagent with an isotopically-labeled reagent.

The pharmaceutical composition of the present disclosure may be prepared by combining the compound of the present disclosure with an appropriate pharmaceutically acceptable adjuvant. For example, the pharmaceutical composition of the present disclosure may be formulated into solid, semi-solid, liquid or gaseous preparations, such as tablets, pills, capsules, powders, granules, ointments, emulsions, suspensions, suppositories, injections, inhalants, gels, microspheres and aerosols.

Typical administration routes of the compound, or the stereoisomer or pharmaceutically acceptable salt thereof, or the pharmaceutical composition thereof of the present disclosure include, but are not limited to oral administration, rectal administration, topical administration, administration by inhalation, parenteral administration, sublingual administration, intravaginal administration, intranasal administration, intraocular administration, intraperitoneal administration, intramuscular administration, subcutaneous administration, and intravenous administration.

The pharmaceutical composition of the present disclosure can be manufactured by using well-known methods in the art, such as conventional mixing method, dissolution method, granulation method, emulsification method, and freeze-drying method.

In some embodiments, the pharmaceutical composition is in oral form. For oral administration, the pharmaceutical composition may be formulated by mixing the active compound with a pharmaceutically acceptable adjuvant well-known in the art. Such adjuvants enable the compounds of the present disclosure to be formulated into tablets, pills, lozenges, dragees, capsules, liquids, gels, syrups, suspensions, etc., for oral administration to patients.

A solid oral composition can be prepared by a conventional mixing, filling or tableting method. For example, it can be obtained by mixing the active compound with a solid adjuvant, optionally grinding the resulting mixture, adding other suitable adjuvants, if necessary, and then processing the mixture into granules to obtain cores of tablets or dragees. Suitable adjuvants include, but are not limited to, binders, diluents, disintegrants, lubricants, glidants, sweeteners or flavoring agents.

The pharmaceutical composition can also be suitable for parenteral administration, such as sterile solutions, suspensions or lyophilized products in a suitable unit dosage form.

The daily administration dose of the compound of general formula I in all the administration manners described herein is from 0.01 mg/kg body weight to 200 mg/kg body weight, preferably from 0.05 mg/kg body weight to 50 mg/kg body weight, and more preferably from 0.1 mg/kg body weight to 30 mg/kg body weight, in the form of a single dose or divided doses.

## Detailed Description of Embodiments

The embodiments of the present disclosure will be described in detail with examples, but not imply any adverse limitation to the present disclosure. The embodiments of the present disclosure have been described in detail herein, and the specific embodiments thereof are also disclosed. Various changes and improvements to the specific embodiments of the present disclosure would be obvious to those skilled in the art without departing from the spirit and scope of the present invention. All reagents used in the present disclosure are commercially available and can be used without further purification.

Unless otherwise specified, the ratios indicated for mixed solvents are volume mixing ratios.

Unless otherwise specified, % refers to wt%.

Compounds are named by hand or ChemDraw® software, and commercially available compounds are named by the supplier catalog names.

The structures of the compounds are determined by nuclear magnetic resonance (NMR) and/or mass spectrometry (MS). The NMR shifts are calculated in $10^{-6}$ (ppm). The solvents for NMR analysis are deuterated dimethyl sulfoxide, deuterated chloroform, deuterated methanol, etc., and the internal standard is tetramethylsilane (TMS); the "$IC_{50}$" refers to the half inhibitory concentration, the concentration at which half of the maximal inhibitory effect is achieved.

The eluent or mobile phase herein may be formed from two or more solvents to form a mixed eluent or mobile phase, and the ratio of which is the volume ratio of the solvents. For example, the "0 to 10% methanol/dichloromethane" means that the volume ratio of methanol to dichloromethane in the mixed eluent is 0 : 100 to 10 : 100 during gradient elution.

### Example 1. (*S*)-*N*-((8-ethyl-8-hydroxy-9,12-dioxo-2,3,8,9,12,14-hexahydro-11*H*-[1,4]dioxino[2,3-g]pyrano[3',4':6,7]indolizino[1,2-*b*]quinolin-15-yl)methyl)-2-hydroxyacetamide (Compound 1)

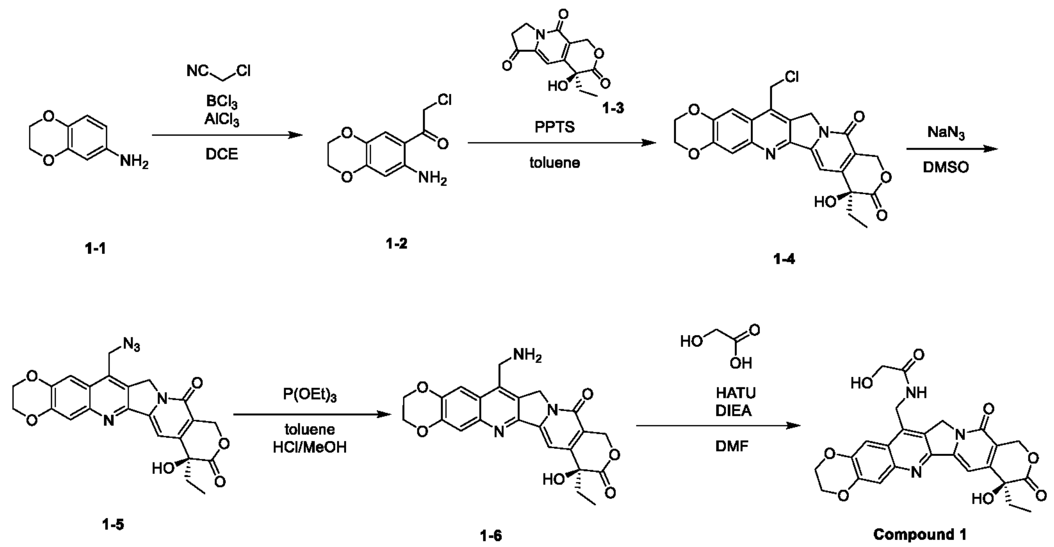


### Step 1: Synthesis of 1-(7-amino-2,3-dihydrobenzo[*b*][1,4]dioxin-6-yl)-2-chloroethan-1-one (Intermediate 1-2)

**Reactant 1-1** (500 mg, 3.31 mmol) was dissolved in 1,2-dichloroethane (3 mL). The reaction solution was cooled to 0 °C, and to which were added boron trichloride (1 M, 2.65 mL) and aluminum trichloride (573.36 mg, 4.30 mmol). Chloroacetonitrile (299.67 mg, 3.97 mmol) was added to the reaction solution at 0°C under nitrogen protection, and the reaction solution was stirred at 90°C under nitrogen protection for 16 h. LC-MS detection showed that the reaction was completed. Ice water (30 mL) and 1 N HCl (10 mL) were added successively after the reaction solution was cooled to room temperature, and then the resulting mixture was stirred for 30 min. Dichloromethane (30 mL*3) was added to the reaction solution for extracting same 3 times. The organic phases were combined, and washed with saturated brine (30 mL). The washed organic phase was dried over an appropriate amount of anhydrous sodium sulfate. The organic phase was concentrated to dryness under reduced pressure, and the crude product was subjected to preparative thin layer chromatography (silica, petroleum ether:ethyl acetate = 9:1) to obtain the title compound (210 mg).

MS m/z (ESI): 228.0 $[M+H]^+$.

**Step 2: Synthesis of (*S*)-15-(chloromethyl)-8-ethyl-8-hydroxy-2,3,11,14-tetrahydro-12*H*-[1,4]dioxino[2,3-*g*] pyrano[3',4':6,7]indolizino[1,2-*b*]quinoline-9,12(8H)-dione (Intermediate 1-4)**

**Intermediate 1-2** (100 mg, 439.28 μmol) and **Intermediate 1-3** (115.64 mg, 439.28 μmol) were dissolved in anhydrous toluene (3 mL), and to the mixture was added pyridinium p-toluenesulfonate (PPTS, 22.08 mg, 87.86 μmol). The reaction solution was stirred at 100°C under nitrogen protection for 16 h. LC-MS detection showed that the reaction was completed. The reaction solution was filtered after the reaction solution was cooled to room temperature. The filter cake was washed with ethanol (5 mL*2) to obtain the crude of the title compound (130 mg).

MS m/z (ESI): 455.1 $[M+H]^+$.

**Step 3: Synthesis of (*S*)-15-(azidomethyl)-8-ethyl-8-hydroxy-2,3,11,14-tetrahydro-12*H*-[1,4]dioxino[2,3-*g*] pyrano[3',4':6,7]indolizino[1,2-*b*]quinoline-9,12(8*H*)-dione (Intermediate 1-5)**

**Intermediate 1-4** (120 mg, 263.82 μmol) was dissolved in dimethyl sulfoxide (1 mL), and to the mixture was added sodium azide (25.73 mg, 395.73 μmol). The reaction solution was stirred at 25°C under nitrogen protection for 3 h. LC-MS detection showed that the reaction was completed. Ice water (2 mL) was added to the reaction solution, and the resulting mixture was stirred for 0.5 h, and filtered to obtain the crude of the title compound (90 mg).

MS m/z (ESI): 462.1 $[M+H]^+$.

**Step 4: Synthesis of (*S*)-15-(aminomethyl)-8-ethyl-8-hydroxy-2,3,11,14-tetrahydro-12*H*-[1,4]dioxino[2,3-*g*] pyrano[3',4':6,7]indolizino[1,2-*b*]quinoline-9,12(8*H*)-dione (Intermediate 1-6)**

**Intermediate 1-5** (90 mg, 195.05 μmol) was dissolved in anhydrous toluene (1 mL), and to the mixture was added triethyl phosphite (81.02 mg, 487.62 μmol). The reaction solution was stirred at 100°C under nitrogen protection for 3 h. The reaction solution was cooled to 25°C, and to which was added hydrogen chloride methanol solution (0.5 mL). The reaction solution was stirred at 85°C under nitrogen protection for 16 h. LC-MS detection showed that the reaction was completed. After the reaction solution was cooled to room temperature, filtration was performed to obtain the title compound (18 mg).

MS m/z (ESI): 436.1 $[M+H]^+$.

**Step 5: Synthesis of (S)-N-((8-ethyl-8-hydroxy-9,12-dioxo-2,3,8,9,12,14-hexahydro-11*H*-[1,4]dioxino[2,3-*g*] pyirano[3',4':6,7]indolizino[1,2-*b*]quinolin-15-yl)methyl)-2-hydroxyacetamide (Compound 1)**

**Intermediate 1-6** (18 mg, 41.34 μmol) and 2-hydroxyacetic acid (15.72 mg, 206.69 μmol) were dissolved in anhydrous *N*,*N*-dimethylformamide (1 mL), and to the mixture were added 2-(7-azabenzotriazol-1-yl)-*N*,*N*,*N'*,*N'*-tetramethyluronium hexafluorophosphate (HATU) (23.58 mg, 62.01 μmol) and *N*,*N*-diisopropylethylamine (DIEA) (16.03 mg, 124.02 μmol). The reaction solution was stirred at 25°C for 3 h. LC-MS detection showed that the reaction was completed. The reaction solution was filtered, and purified by preparative high performance liquid chromatography (YMC-Actus Triart C18 column, 5 μm silica, 25 mm in diameter, 100 mm in length; using a decreasingly polar mixture of water (containing 0.05% formic acid) and acetonitrile as the eluent; acetonitrile gradient proportion: 10%-30%, elution time: 12 min) to obtain the title compound (7 mg).

MS m/z (ESI): 494.1 $[M+H]^+$.

**$^1$H NMR (400MHz, DMSO-$d_6$)** δ = 8.69 (t, *J* = 6.0 Hz, 1H), 7.92 (s, 1H), 7.56 (s, 1H), 7.26 (s, 1H), 6.49 (s, 1H), 5.57 (t, *J* = 5.7 Hz, 1H), 5.46 (s, 2H), 5.43 (s, 2H), 4.74 (d, *J* = 6.0 Hz, 2H), 4.44 (s, 4H), 3.82 (d, *J* = 5.6 Hz, 2H), 1.94-1.80 (m, 2H), 0.88 (m, 3H).

**Example 2. (*S*)-II-(aminomethyl)-9-chloro-4-ethyl-8-fluoro-4-hydroxy-1,12-dihydro-14*H*-pyrano[3',4':6,7]indolizino[1,2-b]quinoline-3,14(4*H*)-dione (Compound 2)**

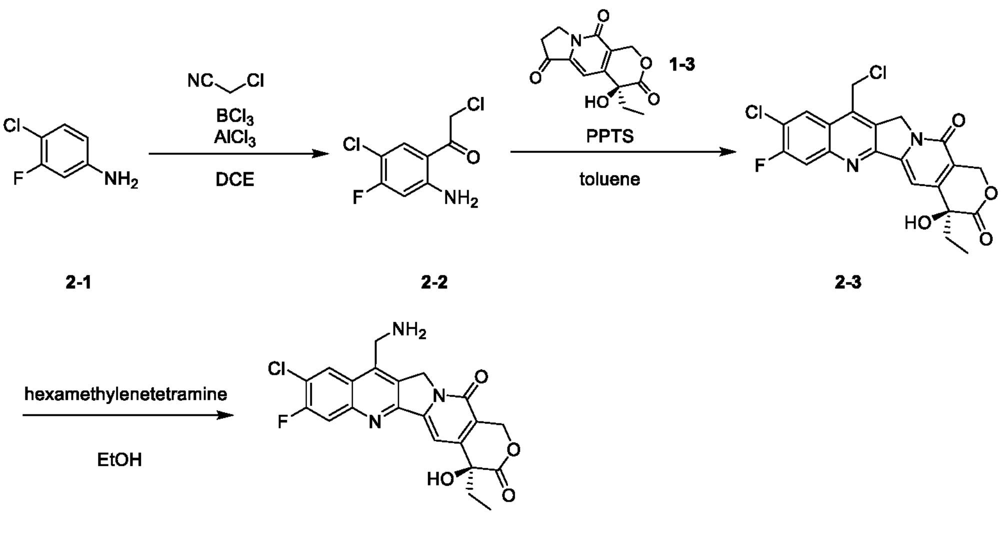


2-1 2-2 2-3

Compound 2

**Step 1: Synthesis of 1-(2-amino-5-chloro-4-fluorophenyl)-2-chloroethan-1-one (Intermediate 2-2)**

Boron trichloride (1 M, 13.74 mL) was dissolved in 1,2-dichloroethane (24 mL). The reaction solution was cooled to 0°C, and to which were added **Reactant 2-1** (2 g, 13.74 mmol) and chloroacetonitrile (1.56 g, 20.61 mmol). The reaction solution was stirred at 0°C for 10 min, and to which was added aluminum trichloride (2.38 g, 17.86 mmol). The reaction solution was then warmed to 25°C under nitrogen protection and stirred for 10 min. The reaction solution was stirred at 90°C under nitrogen protection for 18 h. LC-MS detection showed that the reaction was completed. Ice water (50 mL) and 5% HCl (10 mL) were added successively and slowly, and stirred at 25°C for 30 min after the reaction solution was cooled to room temperature. Then dichloromethane (50 mL) was added. The organic phase was washed with water (2 mL*2), and the washed organic phase was dried over an appropriate amount of anhydrous sodium sulfate. The organic phase was concentrated to dryness under reduced pressure, and the crude product was purified by preparative high performance liquid chromatography (YMC-Actus Triart C18 column, 5 μm silica, 25 mm in diameter, 100 mm in length; using a decreasingly polar mixture of water (containing 0.05% formic acid) and acetonitrile as the eluent; acetonitrile gradient proportion: 40%-60%, elution time: 10 min) to obtain the title compound (320 mg).

MS m/z (ESI): 222.0 $[M+H]^+$.

**Step 2: Synthesis of (*S*)-9-chloro-11-(chloromethyl)-4-ethyl-8-fluoro-4-hydroxy-1,12-dihydro-14*H*-pyrano[3',4':6,7]indolizino[1,2-*b*]quinoline-3,14(4*H*) - dione (Intermediate 2-3)**

**Intermediate 2-2** (220 mg, 990.80 μmol) and **Intermediate 1-3** (273.86 mg, 1.04 mmol) were dissolved in toluene (2 mL), and to the mixture was added pyridinium p-toluenesulfonate (24.90 mg, 99.08 μmol). The reaction solution was stirred at 100°C for 18 h. LC-MS detection showed that the reaction was completed. Ethanol (1 mL) was added after the reaction solution was cooled to room temperature. The reaction solution was stirred at 25°C for 0.5 h. The reaction solution was filtered. The filter cake was washed with ethanol (2 mL*2) to obtain the crude of the title compound (270 mg).

MS m/z (ESI): 449.0 $[M+H]^+$.

**Step 3: Synthesis of (*S*)-11-(aminomethyl)-9-chloro-4-ethyl-8-fluoro-4-hydroxy-1,12-dihydro-14*H*-pyrano[3',4':6,7]indolizino[1,2-*b*]quinoline-3,14(4*H*) - dione (Compound 2)**

**Intermediate 2-3** (50 mg, 111.29 μmol) was dissolved in ethanol (1 mL), and to the mixture was added hexamethylenetetramine (23.40 mg, 166.94 μmol). The reaction solution was stirred at 90°C for 1.5 h. LC-MS detection showed that the reaction was completed. The reaction solution was concentrated to dryness under reduced pressure after the reaction solution was cooled to room temperature, and purified by preparative high performance liquid chromatography (YMC-Actus Triart C18 column, 5 μm silica, 25 mm in diameter, 100 mm in length; using a decreasingly polar mixture of water (containing 0.225% formic acid) and methanol as the eluent; methanol gradient proportion: 0%-30%, elution time: 12 min) to obtain the title compound (22 mg).

MS m/z (ESI): 430.1 $[M+H]^+$.

**$^1$H NMR (400MHz, DMSO-$d_6$)** $\delta$ = 8.71 (d, *J* = 8.0 Hz, 1H), 8.23 (d, *J* = 10.3 Hz, 1H), 8.14 (s, 0.3H, HCOOH),

7.36 (s, 1H), 6.57 (s, 1H), 5.52 (s, 2H), 5.46 (s, 2H), 4.55 (s, 2H), 1.93-1.84 (m, 2H), 0.90-0.85 (m, 3H).

**Example 3. (*S*)-*N*-((9-chloro-4-ethyl-8-fluoro-4-hydroxy-3,14-dioxo-3,4,12, 14-tetrahydro-1*H*-pyrano[3',4':6,7]indolizino[1,2-*b*]quinolin-11-yl)methyl)-2-hydroxyacetamid (Compound 3)**

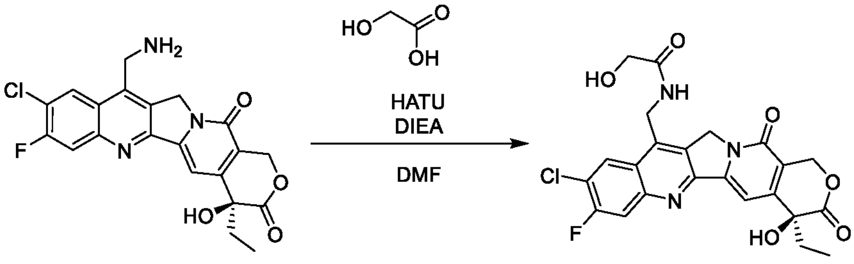


**Compound 2** **Compound 3**

**Compound 2** (22 mg, 51.18 μmol) and 2-hydroxyacetic acid (19.46 mg, 255.92 μmol) were dissolved in anhydrous *N*,*N*-dimethylformamide (1 mL), and to the mixture were added HATU (29.19 mg, 76.77 μmol) and *N*,*N*-diisopropylethylamine (19.84 mg, 153.55 μmol). The reaction solution was stirred at 25°C for 1.5 h. LC-MS detection showed that the reaction was completed. The reaction solution was filtered, and concentrated to dryness under reduced pressure, and the crude product was purified by preparative high performance liquid chromatography (YMC-Actus Triart C18 column, 5 μm silica, 25 mm in diameter, 100 mm in length; using a decreasingly polar mixture of water (containing 0.05% formic acid) and acetonitrile as the eluent; acetonitrile gradient proportion: 10%-40%, elution time: 12 min) to obtain the title compound (2.20 mg).

MS m/z (ESI): 488.1 $[M+H]^+$.

**$^1$H NMR (400MHz, DMSO-$d_6$)** δ = 8.90-8.85 (m, 2H), 8.20 (d, *J* = 10.3 Hz, 1H), 7.35 (s, 1H), 6.55 (s, 1H), 5.60 (t, *J* = 5.7 Hz, 1H), 5.56 (s, 2H), 5.45 (s, 2H), 4.83 (d, *J* = 6.0 Hz, 2H), 3.83 (d, *J*=5.8 Hz, 2H), 1.93-1.81 (m, 2H), 0.88 (m, 3H).

**Example 4. (*S*)-11-(aminomethyl)-9-bromo-4-ethyl-8-fluoro-4-hydroxy-1,12-dihydro-14*H*-pyrano[3',4':6,7]indolizino[1,2-*b*]quinoline-3,14(4*H*)-dione (Compound 4)**

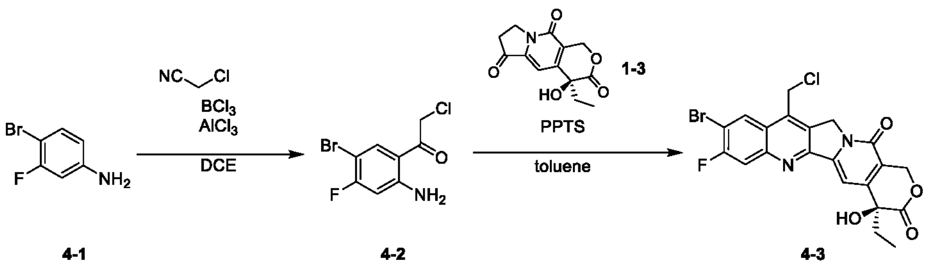


**4-1** **4-2** **4-3**

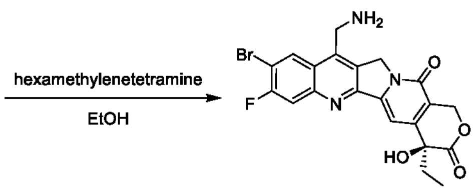


**Compound 4**

**Step 1: Synthesis of 1-(2-amino-5-bromo-4-fluorophenyl)-2-chloroethan-1-one (Intermediate 4-2)**

Boron trichloride (1 M, 10.53 mL) was dissolved in 1,2-dichloroethane (24 mL). The reaction solution was cooled to 0°C, and to which were added **Intermediate 4-1** (2 g, 10.53 mmol) and chloroacetonitrile (1.19 g, 15.79 mmol). The reaction solution was stirred at 0°C for 10 min, and to which was added aluminum trichloride (1.82 g, 13.68 mmol). The reaction solution was stirred at 25°C under nitrogen protection for 10 min, and then warmed to 90°C and stirred for 18 h. LC-MS detection showed that the reaction was completed. Ice water (50 mL) and 5% HCl (10 mL) were added successively and slowly, and stirred at 25°C for 30 min after the reaction solution was cooled to room temperature. Then dichloromethane (50 mL) was added. The organic phase was washed with water (2 mL*2), and the washed organic phase was dried over an appropriate amount of anhydrous sodium sulfate. The organic phase was purified by preparative high performance liquid chromatography (YMC-Actus Triart C18 column, 5 μm silica, 25 mm in diameter, 100 mm in length; using a decreasingly polar mixture of water (containing 0.05% formic acid) and acetonitrile as the eluent; acetonitrile gradient proportion: 39%-49%, elution time: 12 min) to obtain the title compound (380 mg).

MS m/z (ESI): 265.9 $[M+H]^+$.

**Step 2: Synthesis of (*S*)-9-bromo-11-(chloromethyl)-4-ethyl-8-fluoro-4-hydroxy-1,12-dihydro-14*H*-pyrano[3',4':6,7]indolizino[1,2-*b*]quinoline-3,14(4*H*) - dione (Intermediate 4-3)**

**Intermediate 4-2** (200 mg, 750.48 μmol) and **Intermediate 1-3** (207.44 mg, 788.01 mmol) were dissolved in anhydrous toluene (4 mL), and to the mixture was added pyridinium p-toluenesulfonate (22.63 mg, 90.06 μmol). The reaction solution was stirred at 100°C for 18 h. LC-MS detection showed that the reaction was completed. Ethanol (1 mL) was added after the reaction solution was cooled to room temperature. The reaction solution was stirred at 25°C for 0.5 h. The reaction solution was filtered. The filter cake was washed with ethanol (2 mL*2) to obtain the crude of the title compound (200 mg).

MS m/z (ESI): 493.0 $[M+H]^+$.

**Step 3: Synthesis of (S)-11-(aminomethyl)-9-bromo-4-ethyl-8-fluoro-4-hydroxy-1,12-dihydro-14*H*-pyrano[3',4':6,7]indolizino[1,2-*b*]quinoline-3,14(4*H*) - dione (Compound 4)**

**Intermediate 4-3** (200 mg, 405.10 μmol) was dissolved in ethanol (4 mL), and to the mixture was added hexamethylenetetramine (113.58 mg, 810.19 μmol). The reaction solution was stirred at 90°C for 1.5 h. LC-MS detection showed that the reaction was completed. The reaction solution was concentrated to dryness under reduced pressure after the reaction solution was cooled to room temperature. The crude product was purified by preparative high performance liquid chromatography (YMC-Actus Triart C18 column 5 μm, 25 mm in diameter, 100 mm in length; using a decreasingly polar mixture of water (containing 0.225% formic acid) and acetonitrile as the eluent; acetonitrile gradient proportion: 6%-26%, elution time: 12 min) to obtain the title compound (30 mg).

MS m/z (ESI):476.0 $[M+H]^+$.

**$^1$H NMR (400MHz, DMSO-$d_6$)** δ = 8.83 (d, *J* = 7.4 Hz, 1H), 8.18 (d, *J* = 9.8 Hz, 1H), 8.14 (s, 0.4H, HCOOH), 7.36 (s, 1H), 6.57 (s, 1H), 5.52 (s, 2H), 5.46 (s, 2H), 4.53 (s, 2H), 1.92-1.85 (m, 2H), 0.88 (t, *J* = 7.3 Hz, 3H).

**Example 5. (*S*)-*N*-((9-bromo-4-ethyl-8-fluoro-4-hydroxy-3,14-dioxo-3,4, 12,14-tetrahydro-1*H*-pyrano[3',4':6,7]indolizino[1,2-*b*]quinolin-11-yl)methyl)-2-hydroxyacetamide (Compound 5)**

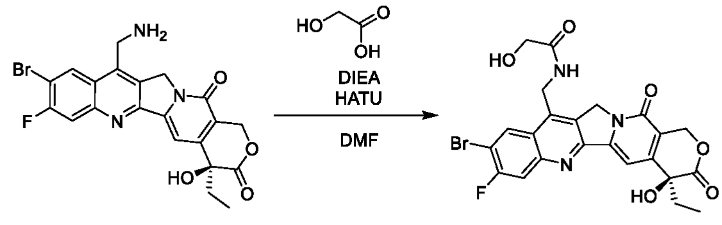


**Compound 4** **Compound 5**

**Compound 4** (15 mg, 26.88 μmol) and 2-hydroxyacetic acid (10.22 mg, 134.41 μmol) were dissolved in anhydrous *N,N*-dimethylformamide (1 mL), and to the mixture were added HATU (15.33 mg, 40.32 μmol) and *N,N*-diisopropylethylamine (10.42 mg, 80.65 μmol). The reaction solution was stirred at 25°C for 1 h. LC-MS detection showed

that the reaction was completed. The reaction solution was filtered, and concentrated to dryness under reduced pressure, and the crude product was purified by preparative high performance liquid chromatography (YMC-Actus Triart C18 column 5 μm, 25 mm in diameter, 100 mm in length; using a decreasingly polar mixture of water (containing 0.05% formic acid) and acetonitrile as the eluent; acetonitrile gradient proportion: 6%-36%, elution time: 12 min) to obtain the title compound (2.09 mg).

MS m/z (ESI): 532.0 $[M+H]^+$.

**$^1$H NMR (400MHz, DMSO-$d_6$)** δ = 9.00 (d, *J* = 7.5 Hz, 1H), 8.86 (t, *J* = 5.9 Hz, 1H), 8.15 (d, *J* = 9.8 Hz, 1H), 7.35 (s, 1H), 6.54 (s, 1H), 5.60 (t, *J* = 5.7 Hz, 1H), 5.56 (s, 2H), 5.45 (s, 2H), 4.83 (d, *J* = 5.8 Hz, 2H), 3.83 (d, *J* = 5.9 Hz, 2H), 1.94-1.82 (m, 2H), 0.88 (t, *J* = 7.4 Hz, 3H).

**Example 6. (*S*)-*N*-((8-chloro-4-ethyl-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxyacetamide (Compound 6)**

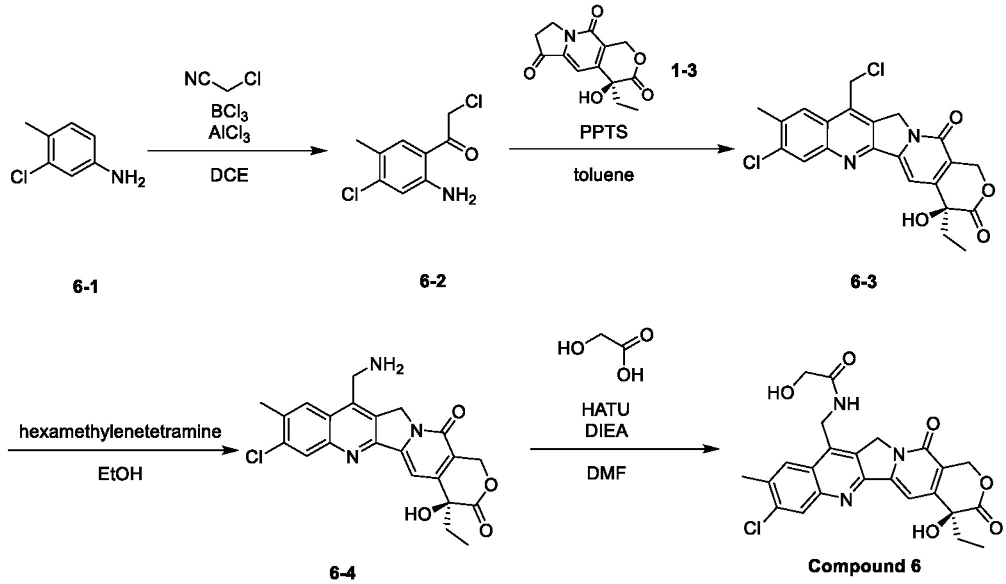

6-1
6-2
6-3
6-4
Compound 6

**Step 1: Synthesis of 1-(2-amino-4-chloro-5-methylphenyl)-2-chloroethan-1-one (Intermediate 6-2)**

Boron trichloride (1 M, 2.82 mL) was dissolved in 1,2-dichloroethane (8 mL). The reaction solution was cooled to 0°C, and to which were added **Reactant 6-1** (0.5 g, 3.53 mmol) and chloroacetonitrile (319.91 g, 4.24 mmol). The reaction solution was stirred at 0°C for 10 min, and to which was added aluminum trichloride (612.09 mg, 4.59 mmol). The reaction solution was stirred at 25°C under nitrogen protection for 10 min, and then warmed to 90°C and stirred for 18 h. LC-MS detection showed that the reaction was completed. Ice water (25 mL) and 5% HCl (5 mL) were added successively and slowly, and stirred at 25°C for 30 min after the reaction solution was cooled to room temperature. Then dichloromethane (20 mL) was added. The organic phase was washed with water (20 mL*2), and the washed organic phase was dried over an appropriate amount of anhydrous sodium sulfate. Preparative thin layer chromatography (silica, petroleum ether : ethyl acetate = 9 : 1) was performed to obtain the title compound (500 mg).

MS m/z (ESI): 218.0 $[M+H]^+$

**Step 2: Synthesis of (*S*)-8-chloro-11-(chloromethyl)-4-ethyl-4-hydroxy-9-methyl-1,12-dihydro-14*H*-pyrano[3',4':6,7]indolizino[1,2-*b*]quinoline-3,14(4*H*)-dione (Intermediate 6-3)**

**Intermediate 6-2** (250 mg, 1.15 mmol) and **Intermediate 1-3** (316.87 mg, 1.20 mmol) were dissolved in toluene (5 mL), and to the mixture was added pyridinium p-toluenesulfonate (34.57 mg, 137.56 μmol). The reaction solution was stirred at 100°C for 18 h. LC-MS detection showed that the reaction was completed. Ethanol (1 mL) was added after the reaction solution was cooled to room temperature. The reaction solution was stirred at 25°C for 0.5 h. The reaction solution was filtered. The filter cake was washed with ethanol (2 mL*2) to obtain the crude of the title compound (230 mg).

MS m/z (ESI): 445.1 $[M+H]^+$.

**Step 3: Synthesis of (*S*)-11-(aminomethyl)-8-chloro-4-ethyl-4-hydroxy-9-methyl-1,12-14*H*-pyrano[3',4':6,7]indolizino[1,2-*b*]quinoline-3,14(4*H*)-dione (Intermediate 6-4)**

**Intermediate 6-3** (49.56 mg, 111.29 μmol) was dissolved in ethanol (0.5 mL), and to the mixture was added hexamethylenetetramine (23.40 mg, 166.94 μmol). The reaction solution was stirred at 90°C for 1.5 h. LC-MS detection showed that the reaction was completed. The reaction solution was concentrated to dryness under reduced pressure after the reaction solution was cooled to room temperature, and purified by preparative high performance liquid chromatography (YMC-Actus Triart C18 column 5 μm, 25 mm in diameter, 100 mm in length; using a decreasingly polar mixture of water (containing 0.225% formic acid) and acetonitrile as the eluent; acetonitrile gradient proportion: 2%-32%, elution time: 12 min) to obtain the title compound (11.0 mg).

MS m/z (ESI): 426.2 $[M+H]^+$.

**Step 4: Synthesis of (*S*)-*N*-((8-chloro-4-ethyl-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxyacetamide (Compound 6)**

**Intermediate 6-4** (11 mg, 25.83 μmol) and 2-hydroxyacetic acid (9.82 mg, 129.15 μmol) were dissolved in anhydrous *N*,*N*-dimethylformamide (0.5 mL), and to the mixture were added HATU (14.73 mg, 38.74 μmol) and *N*,*N*-diisopropylethylamine (10.01 mg, 77.49 μmol). The reaction solution was stirred at 25°C for 1 h. LC-MS detection showed that the reaction was completed. The reaction solution was filtered, and concentrated to dryness under reduced pressure, and the crude product was purified by preparative high performance liquid chromatography (YMC-Actus Triart C18 column 5 μm, 25 mm in diameter, 100 mm in length; using a decreasingly polar mixture of water (containing 0.05% formic acid) and acetonitrile as the eluent; acetonitrile gradient proportion: 10%-40%, elution time: 12 min) to obtain the title compound (3.00 mg).

MS m/z (ESI): 484.1 $[M+H]^+$.

**$^1$H NMR (400MHz, DMSO-$d_6$)** δ = 8.77 (t, *J* = 6.1 Hz, 1H), 8.52 (s, 1H), 8.26 (s, 1H), 7.32 (s, 1H), 6.54 (s, 1H), 5.59 (t, *J* = 5.8 Hz, 1H), 5.52 (s, 2H), 5.44 (s, 2H), 4.85 (d, *J* = 6.0 Hz, 2H), 3.84 (d, *J* = 5.6 Hz, 2H), 2.60 (s, 3H), 1.91-1.82 (m, 2H), 0.88 (t, *J* = 7.3 Hz, 3H).

**Example 7. (*S*)-*N*-((8-ethyl-8-hydroxy-9,12-dioxo-2,3,8,9,12,14-hexahydro-1*H*,11*H*-cyclopentadieno[*f*] pyrano[3',4':6,7]indolizino[1,2-b]quinolin-15-yl)methyl)-2-hydroxyacetamide (Compound 7)**

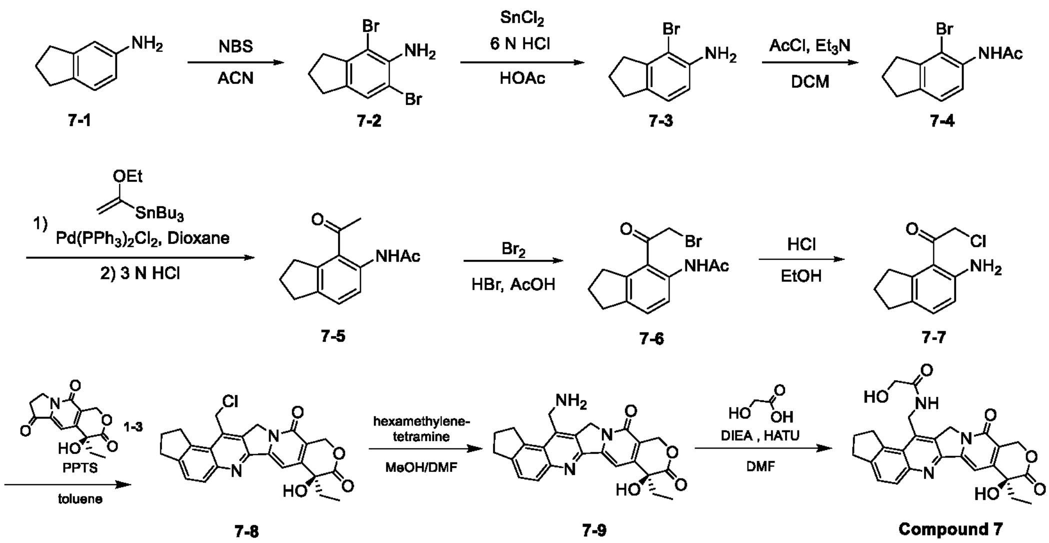


**Step 1: Synthesis of 4,6-dibromo-2,3-dihydro-1*H*-inden-5-amine (Intermediate 7-2)**

**Intermediate 7-1** (10.0 g) was dissolved in anhydrous acetonitrile, and *N*-bromosuccinimide (NBS) (27.5 g) was added in batches at 0°C, and then the mixture was stirred at room temperature overnight. The reaction solution was filtered, the filtrate was concentrated under reduced pressure, the residue was dissolved in 200 mL of ethyl acetate, and washed with water (100 mL*2). The resulting organic phase was dried over anhydrous sodium sulfate. The sample

was mixed with silica gel, then placed on diatomite, and rinsed with 500 mL of petroleum ether. The filtrate was concentrated to obtain the title compound (17.0 g).

MS m/z (ESI): 289.9 $[M+H]^+$.

**Step 2: Synthesis of 4-bromo-2,3-dihydro-1*H*-inden-5-amine (Intermediate 7-3)**

**Intermediate 7-2** (15.0 g) and stannous chloride (15.0 g) were dissolved in 75 mL of acetic acid, and 140 mL of 6 N concentrated hydrochloric acid was added. The reaction was performed at 90°C for 3 h. The reaction system was cooled to room temperature, and concentrated to remove acetic acid. The residue was dissolved in 100 mL of ethyl acetate, adjusted with a saturated aqueous sodium carbonate solution to pH of about 8, and filtered. The organic phase was separated from the filtrate, and the aqueous phase was further extracted with ethyl acetate (50 mL*3). The organic phases were combined and dried over anhydrous sodium sulfate, and filtered. The filtrate was concentrated under reduced pressure to obtain the title compound (10 g).

MS m/z (ESI): 212.0 $[M+H]^+$.

**Step 3: Synthesis of *N*-(4-bromo-2,3-dihydro-1*H*-inden-5-yl)acetamide (Intermediate 7-4)**

**Intermediate 7-3** (10.0 g) was dissolved in 100 mL of anhydrous dichloromethane, triethylamine (10.6 g) was added, and acetyl chloride (5.5 g) was added slowly dropwise at 0°C. Then the reaction system was stirred at room temperature overnight. The reaction solution was washed with water (100 mL*2). The organic phase was dried over anhydrous sodium sulfate, and filtered. The filtrate was concentrated under reduced pressure, and the residue was purified by silica gel column chromatography (petroleum ether : ethyl acetate = 90 : 10) to obtain the title compound (7.1 g).

MS m/z (ESI): 254.0 $[M+H]^+$.

**Step 4: Synthesis of *N*-(4-acetyl-2,3-dihydro-1*H*-inden-5-yl)acetamide (Intermediate 7-5)**

Under nitrogen protection, **Intermediate 7-4** (6.7 g) and tributyl(2-ethoxyethenyl)stannane (10.4 g) were dissolved in 100 mL of anhydrous 1,4-dioxane, and then bis(triphenylphosphine)palladium dichloride (1.8 g) was added. The reaction system was stirred at 100°C overnight. The reaction solution was cooled to room temperature, and 30 mL of 3 N hydrochloric acid was added. The resulting mixture was stirred at room temperature for 1 h. The reaction solution was filtered with diatomite, and the resulting filtrate was diluted with 100 mL of ethyl acetate, and washed with water (100 mL*2). The organic phase was dried over anhydrous sodium sulfate, and filtered. The filtrate was concentrated under reduced pressure, and the residue was purified by silica gel column chromatography (petroleum ether : ethyl acetate = 85 : 15) to obtain the title compound (4.7 g).

MS m/z (ESI): 218.1 $[M+H]^+$.

**Step 5: Synthesis of *N*-(4-(2-bromoacetyl)-2,3-dihydro-1*H*-inden-5-yl)acetamide (Intermediate 7-6)**

**Intermediate 7-5** (4.7 g) was dissolved in 50 mL of acetic acid, 7.3 g of a 33% hydrobromic acid-acetic acid solution was added, and bromine (2.85 g) was added slowly dropwise at room temperature. Then the resulting mixture was stirred and reacted at room temperature for another 3 h. After the reaction was completed, the reaction solution was poured into ice water, stirred until a large amount of solids precipitated, and filtered. The filter cake was washed with petroleum ether, and the resulting solid was dried to obtain the title compound (5.0 g).

MS m/z (ESI): 296.0 $[M+H]^+$.

**Step 6: Synthesis of 1-(5-amino-2,3-dihydro-1*H*-inden-4-yl)-2-chloroethan-1-one (Intermediate 7-7)**

**Intermediate 7-6** (5.0 g) was dissolved in 30 mL of ethanol, and 35 mL of 6 N concentrated hydrochloric acid was added. The reaction system was stirred at 80°C for 2 h. The reaction system was cooled to room temperature, and concentrated under reduced pressure to remove the solvent. The residue was dissolved in 100 mL of dichloromethane, adjusted with a saturated aqueous sodium bicarbonate solution to pH of about 7. The organic phase was separated, and the aqueous phase was further extracted with dichloromethane (50 mL*2). The organic phases were combined and dried over anhydrous sodium sulfate, and filtered. The filtrate was concentrated under reduced pressure, and the residue was purified by silica gel column chromatography (petroleum ether : ethyl acetate = 75 : 25) to obtain the title compound (840 mg).

MS m/z (ESI): 210.0 $[M+H]^+$.

**Step 7: Synthesis of (*S*)-15-(chloromethyl)-8-ethyl-8-hydroxy-1,2,3,8,11, 14-hexahydro-9*H*,12*H*-cyclopency-clopentadieno[*f*]pyrano[3',4':6,7]indolizino [1,2-*b*]quinoline - 9,12-dione (Intermediate 7-8)**

**Intermediate** 7-7 (100 mg) and **Intermediate 1-3** (125.55 mg) were dissolved in toluene (5 mL), and to the mixture was added pyridinium p-toluenesulfonate (5.99 mg). The reaction solution was stirred at 90°C for 18 h. Ethanol (1 mL) was added after the reaction solution was cooled to room temperature. The reaction solution was stirred at 25°C for 0.5 h. The reaction solution was filtered. The filter cake was washed with petroleum ether (2 mL*2) to obtain the title compound (180 mg).

MS m/z (ESI): 437.0 $[M+H]^+$.

**Step 8: Synthesis of (*S*)-15-(aminomethyl)-8-ethyl-8-hydroxy-1,2,3,8,11,14-hexahydro-9*H*,12*H*-cyclopentadi-eno[*f*]pyrano[3',4':6,7]indolizino[1,2-*b*]quinoline -9,12-dione (Intermediate 7-9)**

**Intermediate 7-8** (50 mg) was dissolved in a mixed solution of methanol (1 mL) and *N*,*N*-dimethylformamide (1 mL), and to the mixture was added hexamethylenetetramine (483.13 mg). The reaction solution was stirred at 50°C for 4 h. After the reaction was completed, the reaction solution was cooled to room temperature. Concentrated hydrochloric acid (0.5 mL) was added. The resulting mixture was stirred for 0.5 h, and then concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.225% formic acid) and acetonitrile as the eluent; acetonitrile gradient proportion: 20%-40%, elution time: 12 min) to obtain the title compound (22.0 mg).

MS m/z (ESI): 418.2 $[M+H]^+$.

**Step 9: Synthesis of (*S*)-*N*-((8-ethyl-8-hydroxy-9,12-dioxo-2,3,8,9,12,14-hexahydro-1*H*,11*H*-cyclopentadieno[*f*] pyrano[3',4':6,7]indolizino[1,2-*b*]quinolin-15-yl)methyl)-2-hydroxyacetamide (Compound 7)**

**Intermediate 7-9** (15 mg) and hydroxyacetic acid (10.93 mg) were dissolved in anhydrous *N*,*N*-dimethylformamide (0.5 mL), and to the mixture were added HATU (27.32 mg) and diisopropylethylamine (4.64 mg). The reaction solution was stirred at 25°C for 1 h. After the reaction was completed, the reaction solution was filtered. The filtrate was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 30%-50%, elution time: 12 min) to obtain the title compound (9.0 mg).

MS m/z (ESI): 476.2$[M+H]^+$.

**$^1$H NMR (400MHz, DMSO-$d_6$)** δ = 8.28 (t, *J* = 5.1 Hz, 1H), 8.02 (d, *J* = 8.5 Hz, 1H), 7.78 (d, *J* = 8.3 Hz, 1H), 7.30 (s, 1H), 6.53 (s, 1H), 5.49-5.45 (m, 1H), 5.43 (s, 2H), 5.36 (s, 2H), 4.97 (d, *J* = 5.0 Hz, 2H), 3.88 (d, *J* = 5.5 Hz, 2H), 3.57 (t, *J* = 7.2 Hz, 2H), 3.09 (t, *J* = 7.4 Hz, 2H), 2.23-2.15 (m, 2H), 1.95-1.80 (m, 2H), 0.88 (t, *J* = 7.3 Hz, 3H).

**Example 8. (*S*)-*N*-((4-ethyl-4,9-dihydroxy-3,14-dioxo-3,4,12,14-tetrahydro-1*H*-pyrano[3',4':6,7]indolizino[1,2-*b*] quinolin-10-yl)methyl)-2-hydroxyacetamide (Compound 8)**

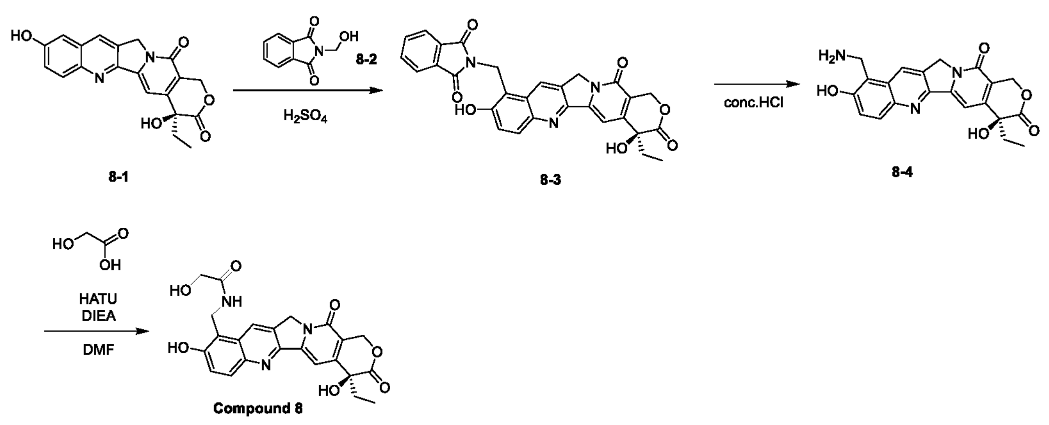


8-1

8-3

8-4

Compound 8

**Step 1: Synthesis of (*S*)-10-((1,3-dioxoisoindolin-2-yl)methyl)-4-ethyl-4,9-dihydroxy-1,12-dihydro-14*H*-pyrano[3',4':6,7]indolizino[1,2-*b*]quinoline-3,14(4*H*)-dione (Intermediate 8-3)**

**Reactant 8-1** (200 mg, 548.92 μmol) was dissolved in concentrated sulfuric acid (2 mL), and the reaction solution was cooled to 0°C. To this reaction solution was added **Reactant 8-2** (116.69 mg, 658.71 μmol) slowly, and the reaction solution was stirred at 0°C under nitrogen atmosphere for 0.5 h. After 0.5 h, under nitrogen protection, the reaction solution was warmed to 25°C and stirred for 5 h. LC-MS detection showed that the reaction was completed. Ice water (10 mL) and dichloromethane (25 mL) were added successively. The organic phase was washed with water (20 mL*2), and the washed organic phase was dried over an appropriate amount of anhydrous sodium sulfate. The organic phase was concentrated to dryness under reduced pressure, and the crude product was subjected to preparative thin layer chromatography (silica, dichloromethane : methanol = 10 : 1) to obtain the title compound (280 mg).
MS m/z (ESI): 524.3 $[M+H]^+$.

**Step 2: Synthesis of (*S*)-10-(aminomethyl)-4-ethyl-4,9-dihydroxy-1,12-dihydro-14*H*-pyrano[3',4':6,7]indolizino[1,2-*b*]quinoline-3,14(4*H*)-dione (Intermediate 8-4)**

**Intermediate 8-3** (240 mg, 458.46 μmol) was dissolved in concentrated hydrochloric acid (3 mL), and the reaction solution was stirred at 90°C for 16 h. LC-MS detection showed that the reaction was completed. Ice water (5 mL) was added after the reaction solution was cooled to room temperature, and ammonia water was added dropwise to adjust pH to 8 to 9. The reaction solution was filtered, and the cake was concentrated to dryness to obtain the crude of **Intermediate 8-4** (150 mg).
MS m/z (ESI): 394.1 $[M+H]^+$
$^1$**H** NMR (400MHz, DMSO-$d_6$) δ = 8.75 (s, 1H), 8.04 (d, *J* = 9.1 Hz, 1H), 7.51 (d, *J* = 9.1 Hz, 1H), 7.27 (s, 1H), 5.43 (s, 2H), 5.26 (s, 2H), 4.44 (s, 2H), 1.92-1.82 (m, 2H), 0.92-0.83 (m, 3H).

**Step 3: Synthesis of (*S*)-*N*-((4-ethyl-4,9-dihydroxy-3,14-dioxo-3,4,12,14-tetrahydro-1*H*-pyrano[3',4':6,7]indolizino[1,2-*b*]quinolin-10-yl)methyl)-2-hydroxyacetamide (Compound 8)**

**Intermediate 8-4** (80 mg, 203.36 μmol) and hydroxyacetic acid (30.94 mg, 406.72 μmol) were dissolved in *N*,*N*-dimethylformamide (2 mL), and to the mixture were added HATU (115.99 mg, 305.04 μmol) and *N*,*N*-diisopropylethylamine (78.85 mg, 610.08 μmol). The reaction solution was stirred at 25°C for 1.5 h. LC-MS detection showed that the reaction was completed. The reaction solution was filtered, and concentrated to dryness under reduced pressure, and the crude product was purified by preparative high performance liquid chromatography (YMC-Actus Triart C18 column, 5 μm silica, 25 mm in diameter, 100 mm in length; using a decreasingly polar mixture of water (containing 0.05% formic acid) and acetonitrile as the eluent; acetonitrile gradient proportion: 11%-41%, elution time: 12 min) to obtain the title compound (46 mg).
MS m/z (ESI): 452.1 $[M+H]^+$.
**$^1$H NMR (400MHz, DMSO-$d_6$)** δ = 10.91 (s, 1H), 8.92 (s, 1H), 8.49 (t, *J* = 5.8 Hz, 1H), 8.04 (d, *J* = 9.3 Hz, 1H), 7.51 (d, *J* = 9.1 Hz, 1H), 7.28 (s, 1H), 6.51 (s, 1H), 5.60 (t, *J* = 5.8 Hz, 1H), 5.42 (s, 2H), 5.28 (s, 2H), 4.73 (d, *J* = 5.8 Hz, 2H), 3.88 (d, *J* = 5.8 Hz, 2H), 1.95-1.81 (m, 2H), 0.89 (t, *J* = 7.3 Hz, 3H).

**Example 9. (*S*)-*N*-((4-ethyl-8-fluoro-4-hydroxy-9-methyl-3,14-dioxo-3,4,12, 14-tetrahydro-1*H*-pyrano[3',4':6,7]indolizino[1,2-*b*]quinolin-11-yl)methyl)-1-hydroxycyclopropane-1-carboxamide (Compound 9)**

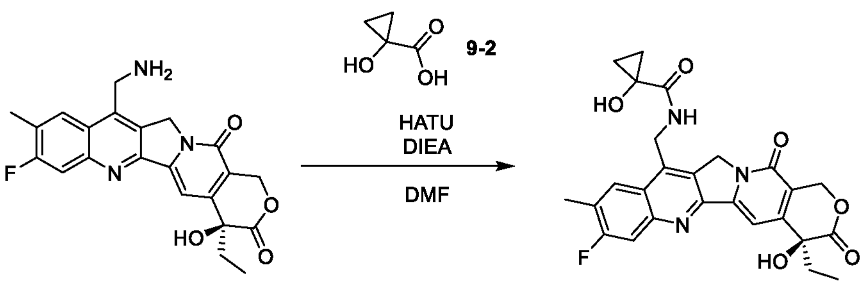


9-1

Compound 9

**Intermediate 9-1** (6.00 mg, 14.66 μmol, which could be synthesized according to the method reported in the patent document WO 2020219287) and **Intermediate 9-2** (4.49 mg, 43.97 μmol) were dissolved in anhydrous *N*,*N*-dimethylformamide (0.5 mL), and to the mixture were added HATU (8.36 mg, 21.98 μmol) and *N*,*N*-diisopropylethylamine (5.68 mg, 43.97 μmol). The reaction solution was stirred at 25°C for 1 h. LC-MS detection showed that the reaction was completed. The reaction solution was filtered, and purified by preparative high performance liquid chromatography (YMC-Actus Triart C18 column, 5 μm silica, 25 mm in diameter, 100 mm in length; using a decreasingly polar mixture of water (containing 0.05% formic acid) and acetonitrile as the eluent; acetonitrile gradient proportion: 6%-36%, elution time: 12 min) to obtain the title compound (3.00 mg).

MS m/z (ESI): 494.2 [M+H]$^+$.

**$^1$H NMR (400MHz, DMSO-$d_6$)** δ = 8.96 (t, *J* = 6.0 Hz, 1H), 8.50 (d, *J* = 8.3 Hz, 1H), 7.90 (d, *J* = 10.9 Hz, 1H), 7.32 (s, 1H), 6.53 (s, 1H), 6.30 (s, 1H), 5.52 (s, 2H), 5.44 (s, 2H), 4.85 (d, *J* = 5.9 Hz, 2H), 2.53 (s, 3H), 1.92-1.83 (m, 2H), 1.05-1.00 (m, 2H), 0.88 (t, *J* = 7.3 Hz, 3H), 0.85-0.81 (m, 2H).

**Example 10. (3)-7V-((8-chloro-4-ethyl-4-hydroxy-9-methyl-3,14-dioxo-3,4, 12,14-tetrahydro-1*H*-pyrano[3',4':6,7] indolizino[1,2-*b*]quinolin-11-yl)methyl)-1-hydroxycyclopropan-1-carboxamide (Compound 10)**

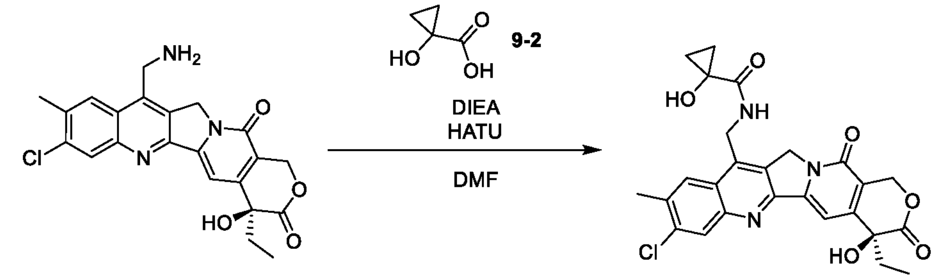


6-4

Compound 10

**Intermediate 6-4** (6.24 mg, 14.66 μmol) and **Intermediate 9-2** (4.49 mg, 43.97 μmol) were dissolved in *N*,*N*-dimethylformamide (1 mL), and to the mixture were added HATU (8.36 mg, 21.98 μmol) and DIEA (5.68 mg, 43.97 μmol). The reaction solution was stirred at 25°C for 1 h. LC-MS detection showed that the reaction was completed. The reaction solution was filtered, and concentrated to dryness under reduced pressure. The crude product was purified by preparative high performance liquid chromatography (YMC-Actus Triart C18 column, 5 μm silica, 25 mm in diameter, 100 mm in length; using a decreasingly polar mixture of water (containing 0.225% formic acid) and acetonitrile as the eluent; acetonitrile gradient proportion: 16%-46%, elution time: 12 min) to obtain the title compound (2.20 mg).

MS m/z (ESI): 510.1 [M+H]$^+$.

**1H NMR (400MHz, DMSO-$d_6$)** δ = 8.96 (t, *J* = 5.9 Hz, 1H), 8.54 (s, 1H), 8.26 (s, 1H), 7.32 (s, 1H), 6.54 (s, 1H), 6.29 (s, 1H), 5.52 (s, 2H), 5.44 (s, 2H), 4.84 (d, *J* = 6.0 Hz, 2H), 2.59 (s, 3H), 1.94-1.80 (m, 2H), 1.01 (d, *J* = 3.0 Hz, 2H), 0.88 (t, *J* = 7.3 Hz, 3H), 0.83 (d, *J* = 3.0 Hz, 2H).

**Example 11. *N*-(((*S*)-8-chloro-4-ethyl-4-hydroxy-9-methyl-3,14-dioxo-3,4, 12,14-tetrahydro-1*H*-pyrano[3',4':6,7] indolizino[1,2-*b*]quinolin-11-yl)methyl)-2-cyclopropyl-2-hydroxyacetamide (Compound 11)**

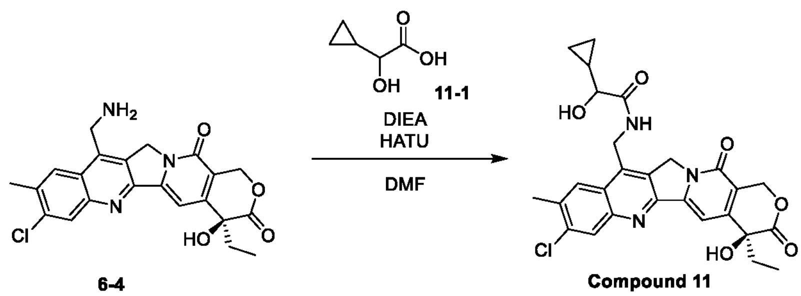


6-4

Compound 11

**Intermediate 6-4** (6.0 mg, 14.09 μmol) and **Intermediate 11-1** (8.18 mg, 70.45 μmol) were dissolved in

*N*,*N*-dimethylformamide (0.5 mL), and to the mixture were added HATU (8.04 mg, 21.13 μmol) and DIEA (5.46 mg, 42.27 μmol). The reaction solution was stirred at 25°C for 1 h. LC-MS detection showed that the reaction was completed. The reaction solution was filtered, and concentrated to dryness under reduced pressure, and the crude product was purified by preparative high performance liquid chromatography (YMC-Actus Triart C18 column 5 μm, 25 mm in diameter, 100 mm in length; using a decreasingly polar mixture of water (containing 0.225% formic acid) and acetonitrile as the eluent; acetonitrile gradient proportion: 16%-46%, elution time: 12 min) to obtain the title compound (3.0 mg).

MS m/z (ESI): 524.1 $[M+H]^+$.

**$^1$H NMR (400MHz, DMSO-$d_6$)** δ = 8.66 (t, *J* = 5.9 Hz, 1H), 8.47 (s, 1H), 8.26 (s, 1H), 7.32 (s, 1H), 6.54 (s, 1H), 5.53 (d, *J* = 5.0 Hz, 1H), 5.50 (s, 2H), 5.44 (s, 2H), 4.91-4.76 (m, 2H), 3.61-3.55 (m, 1H), 2.59 (s, 3H), 1.94-1.82 (m, 2H), 1.05-0.97 (m, 1H), 0.88 (t, *J* = 7.3 Hz, 3H), 0.34- 0.31 (m, 2H), 0.29-0.20 (m, 2H).

**Example 12. (*S*)-11-(aminomethyl)-9-chloro-4-ethyl-8,10-difluoro-4-hydroxy - 1,12-dihydro-14*H*-pyrano[3',4':6,7] indolizino[1,2-*b*]quinoline-3,14(4*H*)-dione (Compound 12)**

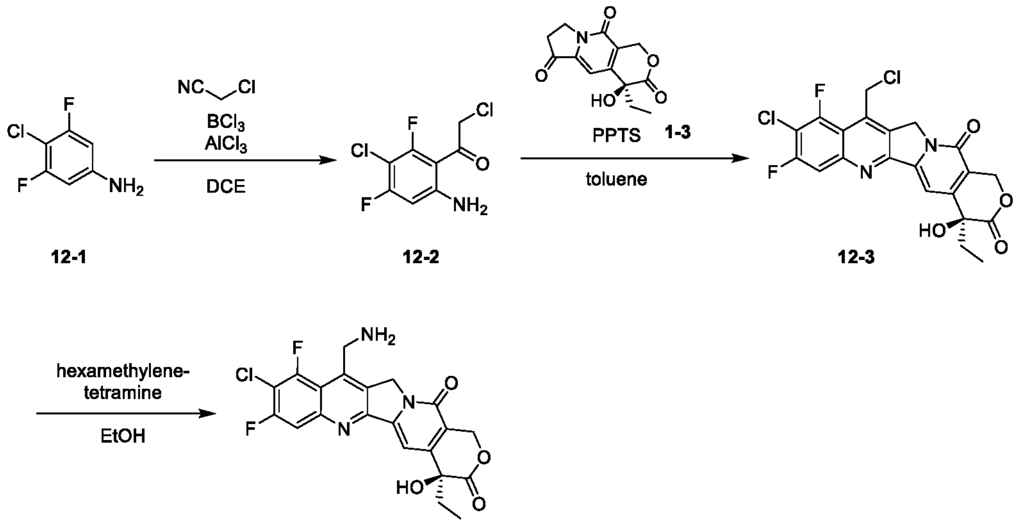


12-1 12-2 12-3

Compound 12

**Step 1: Synthesis of 1-(6-amino-3-chloro-2,4-difluorophenyl)-2-chloroethan-1-one (Intermediate 12-2)**

Boron trichloride (1 M, 6.11 mL) was dissolved in 1,2-dichloroethane (12 mL). The reaction solution was cooled to 0°C, and to which were added **Reactant 12-1** (1 g, 6.11 mmol) and chloroacetonitrile (784.73 mg, 10.39 mmol). The reaction solution was stirred at 0°C for 10 min, and to which was added aluminum trichloride (1.06 mg, 7.95 mmol). The reaction solution was then warmed to 25°C under nitrogen protection and stirred for 10 min. The reaction solution was stirred at 90°C under nitrogen protection for 18 h. LC-MS detection showed that the reaction was completed. Ice water (25 mL) and 5% hydrochloric acid (5 mL) were added successively and slowly, and stirred at 25°C for 30 min after the reaction solution was cooled to room temperature. Then dichloromethane (50 mL) was added. The organic phase was washed with water (2 mL*2), and the washed organic phase was dried over an appropriate amount of anhydrous sodium sulfate. The organic phase was concentrated to dryness under reduced pressure, and the crude product was subjected to preparative thin layer chromatography (silica, petroleum ether:ethyl acetate = 9 : 1) to obtain the title compound (340 mg).

MS m/z (ESI): 240.0 $[M+H]^+$.

**Step 2: Synthesis of (*S*)-9-chloro-11-(chloromethyl)-4-ethyl-8,10-difluoro-4-hydroxy-1,12-dihydro-14*H*-pyrano[3',4':6,7]indolizino[1,2-*b*]quinoline-3,14(4*H*)-dione (Intermediate 12-3)**

**Intermediate 12-2** (0.2 g, 833.22 μmol) and **Intermediate 1-3** (219.34 mg, 833.22 mmol) were dissolved in toluene (4 mL), and to the mixture was added pyridinium p-toluenesulfonate (20.94 mg, 83.32 μmol). The reaction solution was stirred at 100°C for 18 h. LC-MS detection showed that the reaction was completed. Ethanol (1 mL) was added after the reaction solution was cooled to room temperature. The reaction solution was stirred at 25°C for 0.5 h.

The reaction solution was filtered. The filter cake was washed with ethanol (2 mL*2) to obtain the crude of the title compound (190 mg).

MS m/z (ESI): 467.1 $[M+H]^+$.

**Step 3: Synthesis of (*S*)-11-(aminomethyl)-9-chloro-4-ethyl-8,10-difluoro-4-hydroxy-1,12-dihydro-14*H*-pyrano[3',4':6,7]indolizino[1,2-*b*]quinoline-3,14(4*H*)-dione (Compound 12)**

**Intermediate 12-3** (50 mg, 107.01 μmol) was dissolved in ethanol (1 mL), and to the mixture was added hexamethylenetetramine (45.00 mg, 321.03 μmol). The reaction solution was stirred at 80°C for 1.5 h. LC-MS detection showed that the reaction was completed. The reaction solution was concentrated to dryness under reduced pressure after the reaction solution was cooled to room temperature, and purified by preparative high performance liquid chromatography (YMC-Actus Triart C18 column, 5 μm silica, 25 mm in diameter, 100 mm in length; using a decreasingly polar mixture of water (containing 0.225% formic acid) and acetonitrile as the eluent; acetonitrile gradient proportion: 2%-32%, elution time: 12 min) to obtain the title compound (1.22 mg).

MS m/z (ESI): 448.0 $[M+H]^+$.

**$^1$H NMR (400MHz, DMSO-$d_6$)** δ = 8.14 (d, *J* = 9.8 Hz, 1H), 7.36 (s, 1H), 6.57 (s, 1H), 5.55 (s, 2H), 5.46 (s, 2H), 4.35 (d, *J* = 3.0 Hz, 2H), 1.94-1.83 (m, 2H), 0.88 (t, *J* = 7.3 Hz, 3H).

**Example 13. 2-cyclopropyl-*N*-(((*S*)-8-ethyl-8-hydroxy-9,12-dioxo-2,3,8,9, 12,14-hexahydro-1*H*,1*H*-cyclopentadieno[*f*]pyrano[3',4':6,7]indolizino [1,2-*b*]quinolin-15-yl)methyl)-2-hydroxyacetamide (Compound 13)**

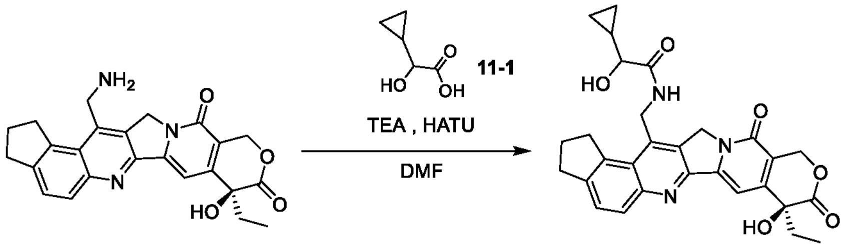


7-9

Compound 13

**Step 1: Synthesis of 2-cyclopropyl-*N*-(((*S*)-8-ethyl-8-hydroxy-9,12-dioxo-2,3,8,9,12,14-hexahydro-1*H*,11*H*-cyclopentadieno[*f*]pyrano[3',4':6,7]indolizino [1,2-*b*]quinolin-15-yl)methyl)-2-hydroxyacetamide (Compound 13)**

**Intermediate 7-9** (10 mg) **and Intermediate 11-1** (8.34 mg) were dissolved in anhydrous *N*,*N*-dimethylformamide (0.5 mL), and to the mixture were added HATU (13.66 mg) and diisopropylethylamine (3.10 mg). The reaction solution was stirred at 25°C for 1 h. After the reaction was completed, the reaction solution was filtered. The filtrate was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 34%-54%, elution time: 12 min) to obtain the title compound (0.8 mg).

MS m/z (ESI): 516.2 $[M+H]^+$.

**$^1$H NMR (400MHz, DMSO-$d_6$)** δ = 8.25 (t, *J* = 5.0 Hz, 1H), 8.01 (d, *J* =8.5 Hz, 1H), 7.78 (d, *J* = 8.5 Hz, 1H), 7.30 (s, 1H), 6.53 (s, 1H), 5.43 (s, 2H), 5.36 (s, 2H), 4.99-4.85 (m, 2H), 3.59 (s, 1H), 3.56 (d, *J* = 6.3 Hz, 2H), 3.08 (t, *J* = 7.7 Hz, 2H), 2.23-2.16 (m, 2H), 1.92-1.72 (m, 2H), 1.15-1.01 (m, 1H), 0.88 (t, *J* = 7.4 Hz, 3H), 0.46-0.19 (m, 4H).

**Example 14. 2-cyclopropyl-*N*-(((*S*)-7-ethyl-7-hydroxy-8,11-dioxo-7,8,11,13 - tetrahydro-10*H*-[1,3]dioxolo[4,5-*g*] pyrano[3',4':6,7]indolizino[1,2-6] quinolin-14-yl)methyl)-2-hydroxyacetamide (Compound 14)**

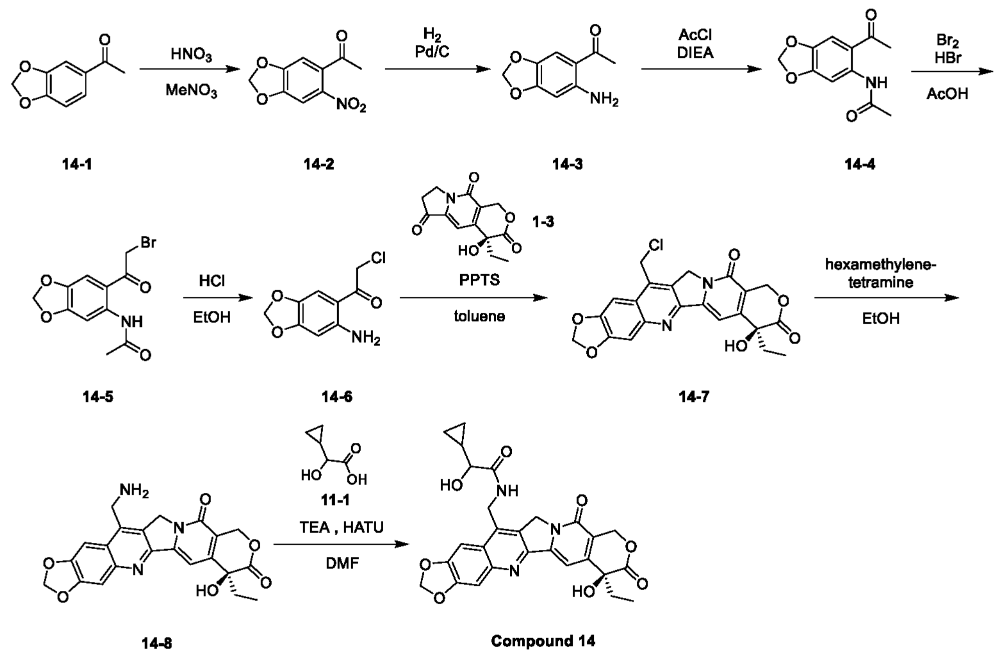


**Step 1: Synthesis of 1-(6-nitrobenzo[*d*][1,3]dioxol-5-yl)ethanone (Intermediate 14-2)**

**Intermediate 14-1** (10.0 g, 60.92 mmol) was dissolved in nitromethane (100 mL), and to the mixture was slowly added nitric acid (35.43 g, 365.50 mmol, 65% purity). The reaction solution was stirred at 25°C for 2.5 h. After the reaction was completed, a saturated sodium bicarbonate solution was slowly added to reaction solution to adjust pH to 7-8, and then dichloromethane (100 mL) was added. The organic phase was washed with water (50 mL*2), and the washed organic phase was dried over an appropriate amount of anhydrous sodium sulfate. The organic phase was purified by preparative thin layer chromatography (petroleum ether:ethyl acetate = 1:2) to obtain the title compound (5 g).

MS m/z (ESI): 210.0 $[M+H]^+$.

**Step 2: Synthesis of 1-(6-aminobenzo[*d*][1,3]dioxol-5-yl)ethanone (Intermediate 14-3)**

**Intermediate 14-2** (2.37 g, 11.33 mmol) was dissolved in absolute alcohol (25 mL), and to the mixture was added palladium on carbon (0.2 g, 10% purity). The reaction solution was stirred at 25°C under hydrogen protection for 16 h. After the reaction was completed, the reaction solution was filtered. The filter cake was washed 2 times with ethyl acetate. The filtrate was concentrated to dryness under reduced pressure to obtain the title compound (1.6 g).

MS m/z (ESI): 180.1 $[M+H]^+$.

**Step 3: Synthesis of *N*-(6-acetylbenzo[*d*][1,3]dioxol-5-yl)acetamide (Intermediate 14-4)**

**Intermediate 14-3** (1.0 g, 5.58 mmol) was dissolved in dichloromethane (10 mL). The reaction solution was cooled to 0°C, and to which were added *N,N*-diisopropylethylamine (DIEA) (1.08 g, 8.37 mmol) and acetyl chloride (569.55 mg, 7.26 mmol). The reaction solution was stirred at 25°C for 1.5 h. After the reaction was completed, the reaction solution was concentrated to dryness under reduced pressure to obtain the title compound (1.23 g).

MS m/z (ESI):222.1 $[M+H]^+$.

**Step 4: Synthesis of *N*-(6-(2-bromoacetyl)benzo[*d*][1,3]dioxol-5-yl)acetamide (Intermediate 14-5)**

**Intermediate 14-4** (1.23 g, 5.00 mmol) was dissolved in acetic acid (12 mL), to the mixture was added a solution of hydrogen bromide in acetic acid (1.84 g, 7.51 mmol, 33% purity), and then to which was slowly added liquid bromine (959.69 mg, 6.01 mmol). The reaction solution was stirred at 25°C for 1 h. After the reaction was completed, the reaction solution was poured into ice water and stirred for 10 min. The reaction solution was filtered. The filter cake was washed 2 times with water. The filtrate was concentrated to dryness under reduced pressure. Ethyl acetate (2 mL) and petroleum ether (10 mL) were added to the residue. The reaction solution was stirred at 25°C for 0.5 h. The reaction solution was filtered. The filter cake was dried to obtain the title compound (500 mg).

MS m/z (ESI): 300.0 $[M+H]^+$.

**Step 5: Synthesis of 1-(6-aminobenzo[*d*][1,3]dioxol-5-yl)-2-chloroethanone (Intermediate 14-6)**

**Intermediate 14-5** (0.2 g, 666.43 μmol) was dissolved in absolute alcohol (1 mL) and concentrated hydrochloric acid (1 mL), and the reaction solution was stirred at 60°C for 16 h. After the reaction was completed, ice water (10 mL) and saturated sodium bicarbonate (10 mL) were added successively and slowly after the reaction solution was cooled to room temperature. Then dichloromethane (50 mL) was added. The organic phase was washed with water (20 mL*2), and the washed organic phase was dried over an appropriate amount of anhydrous sodium sulfate. Preparative thin layer chromatography (petroleum ether:ethyl acetate = 6:1) was performed to obtain the title compound (160 mg).
MS m/z (ESI): 214.0 $[M+H]^+$.

**Step 6: Synthesis of (*S*)-14-(bromomethyl)-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione (Intermediate 14-7)**

**Intermediate 14-6** (50 mg, 234.06 μmol) and **Intermediate 1-3** (61.62 mg, 234.06 μmol) were dissolved in toluene (1 mL), and to the mixture was added pyridinium p-toluenesulfonate (5.88 mg, 23.41 μmol). The reaction solution was stirred at 90°C for 16 h. After the reaction was completed, ethanol (1 mL) was added after the reaction solution was cooled to room temperature. The reaction solution was stirred at 25°C for 0.5 h. The reaction solution was filtered. The filter cake was washed with ethanol (2 mL*2), and then dried to obtain the title compound (60 mg).
MS m/z (ESI): 441.1 $[M+H]^+$.

**Step 7: Synthesis of (*S*)-14-(aminomethyl)-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione (Intermediate 14-8)**

**Intermediate 14-7** (55.00 mg, 124.76 μmol) was dissolved in ethanol (1 mL), and to the mixture was added hexamethylenetetramine (52.47 mg, 374.29 μmol). The reaction solution was stirred at 80°C for 1.5 h. After the reaction was completed, the reaction solution was concentrated to dryness under reduced pressure after the reaction solution was cooled to room temperature, and purified by preparative high performance liquid chromatography (YMC-Actus Triart C18 column 5 μm, 25 mm in diameter, 100 mm in length; using a decreasingly polar mixture of water (containing 0.225% formic acid) and methanol as the eluent; methanol gradient proportion: 0%-27%, elution time: 12 min) to obtain the title compound (10 mg).
MS m/z (ESI): 422.1 $[M+H]^+$.

**Step 8: Synthesis of 2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 14)**

**Intermediate 14-8** (10.00 mg, 20.17 μmol) and **Intermediate 11-1** (23.42 mg, 201.71 μmol) were dissolved in anhydrous *N*,*N*-dimethylformamide (0.5 mL), and to the mixture were added 2-(7-azabenzotriazol-1-yl)-*N*,*N*,*N*',*N*'-tetramethyluronium hexafluorophosphate (HATU) (11.50 mg, 30.26 μmol) and *N*,*N*-diisopropylethylamine (7.82 mg, 60.51 μmol). The reaction solution was stirred at 25°C for 1.5 h. After the reaction was completed, the reaction solution was filtered, and purified by preparative high performance liquid chromatography (YMC-Actus Triart C18 column 5 μm, 30 mm in diameter, 150 mm in length; using a decreasingly polar mixture of water (containing 0.225% formic acid) and acetonitrile as the eluent; acetonitrile gradient proportion: 4%-44%, elution time: 9 min) to obtain the title compound (3 mg).
MS m/z (ESI): 520.1 $[M+H]^+$.
**$^1$H NMR (400MHz, DMSO-*d*$_6$)** δ = 8.61 (t, *J* = 5.8 Hz, 1H), 7.86 (s, 1H), 7.52 (s, 1H), 7.24 (s, 1H), 6.49 (s, 1H), 6.29 (s, 2H), 5.51 (d, *J* = 5.0 Hz, 1H), 5.47 (s, 2H), 5.42 (s, 2H), 4.72 (d, *J* = 5.5 Hz, 2H), 1.92-1.78 (m, 2H), 0.99 (d, *J* = 6.3 Hz, 1H), 0.87 (t, *J* = 7.3 Hz, 3H), 0.40-0.29 (m, 2H), 0.29-0.19 (m, 2H).

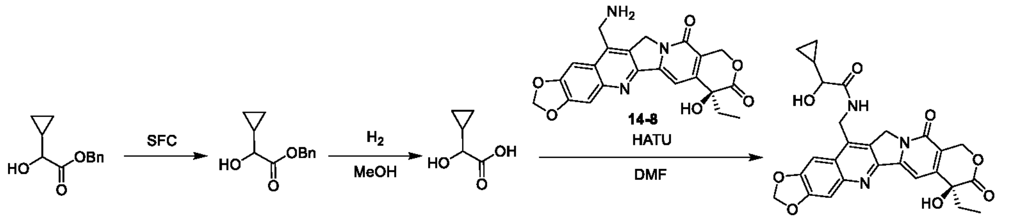


14-9 14-9-P1/P2 14-10-P1/P2 Compound 14-P1/P2

(

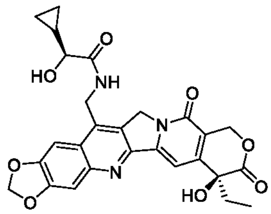

,

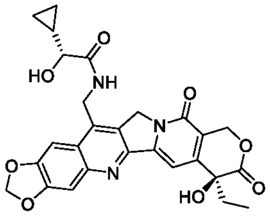

)

**Step 9: Preparation of benzyl 2-cyclopropyl-2-hydroxyacetate (Intermediate 14-9-P1/P2)**

Intermediate 14-9 was resolved to prepare Isomers 14-9-P1 and 14-9-P2. **Intermediate 14-9** (1.3 g) was taken and resolved by preparative supercritical fluid chromatography (DAICEL CHIRALPAK AD column, 10 μm silica, 30 mm in diameter, 250 mm in length; using ethanol (containing 0.1% ammonia water) as the eluent) to obtain **Intermediate 14-9-P1** (600 mg) and **Intermediate 14-9-P2** (600 mg).

The above-mentioned two isomers were further analyzed under the following chiral supercritical fluid chromatography conditions.

| | | |
|---|---|---|
| Column | Chiralpak AD-3 150*4.6 mm I.D., 3 μm | |
| Mobile phase | A: Carbon dioxide | |
| | B: Methanol (0.05% diethylamine) | |
| | Time (min) | % Phase B |
| | 0 | 5 |
| | 4 | 40 |
| | 0.2 | 5 |
| | 1.8 | 5 |
| Flow rate | 2.5 mL/min | |
| Wavelength | PDA 220 nm | |
| Column temperature | 35°C | |
| Column pressure | 1500 Psi | |
| Model of instrument | Waters UPCC with PDA Detector | |

**Intermediate 14-9-PI:**

Under the above chiral supercritical fluid chromatography conditions, the retention time was 2.990 min; **$^1$H NMR (400MHz, METHANOL-*d*$_4$)** δ 7.43-7.29 (m, 5H), 5.29-5.16 (m, 2H), 3.67 (d, *J* = 7.6 Hz, 1H), 1.19-1.07 (m, 1H), 0.58-0.38 (m, 4H).

**Intermediate 14-9-P2:**

Under the above chiral supercritical fluid chromatography conditions, the retention time was 2.661 min;

**$^1$H NMR (400MHz, METHANOL-$d_4$)** δ 7.46-7.28 (m, 5H), 5.30-5.16 (m, 2H), 3.67 (d, *J*= 7.6 Hz, 1H), 1.21-1.03 (m, 1H), 0.60-0.36 (m, 4H).

**Step 10: Synthesis of 2-cyclopropyl-2-hydroxyacetic acid (Intermediate 14-10-P1/P2)**

Under a hydrogen atmosphere, **Intermediate 14-9-P1** (500 mg) was added to methanol (15 mL), and to the reaction solution was added wet palladium on carbon (10 mg, 10%). The reaction solution was stirred at 25°C under hydrogen atmosphere for 16 h. After the reaction was completed, the reactant was filtered. The filtrate was concentrated under reduced pressure to obtain **Intermediate 14-10-P1** (273 mg).
**$^1$H NMR (400MHz, METHANOL-$d_4$)** δ 3.63 (d, *J* = 7.2 Hz, 1H), 1.21-1.09 (m, 1H), 0.61-0.40 (m, 4H).
Under a hydrogen atmosphere, **Intermediate 14-9-P2** (500 mg) was added to methanol (15 mL), and to the reaction solution was added wet palladium on carbon (10 mg, 10%). The reaction solution was stirred at 25°C under hydrogen atmosphere for 16 h. After the reaction was completed, the reactant was filtered. The filtrate was concentrated under reduced pressure to obtain **Intermediate 14-10-P2** (279 mg).
**$^1$H NMR (400MHz, METHANOL-$d_4$)** δ 3.63 (d, *J* = 7.2 Hz, 1H), 1.19-1.08 (m, 1H), 0.60-0.39 (m, 4H).

**Step 11: Synthesis of 2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 14-P1/P2)**

**Intermediate 14-8** (40.00 mg) and **Intermediate 14-10-P1** (28.11 mg) were dissolved in anhydrous *N,N*-dimethylformamide (1 mL), and to the mixture were added 2-(7-azabenzotriazol-1-yl)-*N,N,N',N'*-tetramethyluronium hexafluorophosphate (HATU) (46.02 mg) and *N,N*-diisopropylethylamine (31.28 mg). The reaction solution was stirred at 25°C for 1.5 h. After the reaction was completed, the reaction solution was purified by preparative high performance liquid chromatography (Boston Green ODS C18 column, 5 μm silica, 30 mm in diameter, 150 mm in length; using a decreasingly polar mixture of water (containing 0.225% formic acid) and acetonitrile as the eluent; acetonitrile gradient proportion: 16%-46%, elution time: 12 min) to obtain **Compound 14-P1** (22.00 mg).
MS m/z (ESI): 520.1 $[M+H]^+$.
**$^1$H NMR (400MHz, DMSO-$d_6$)** δ = 8.62 (t, *J* = 5.7 Hz, 1H), 7.86 (s, 1H), 7.52 (s, 1H), 7.25 (s, 1H), 6.51 (s, 1H), 6.29 (s, 2H), 5.47 (s, 2H), 5.43 (s, 2H), 4.73 (d, *J* = 5.9 Hz, 2H), 3.54 (d, *J* = 5.9 Hz, 1H), 1.93-1.78 (m, 2H), 1.06-0.96 (m, 1H), 0.87 (t, *J* = 7.3 Hz, 3H), 0.39-0.30 (m, 2H), 0.29-0.21 (m, 2H).
**Intermediate 14-8** (10.00 mg) and **Intermediate 14-10-P2** (8.27 mg) were dissolved in anhydrous *N,N*-dimethylformamide (0.5 mL), and to the mixture were added 2-(7-azabenzotriazol-1-yl)-*N,N,N',N'*-tetramethyluronium hexafluorophosphate (18.05 mg) and *N,N*-diisopropylethylamine (6.13 mg). The reaction solution was stirred at 25°C for 1.5 h. After the reaction was completed, the reaction solution was directly purified by preparative high performance liquid chromatography (Boston Green ODS C18 column, 5 μm silica, 30 mm in diameter, 150 mm in length; using a decreasingly polar mixture of water (containing 0.225% formic acid) and acetonitrile as the eluent; acetonitrile gradient proportion: 16%-46%, elution time: 12 min) to obtain **Compound 14-P2** (8.00 mg).
MS m/z (ESI): 520.1 $[M+H]^+$.
**$^1$H NMR (400MHz, DMSO-$d_6$)** δ = 8.63 (t, *J* = 5.9 Hz, 1H), 7.86 (s, 1H), 7.52 (s, 1H), 7.24 (s, 1H), 6.30 (s, 2H), 5.46 (s, 2H), 5.43 (s, 2H), 4.72 (d, *J* = 6.0 Hz, 2H), 3.55 (d, *J* = 6.0 Hz, 1H), 1.92-1.81 (m, 2H), 1.03-0.97 (m, 1H), 0.88 (t, *J* = 7.3 Hz, 3H), 0.38-0.30 (m, 2H), 0.28-0.22 (m, 2H).
The two isomers were further analyzed separately by the following chiral supercritical fluid chromatography.

| | |
|---|---|
| Column | Chiralcel OJ-3 100A 4.6 mm I.D., 3 μm |
| Mobile phase | A: Carbon dioxide |
| | B: Ethanol (0.05% diethylamine) |
| Flow rate | 2.8 mL/min |
| Wavelength | PDA 254 nm |
| Column temperature | 35°C |
| Column pressure | 1500 Psi |
| Model of instrument | Waters UPCC with PDA Detector and QDa Detector |

**Compound 14-P1:**

Under the above chiral supercritical fluid chromatography conditions, the retention time was 3.673 min;

**Compound 14-P2:**

Under the above chiral supercritical fluid chromatography conditions, the retention time was 3.735 min.

**Example 15. (*S*)-*N*-((9-bromo-4-ethyl-4-hydroxy-3,14-dioxo-3,4,12,14-tetrahydro-1*H*-pyrano[3',4':6,7]indolizino[1,2-*b*][1,7]naphthyridin-11-yl)methyl)-2-hydroxyacetamide (Compound 15)**

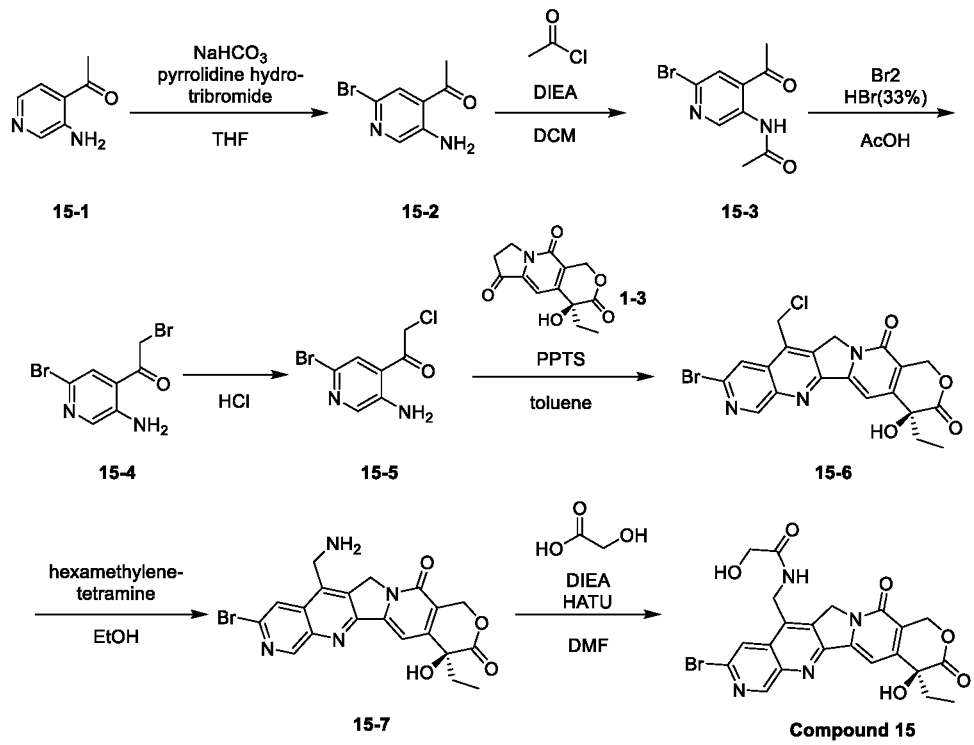


**Step 1: Synthesis of 1-(5-amino-2-bromopyridin-4-yl)ethanone (Intermediate 15-2)**

**Intermediate 15-1** (500 mg, 3.67 mmol) was dissolved in anhydrous tetrahydrofuran (90 mL), and to the mixture were added sodium bicarbonate (617.04 mg, 7.34 mmol) and 2-pyrrolidone hydrotribromide (1.64 g, 5.03 mmol). The reaction solution was stirred at 25°C for 8 h. After the reaction was completed, the reaction solution was filtered, and subjected to preparative thin layer chromatography (silica, petroleum ether:ethyl acetate = 20:1) to obtain the title compound (300 mg).

MS m/z (ESI): 214.9 $[M+H]^+$.

**Step 2: Synthesis of *N*-(4-acetyl-6-bromopyridin-3-yl)acetamide (Intermediate 15-3)**

**Intermediate 15-2** (150 g, 697.52 mmol) was dissolved in dichloromethane (2 mL). The reaction solution was cooled to 0°C, and to which were added *N,N*-diisopropylethylamine (180.30 mg, 1.40 mmol) and acetyl chloride (109.51 mg, 1.40 mmol). The reaction solution was stirred at 25°C for 3 h. After the reaction was completed, the reaction solution was concentrated to dryness under reduced pressure, and preparative thin layer chromatography (silica, petroleum ether : ethyl acetate = 3:1) was performed to obtain the title compound (100 mg).

MS m/z (ESI):257.0 $[M+H]^+$.

**Step 3: Synthesis of 1-(5-amino-2-bromopyridin-4-yl)-2-bromoethanone (Intermediate 15-4)**

**Intermediate 15-3** (95.00 mg, 273.45 μmol) was dissolved in acetic acid (2 mL), to the mixture was added a solution of hydrogen bromide in acetic acid (100.57 g, 410.18 μmol, 33% purity), and then to which was slowly added liquid bromine (48.07 mg, 300.80 μmol). The reaction solution was stirred at 25°C for 1 h. After the reaction was completed, the reaction solution was concentrated to dryness under reduced pressure, and preparative thin layer chromatography (silica, petroleum ether:ethyl acetate = 3:1) was performed to obtain the title compound (45 mg).

MS m/z (ESI): 292.9 $[M+H]^+$.

**Step 4: Synthesis of 1-(5-amino-2-bromopyridin-4-yl)-2-chloroethanone (Intermediate 15-5)**

**Intermediate 15-4** (50.00 mg, 170.10 μmol) was dissolved in concentrated hydrochloric acid (1 mL), and the reaction solution was stirred at 60°C for 16 h. After the reaction was completed, ice water (10 mL) and saturated sodium bicarbonate (10 mL) were added successively and slowly after the reaction solution was cooled to room temperature. Then dichloromethane (30 mL) was added. The organic phase was washed with water (20 mL*2), and the washed organic phase was dried over an appropriate amount of anhydrous sodium sulfate. The organic phase was concentrated to dryness under reduced pressure to obtain the title compound (25 mg).

MS m/z (ESI): 248.9 $[M+H]^+$.

**Step 5: Synthesis of (*S*)-9-bromo-11-(chloromethyl)-4-ethyl-4-hydroxy-1,12-dihydro-14*H*-pyrano[3',4':6,7]indolizino[1,2-*b*][1,7]naphthyridine-3,14(4*H*)-dione (Intermediate 15-6)**

**Intermediate 15-5** (25 mg, 100.20 μmol) and **Intermediate 1-3** (26.38 mg, 100.20 μmol) were dissolved in toluene (0.5 mL), and to the mixture was added pyridinium p-toluenesulfonate (2.52 mg, 10.02 μmol). The reaction solution was stirred at 90°C for 16 h. After the reaction was completed, ethanol (1 mL) was added after the reaction solution was cooled to room temperature. The reaction solution was stirred at 25°C for 0.5 h. The reaction solution was filtered. The filter cake was washed with ethanol (2 mL*2) to obtain the title compound (30 mg).

MS m/z (ESI): 475.9 $[M+H]^+$.

**Step 6: Synthesis of (*S*)-11-(aminomethyl)-9-bromo-4-ethyl-4-hydroxy-1,12-dihydro-14*H*-pyrano[3',4':6,7]indolizino[1,2-*b*][1,7]naphthyridine-3,14(4*H*)-dione (Intermediate 15-7)**

**Intermediate 15-6** (30 mg, 62.93 μmol) was dissolved in ethanol (1 mL), and to the mixture was added hexamethylenetetramine (17.64 mg, 125.86 μmol). The reaction solution was stirred at 80°C for 2 h. After the reaction was completed, the reaction solution was concentrated to dryness under reduced pressure after the reaction solution was cooled to room temperature, and purified by preparative high performance liquid chromatography (YMC-Actus Triart C18 column 5 μm, 25 mm in diameter, 100 mm in length; using a decreasingly polar mixture of water (containing 0.225% formic acid) and methanol as the eluent; methanol gradient proportion: 0%-25%, elution time: 12 min) to obtain the title compound (4 mg).

MS m/z (ESI): 457.0 $[M+H]^+$.

**Step 7: Synthesis of (*S*)-N-((9-bromo-4-ethyl-4-hydroxy-3,14-dioxo-3,4,12,14-tetrahydro-1*H*-pyrano[3',4':6,7]indolizino[1,2-*b*][1,7]naphthyridin-11-yl)methyl)-2-hydroxyacetamide (Compound 15)**

**Intermediate 15-7** (4 mg, 8.75 μmol) and hydroxyacetic acid (3.33 mg, 43.74 μmol) were dissolved in anhydrous *N,N*-dimethylformamide (0.5 mL), and to the mixture were added HATU (4.99 mg, 13.12 μmol) and *N,N*-diisopropylethylamine (3.39 mg, 26.24 μmol). The reaction solution was stirred at 25°C for 2 h. After the reaction was completed, the reaction solution was filtered, and purified by preparative high performance liquid chromatography (YMC-Actus Triart C18 column 5 μm, 25 mm in diameter, 100 mm in length; using a decreasingly polar mixture of water (containing 0.05% formic acid) and acetonitrile as the eluent; acetonitrile gradient proportion: 8%-28%, elution time: 12 min) to obtain the title compound (1.00 mg).

MS m/z (ESI): 515.0 $[M+H]^+$.

**$^1$H NMR (400MHz, DMSO-*d*$_6$)** δ = 9.38 (s, 1H), 8.88 (t, *J* = 6.2 Hz, 1H), 8.76 (s, 1H), 7.39 (s, 1H), 6.57 (s, 1H), 5.64-5.61 (m, 1H), 5.60 (s, 2H), 5.45 (s, 2H), 4.80 (d, *J* = 6.0 Hz, 2H), 3.83 (d, *J* = 5.7 Hz, 2H), 1.92-1.80 (m, 2H), 0.88 (t, *J* = 7.3 Hz, 3H).

**Example 16. (*S*)-N-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-1-hydroxycyclopropane-1-carboxamide (Compound 16)**

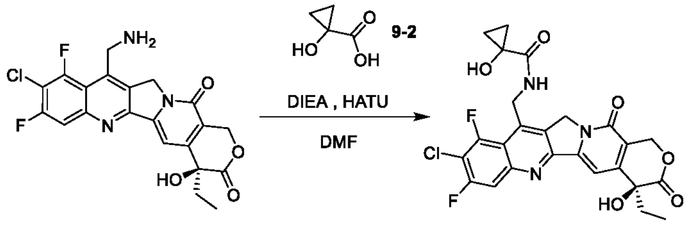


**Compound 12** **Compound 16**

**Step 1: Synthesis of (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-1-hydroxycyclopropane-1-carboxamide (Compound 16)**

**Compound 12** (5.75 mg, 12.84 μmol) and **Intermediate 9-2** (3.93 mg, 38.52 μmol) were dissolved in *N,N*-dimethylformamide (0.5 mL), and to the mixture were added HATU (7.32 mg, 19.26 μmol) and diisopropylethylamine (4.98 mg, 38.52 μmol). The reaction solution was stirred at 30°C for 1 h. After the reaction was completed, the reaction solution was filtered, and purified by preparative high performance liquid chromatography (YMC-Actus Triart C18 column 5 μm, 25 mm in diameter, 100 mm in length; using a decreasingly polar mixture of water (containing 0.225% formic acid) and acetonitrile as the eluent; acetonitrile gradient proportion: 14%-34%, elution time: 12 min) to obtain the title compound (3 mg).

MS m/z (ESI): 532.1 $[M+H]^+$.

**$^1$H NMR (400MHz, DMSO-$d_6$)** δ = 8.46 (t, *J* = 5.9 Hz, 1H), 8.16 (d, *J* = 9.8 Hz, 1H), 7.36 (s, 1H), 6.56 (s, 1H), 6.33 (s, 1H), 5.53 (s, 2H), 5.45 (s, 2H), 4.93 (d, *J* = 3.6 Hz, 2H), 1.92-1.82 (m, 2H), 1.04-0.99 (m, 2H), 0.90-0.87 (m, 2H), 0.87-0.82 (m, 3H).

**Example 17. (*S*)-*N*-((8-chloro-4-ethyl-4-hydroxy-9-methyl-3,14-dioxo-3,4, 12,14-tetrahydro-1*H*-pyrano[3',4':6,7] indolizino[1,2-*b*]quinolin-11-yl)methyl)-2-hydroxy-2-methylpropanamide (Compound 17)**

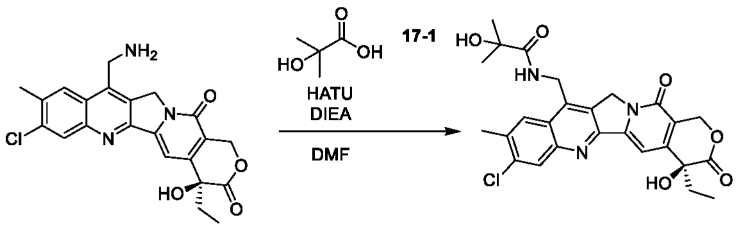


**6-4** **Compound 17**

**Step 1: Synthesis of (*S*)-*N*-((8-chloro-4-ethyl-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxy-2-methylpropanamide (Compound 17)**

**Intermediate 6-4** (5 mg, 11.74 μmol) and **Intermediate 17-1** (2.44 mg, 23.48 μmol) were dissolved in anhydrous *N,N*-dimethylformamide (0.5 mL), and to the mixture were added HATU (8.95 mg, 23.48 μmol) and *N,N*-diisopropylethylamine (1.19 mg, 11.74 μmol). The reaction solution was stirred at 25°C for 1 h. After the reaction was completed, the reaction solution was filtered, and purified by preparative high performance liquid chromatography (Waters Xbridge C18 column 5 μm silica, 25 mm in diameter, 100 mm in length; using a decreasingly polar mixture of water (containing 0.05% formic acid) and acetonitrile as the eluent; acetonitrile gradient proportion: 22%-42%, elution time: 12 min) to obtain the title compound (1 mg).

MS m/z (ESI): 512.1 $[M+H]^+$.

**$^1$H NMR (400MHz, Methanol-*d*$_4$)** $\delta$ = 8.32 (s, 1H), 8.22 (s, 1H), 7.67 (s, 1H), 5.62 (d, *J* = 16.4 Hz, 1H), 5.52 (s, 2H), 5.42 (d, *J* = 16.4 Hz, 1H), 5.01 (s, 2H), 2.65 (s, 3H), 2.05-1.94 (m, 2H), 1.38 (s, 6H), 1.03 (t, *J* = 7.5 Hz, 3H).

**Example 18. *N*-(((*S*)-8-chloro-4-ethyl-4-hydroxy-9-methyl-3,14-dioxo-3,4, 12,14-tetrahydro-1*H*-pyrano[3',4':6,7] indolizino[1,2-*b*]quinolin-11-yl)methyl)-2-hydroxy-3-methylbutanamide (Compound 18)**

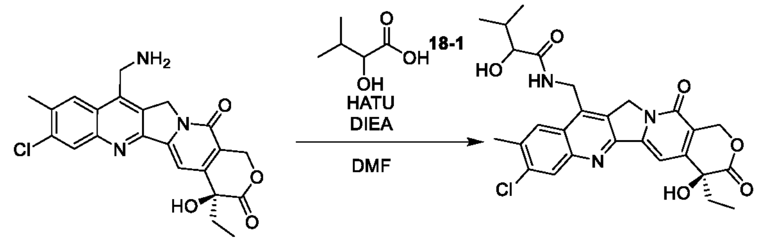


6-4

Compound 18

**Step 1: Synthesis of *N*-(((*S*)-8-chloro-4-ethyl-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxy-3-methylbutanamide (Compound 18)**

**Intermediate 6-4** (5 mg, 11.74 $\mu$mol) and **Intermediate 18-1** (2.77 mg, 23.48 $\mu$mol) were dissolved in anhydrous *N,N*-dimethylformamide (0.5 mL), and to the mixture were added HATU (8.95 mg, 23.48 $\mu$mol) and *N,N*-diisopropylethylamine (1.19 mg, 11.74 $\mu$mol). The reaction solution was stirred at 25°C for 1 h. After the reaction was completed, the reaction solution was filtered, and the filtrate was purified by preparative high performance liquid chromatography (Waters Xbridge C18 column 5 $\mu$m, 25 mm in diameter, 100 mm in length; using a decreasingly polar mixture of water (containing 0.05% formic acid) and acetonitrile as the eluent; acetonitrile gradient proportion: 25%-45%, elution time: 12 min) to obtain the title compound (1.20 mg).

MS m/z (ESI): 526.1 $[M+H]^+$.

**$^1$H NMR (400MHz, Methanol-*d*$_4$)** $\delta$ = 8.30 (s, 1H), 8.13 (s, 1H), 7.61 (s, 1H), 5.60 (d, *J* = 16.3 Hz, 1H), 5.56-5.45 (m, 2H), 5.42-5.37 (m, 1H), 5.00-4.90 (m, 2H), 3.91 (d, *J* = 3.3 Hz, 1H), 3.13 (d, *J* = 6.5 Hz, 1H), 2.62 (s, 3H), 2.01-1.94 (m, 2H), 1.05-1.00 (m, 6H), 0.77-0.70 (m, 3H).

**Example 19. (*S*)-4-ethyl-8-fluoro-4-hydroxy-11-(((1-(hydroxymethyl) cyclopropyl)amino)methyl)-9-methyl-1,12-dihydro-14*H*-pyrano[3',4':6,7] indolizino[1,2-*b*]quinoline-3,14(4*H*)-dione (Compound 19)**

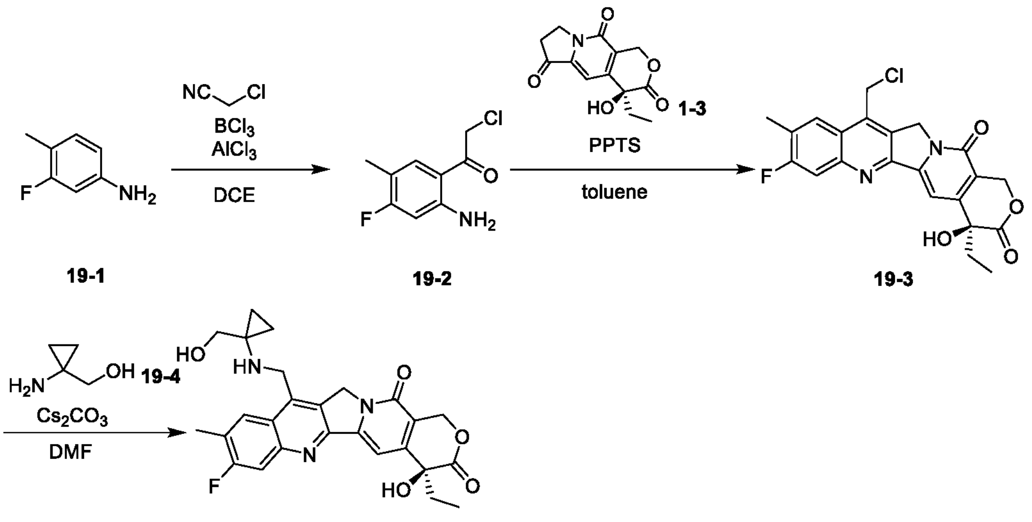


19-1

19-2

19-3

Compound 19

**Step 1: Synthesis of 1-(2-amino-4-fluoro-5-methylphenyl)-2-chloroethanone (Intermediate 19-2)**

Boron trichloride (749.03 mg, 6.39 mml) was dissolved in dichloroethane (8 mL). The reaction solution was cooled to 0°C, and to which were added **Intermediate 19-1** (1 g, 7.99 mmol) and chloroacetonitrile (723.94 mg, 9.59 mmol). The reaction solution was stirred at 0°C for 10 min, and to which was added aluminum trichloride (1.39 mg, 10.39 mmol). The reaction solution was then warmed to 25°C under nitrogen protection and stirred for 10 min. The reaction solution was stirred at 90°C under nitrogen protection for 18 h. After the reaction was completed, ice water (30 mL) and 5% HCl (10 mL) were added successively and slowly, and stirred at 25°C for 30 min after the reaction solution was cooled to room temperature. Then dichloromethane (50 ml) was added. The organic phase was washed with water (20 mL*2), and the washed organic phase was dried over an appropriate amount of anhydrous sodium sulfate. Preparative thin layer chromatography (silica, petroleum ether: ethyl acetate = 9 : 1, a small amount of ethanol) was performed to obtain the title compound (300 mg).

MS m/z (ESI): 201.8 $[M+H]^+$.

**$^1$H NMR (400MHz, Deuterated chloroform)** $\delta$ = 7 47 (d, *J* = 8.0 Hz, 1H), 6.35 (d, *J* = 11.3 Hz, 1H), 4.64 (s, 2H), 2.20 (s, 3H).

**Step 2: Synthesis of (*S*)-11-(chloromethyl)-4-ethyl-8-fluoro-4-hydroxy-9-methyl-1,12-dihydro-14*H*-pyrano[3',4':6,7]indolizino[1,2-*b*]quinoline-3,14(4*H*)-dione (Intermediate 19-3)**

**Intermediate 19-2** (200 mg, 991.94 $\mu$mol) and **Intermediate 1-3** (260.12 mg, 991.94 $\mu$mol) were dissolved in anhydrous toluene (3 mL), and to the mixture was added pyridinium p-toluenesulfonate (12.46 mg, 49.60 $\mu$mol). The reaction solution was stirred at 90°C under nitrogen protection for 18 h. After the reaction was completed, the reaction solution was filtered after the reaction solution was cooled to room temperature. The filter cake was washed with ethanol (3 mL*2) to obtain the crude of the title compound (223 mg).

MS m/z (ESI): 429.1 $[M+H]^+$.

**Step 3: Synthesis of (*S*)-4-ethyl-8-fluoro-4-hydroxy-11-(((1-(hydroxymethyl) cyclopropyl)amino)methyl)-9-methyl-1,12-dihydro-14*H*-pyrano[3',4':6,7] indolizino[1,2-*b*]quinoline-3,14(4*H*)-dione (Compound 19)**

**Intermediate 19-3** (20 mg, 46.64 $\mu$mol) and **Intermediate 19-4** (6.09 mg, 69.96 $\mu$mol) were dissolved in *N,N*-dimethylformamide (2 mL), and to the mixture was added cesium carbonate (30.39 mg, 93.27 $\mu$mol). The reaction solution was stirred at 40°C under nitrogen protection for 1 h. After the reaction was completed, the reaction solution was filtered, and the filtrate was purified by preparative high performance liquid chromatography (Waters Xbridge C18 column 5 $\mu$m, 25 mm in diameter, 100 mm in length; using a decreasingly polar mixture of water (containing 0.05% formic acid) and acetonitrile as the eluent; acetonitrile gradient proportion: 10%-40%, elution time: 12 min) to obtain the title compound (1.3 mg).

MS m/z (ESI): 480.1 $[M+H]^+$.

**$^1$H NMR (400MHz, DMSO-$d_6$)** $\delta$ = 9.35-9.20 (m, 1H), 8.45-8.30 (m, 1H), 8.12-7.84 (m, 1H), 7.35 (d, *J* = 6.5 Hz, 1H), 6.62-6.50 (m, 1H), 5.57-5.43 (m, 4H), 5.20-4.75 (m, 2H), 3.90-3.75 (m, 2H), 2.55 (s, 3H), 1.98-1.76 (m, 2H), 1.30-1.20 (m, 2H), 0.90-0.80 (m, 5H).

**Example 20. (*S*)-*N*-((8-ethyl-8-hydroxy-9,12-dioxo-8,9,12,14-tetrahydro-11*H*-furo[3,2-*f*]pyrano[3',4':6,7]indolizino[1,2-*b*]quinolin-15-yl)methyl)-2-hydroxyacetamide (Compound 20)**

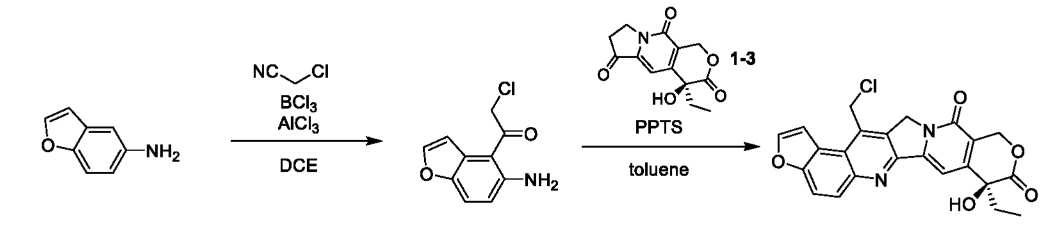


20-1 20-2 20-3

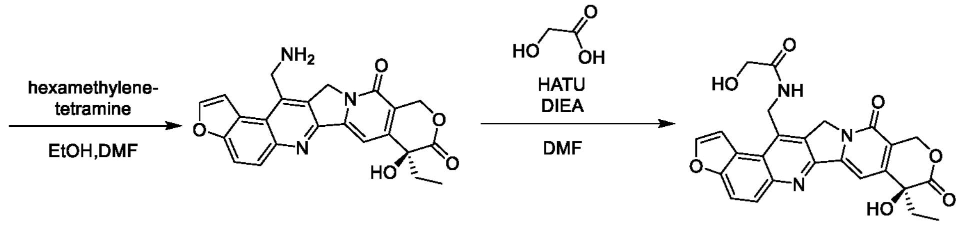


20-4 Compound 20

**Step 1: Synthesis of 1-(5-aminobenzofuran-4-yl)-2-chloroethanone (Intermediate 20-2)**

Boron trichloride (1 M, 9.61 mL) was dissolved in dichloroethane (14 mL). The reaction solution was cooled to 0°C, and to which were added **Intermediate 20-1** (1.6 g, 12.02 mmol) and chloroacetonitrile (1.36 g, 18.03 mmol). The reaction solution was stirred at 0°C for 10 min, and to which was added aluminum trichloride (1.92 g, 14.42 mmol). The reaction solution was then warmed to 25°C under nitrogen protection and stirred for 10 min. The reaction solution was stirred at 90°C under nitrogen protection for 18 h. After the reaction was completed, the reaction solution was cooled to room temperature. Ice water (50 mL) and 5% HCl (10 mL) were added successively and slowly, and stirred at 25°C for 30 min. Then dichloromethane (60 mL) was added. The organic phase was washed with water (30 mL*2), and the washed organic phase was dried over an appropriate amount of anhydrous sodium sulfate. Preparative thin layer chromatography (petroleum ether : (ethyl acetate+ethanol = 3 : 1) = 9 : 1) was performed to obtain the title compound (300 mg).

MS m/z (ESI): 210.0 $[M+H]^+$.

**$^1$H NMR (400MHz, Deuterated chloroform)** $\delta$ = 7.72 (d, *J* = 2.1 Hz, 1H), 7.53 (d, *J* = 9.0 Hz, 1H), 6.89 (d, *J* = 1.4 Hz, 1H), 6.66 (d, *J* = 9.0 Hz, 1H), 4.78 (s, 2H)

**Step 2: Synthesis of (*S*)-15-(chloromethyl)-8-ethyl-8-hydroxy-11,14-dihydro-12*H*-furo[3,2-*f*]pyrano[3',4':6,7]indolizino[1,2-*b*]quinoline-9,12(8*H*)-dione (Intermediate 20-3)**

**Intermediate 20-2** (200 mg, 954.07 μmol) and **Intermediate 1-3** (251.15 mg, 954.07 μmol) were dissolved in anhydrous toluene (4 mL), and to the mixture was added pyridinium p-toluenesulfonate (23.98 mg, 95.41 μmol). The reaction solution was stirred at 90°C under nitrogen protection for 16 h. After the reaction was completed, the reaction solution was cooled to room temperature, and filtered. The filter cake was washed with ethanol (3 mL*2) to obtain the title compound (300 mg).

MS m/z (ESI): 437.1 $[M+H]^+$.

**Step 3: Synthesis of (*S*)-15-(aminomethyl)-8-ethyl-8-hydroxy-11,14-dihydro-12*H*-furo[3,2-*f*]pyrano[3',4':6,7]indolizino[1,2-*b*]quinoline-9,12(8*H*)-dione (Intermediate 20-4)**

**Intermediate 20-3** (70 mg, 160.24 μmol) was dissolved in ethanol (0.5 mL) and anhydrous *N,N*-dimethylformamide (0.5 mL), and to the mixture was added hexamethylenetetramine (89.85 mg, 640.96 μmol). The reaction solution was stirred at 25°C for 3 h. After the reaction was completed, the reaction solution was cooled to room temperature, concentrated to dryness under reduced pressure, and purified by preparative high performance liquid chromatography (YMC-Actus Triart C18 column 5 μm, 25 mm in diameter, 100 mm in length; using a decreasingly polar mixture of water (containing 0.225% formic acid) and methanol as the eluent; methanol gradient proportion: 5%-25%, elution time: 12 min) to obtain the title compound (17 mg).

MS m/z (ESI):418.1 $[M+H]^+$.

**Step 4: (*S*)-*N*-((8-ethyl-8-hydroxy-9,12-dioxo-8,9,12,14-tetrahydro-11*H* furo[3,2-*f*]pyrano[3',4':6,7]indolizino[1,2-*b*]quinolin-15-yl)methyl)-2-hydroxyacetamide (Compound 20)**

**Intermediate 20-4** (5.00 mg, 11.98 μmol) and hydroxyacetic acid (4.55 mg, 59.89 μmol) were dissolved in anhydrous *N,N*-dimethylformamide (0.5 mL), and to the mixture were added HATU (6.83 mg, 17.97 μmol) and *N,N*-diisopropylethylamine (4.64 mg, 35.94 μmol). The reaction solution was stirred at 25°C for 1 h. After the reaction was completed, the reaction solution was filtered, and purified by preparative high performance liquid chromatography (YMC-Actus Triart C18 column 5 μm, 25 mm in diameter, 100 mm in length; using a decreasingly polar mixture of water (containing 0.05% formic acid) and acetonitrile as the eluent; acetonitrile gradient proportion: 15%-35%, elution time: 12 min) to obtain the title compound (3 mg).

MS m/z (ESI): 476.2 $[M+H]^+$.

**$^1$H NMR (400MHz, DMSO-$d_6$)** δ = 8.48-8.41 (m, 1H), 8.35 (d, *J* = 1.8 Hz, 1H), 8.22 (d, *J* = 8.0 Hz, 1H), 8.14 (d, *J* = 8.0 Hz, 1H), 7.76 (s, 1H), 7.35 (s, 1H), 6.54 (s, 1H), 5.58 (t, *J* = 5.6 Hz, 1H), 5.52 (s, 2H), 5.45 (s, 2H), 5.10 (d, *J* = 5.3 Hz, 2H), 3.88 (d, *J* = 5.6 Hz, 2H), 1.93-1.82 (m, 2H), 0.89 (t, *J* = 7.2 Hz, 3H).

**Example 21. (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxyacetamide (Compound 21)**

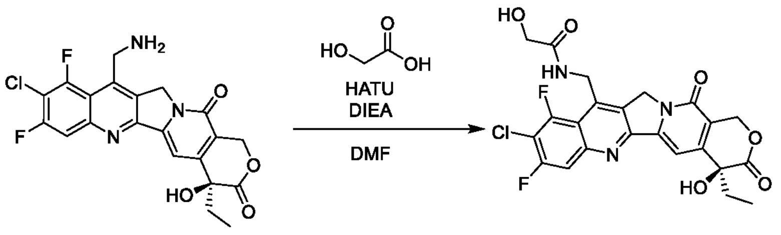


Compound 12

Compoun 21

**Step 1: Synthesis of (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxyacetamide (Compound 21)**

**Compound 12** (7 mg, 15.63 μmol) and hydroxyacetic acid (1.78 mg, 25.48 μmol) were dissolved in anhydrous *N,N*-dimethylformamide (0.5 mL), and to the mixture were added HATU (11.89 mg, 31.26 μmol) and diisopropylethylamine (2.02 mg, 15.63 μmol). The reaction solution was stirred at 25°C for 1 h. After the reaction was completed, the reaction solution was filtered, and the filtrate was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 20%-40%, elution time: 12 min) to obtain the title compound (1 mg).

MS m/z (ESI): 506.1 $[M+H]^+$.

**$^1$H NMR (400MHz, Methanol-$d_4$)** δ = 8.35 (t, *J* = 5.8 Hz, 1H), 8.15 (dd, *J* = 1.8, 9.8 Hz, 1H), 7.36 (s, 1H), 6.57 (s, 1H), 5.59-5.50 (m, 3H), 5.45 (s, 2H), 4.91 (d, *J*= 3.2 Hz, 2H), 3.84 (d, *J* = 5.7 Hz, 2H), 1.90-1.80 (m, 2H), 0.87 (t, *J* = 7.3 Hz, 3H).

**Example 22. (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxy-2-methylpropanamide (Compound 22)**

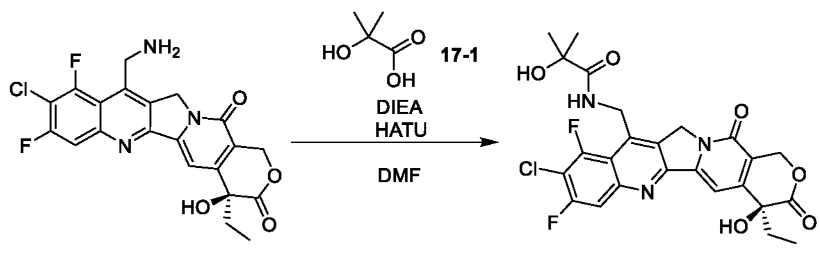


**Compound 12** **Compound 22**

**Step 1: (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxy-2-methylpropanamide (Compound 22)**

**Compound 12** (20 mg, 44.66 μmol) and **Intermediate 17-1** (13.95 mg, 133.98 μmol) were dissolved in *N,N*-dimethylformamide (0.5 mL), and to the mixture were added HATU (25.47 mg, 66.99 μmol) and *N,N*-diisopropylethylamine (17.32 mg, 133.98 μmol). The reaction solution was stirred at 25°C for 1 h. After the reaction was completed, the reaction solution was filtered, and purified by preparative high performance liquid chromatography (YMC-Actus Triart C18 column 5 μm, 25 mm in diameter, 100 mm in length; using a decreasingly polar mixture of water (containing 0.225% formic acid) and acetonitrile as the eluent; acetonitrile gradient proportion: 20%-40%, elution time: 12 min) to obtain the title compound (1.07 mg).

MS m/z (ESI): 534.2 $[M+H]^+$.

**$^1$H NMR (400MΣlz, DMSO-*d*$_6$)** δ = 8.30 (t, *J* = 5.9 Hz, 1H), 8.19-8.07 (m, 1H), 7.36 (s, 1H), 6.56 (s, 1H), 5.55-5.45 (m, 3H), 5.45 (s, 2H), 4.90 (d, *J* = 3.7 Hz, 2H), 1.90-1.82 (m, 2H), 1.24 (d, *J* = 4.3 Hz, 6H), 0.87 (t, *J* = 7.3 Hz, 3H).

**Example 23. *N*-(((*S*)-9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-cyclopropyl-2-hydroxyacetamide (Compound 23)**

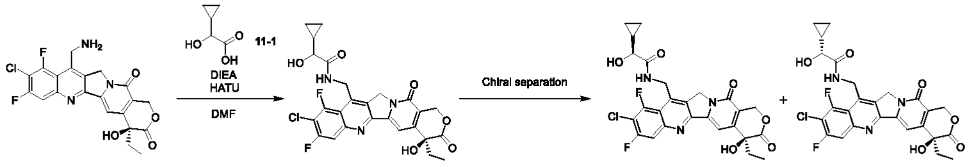


**Compound 12** **Compound 23**

**Step 1: *N*-(((*S*)-9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-cyclopropyl-2-hydroxyacetamide (Compound 23)**

**Compound 12** (7.0 mg, 15.63 μmol) and **Intermediate 11-1** (9.08 mg, 78.16 μmol) were dissolved in *N,N*-dimethylformamide (0.5 mL), and to the mixture were added HATU (8.92 mg, 23.45 μmol) and diisopropylethylamine (6.06 mg, 46.89 μmol). The reaction solution was stirred at 25°C for 1 h. After the reaction was completed, the reaction solution was filtered, and purified by preparative high performance liquid chromatography (YMC-Actus Triart C18 column 5 μm, 25 mm in diameter, 100 mm in length; using a decreasingly polar mixture of water (containing 0.225% formic acid) and acetonitrile as the eluent; acetonitrile gradient proportion: 41%-61%, elution time: 12 min) to obtain the title compound (7 mg).

MS m/z (ESI): 546.2 $[M+H]^+$.

**Compound 23** (7 mg) was separated and purified by preparative supercritical fluid chromatography (column: DAICEL CHIRALCEL OD-H (250 mm*30 mm, 5 μm); mobile phase: A: carbon dioxide; B: ethanol; B%: 50%; flow rate: 80 ml/min) to obtain **Compound 23-P1** (2.1 mg, RT: 5.106 min) and **Compound 23-P2** (2.09 mg, RT: 5.641 min).

**Compound 23-P1:**

**$^1$H NMR (400MHz, DMSO-*d*$_6$)** δ = 8.32 (t, *J* = 5.5 Hz, 1H), 8.15 (d, *J* = 9.7 Hz, 1H), 7.36 (s, 1H), 6.57 (s, 1H), 5.53 (s, 2H), 5.47 (d, *J* = 5.1 Hz, 1H), 5.45 (s, 2H), 4.93-4.86 (m, 2H), 2.02-1.96 (m, 1H), 1.91-1.81 (m, 2H), 1.04-0.96

(m, 1H), 0.87 (t, $J$ = 7.3 Hz, 3H), 0.37-0.31 (m, 2H), 0.29-0.23 (m, 2H).

MS m/z (ESI): 546.2 $[M+H]^+$.

**Compound 23-P2:**

**$^1$H NMR (400MΣlz, DMSO-$d_6$)** $\delta$ = 8.37-8.29 (m, 1H), 8.15 (d, $J$ = 9.9 Hz, 1H), 7.36 (s, 1H), 6.57 (s, 1H), 5.53 (s, 2H), 5.50-5.46 (m, 1H), 5.45 (s, 2H), 4.96-4.86 (m, 2H), 2.10-1.95 (m, 1H), 1.92-1.81 (m, 2H), 1.04-0.98 (m, 1H), 0.87 (t, $J$ = 7.3 Hz, 3H), 0.40-0.31 (m, 2H), 0.30-0.25 (m, 2H).

MS m/z (ESI): 546.2 $[M+H]^+$.

**Example 24. (*S*)-9-chloro-4-ethyl-8,10-difluoro-4-hydroxy-11-(((1-(hydroxymethyl)cyclopropyl)amino)methyl)-1,12-dihydro-14*H*-pyrano[3',4':6,7]indolizino[1,2-*b*]quinoline-3,14(4*H*)-dione (Compound 24)**

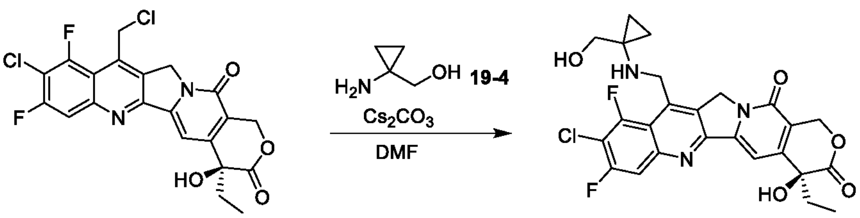


**12-3** **Compound 24**

**Step 1: Synthesis of (*S*)-9-chloro-4-ethyl-8,10-difluoro-4-hydroxy-11-(((1-(hydroxymethyl)cyclopropyl)amino)methyl)-1,12-dihydro-14*H*-pyrano [3',4':6,7]indolizino[1,2-*b*]quinoline-3,14(4*H*)-dione (Compound 24)**

**Intermediate 12-3** (30 mg, 64.21 μmol) and **Intermediate 19-4** (27.97 mg, 321.03 μmol) were dissolved in *N,N*-dimethylformamide (2 mL), and to the mixture was added cesium carbonate (41.84 mg, 128.41 μmol). The reaction solution was stirred at 40°C under nitrogen protection for 1 h. After the reaction was completed, the reaction solution was filtered, and the filtrate was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 30%-50%, elution time: 12 min) to obtain the title compound (3.1 mg).

MS m/z (ESI): 518.1 $[M+H]^+$.

**$^1$H NMR (400MHz, DMSO-$d_6$)** $\delta$ = 8.13-7.97 (m, 1H), 7.26 (s, 1H), 6.80-6.68 (m, 1H), 6.56 (s, 1H), 5.45-5.35 (m, 2H), 4.25-4.15 (m, 1H), 4.10-4.05 (m, 1H), 2.98-2.90 (m, 3H), 2.87-2.72 (m, 1H), 2.69-2.56 (m, 1H), 2.00-1.78 (m, 2H), 0.87 (t, $J$ = 7.2 Hz, 3H), 0.32-0.19 (m, 2H), 0.18-0.01 (m, 2H).

**Example 25. (*R*)-*N*-(((*S*)-9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxypropanamide (Compound 25)**

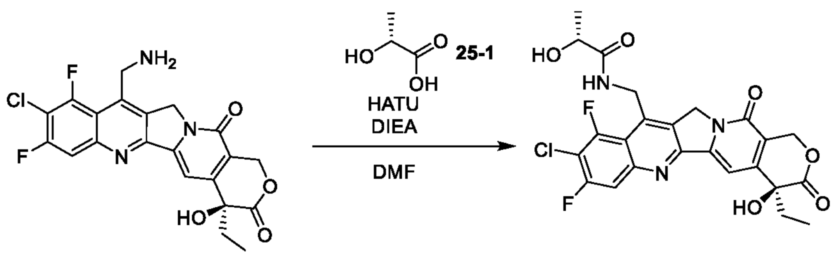


**Compound 12** **Compound 25**

**Step 1: Synthesis of (*R*)-*N*-(((*S*)-9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxypropanamide (Compound 25)**

**Compound 12** (6 mg, 13.40 μmol) and **Intermediate 25-1** (2.41 mg, 26.80 μmol) were dissolved in anhydrous *N,N*-dimethylformamide (0.5 mL), and to the mixture were added HATU (10.09 mg, 26.80 μmol) and diisopropylethylamine (1.73 mg, 13.49 μmol). The reaction solution was stirred at 25°C for 1 h. After the reaction was completed, the reaction solution was filtered, and the filtrate was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 20%-40%, elution time: 12 min) to obtain the title compound (1.80 mg).

MS m/z (ESI): 520.1 $[M+H]^+$.

**$^1$H NMR (400MHz, DMSO-$d_6$)** δ = 8.34 (t, *J* = 5.7 Hz, 1H), 8.15 (d, *J* = 9.9 Hz, 1H), 7.36 (s, 1H), 6.57 (s, 1H), 5.59 (d, *J* = 5.0 Hz, 1H), 5.51 (s, 2H), 5.45 (s, 2H), 4.90 (s, 2H), 4.13-3.89 (m, 1H), 1.95-1.77 (m, 2H), 1.21 (d, *J* =6.8 Hz, 3H), 0.87 (t, *J* = 7.3 Hz, 3H).

**Example 26. (*S*)-*N*-(((*S*)-9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxypropanamide (Compound 26)**

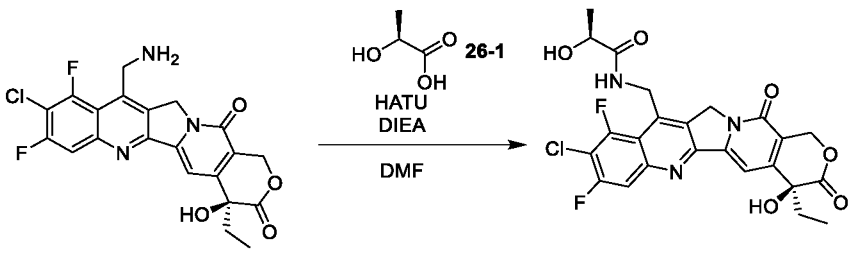


Compound 12 Compound 26

**Step 1: Synthesis of (*S*)-*N*-(((*S*)-9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-2-hydroxypropanamide (Compound 26)**

**Compound 12** (6 mg, 13.40 μmol) and **Intermediate 26-1** (2.41 mg, 26.80 μmol) were dissolved in anhydrous *N,N*-dimethylformamide (0.5 mL), and to the mixture were added HATU (10.09 mg, 26.80 μmol) and *N,N*-diisopropylethylamine (1.73 mg, 13.49 μmol). The reaction solution was stirred at 25°C for 1 h. After the reaction was completed, the reaction solution was filtered, and the filtrate was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 20%-40%, elution time: 12 min) to obtain the title compound (1.60 mg).

MS m/z (ESI): 520.1 $[M+H]^+$.

**$^1$H NMR (400MHz, DMSO-$d_6$)** δ = 8.34 (t, *J* = 5.7 Hz, 1H), 8.23-8.03 (m, 1H), 7.36 (s, 1H), 6.57 (s, 1H), 5.59 (d, *J* = 5.0 Hz, 1H), 5.51 (s, 2H), 5.45 (s, 2H), 4.90 (s, 2H), 4.07-3.96 (m, 1H), 1.93-1.81 (m, 2H), 1.21 (d, *J* = 6.8 Hz, 3H), 0.87 (t, *J* = 7.3 Hz, 3H).

**Example 27. (*S*)-*N*-((4-chloro-8-ethyl-8-hydroxy-9,12-dioxo-8,9,12,14-tetrahydro-11*H*-furo[3,2-*f*]pyrano[3',4':6,7]indolizino[1,2-*b*]quinolin-15-yl)methyl)-2-hydroxyacetamide (Compound 27)**

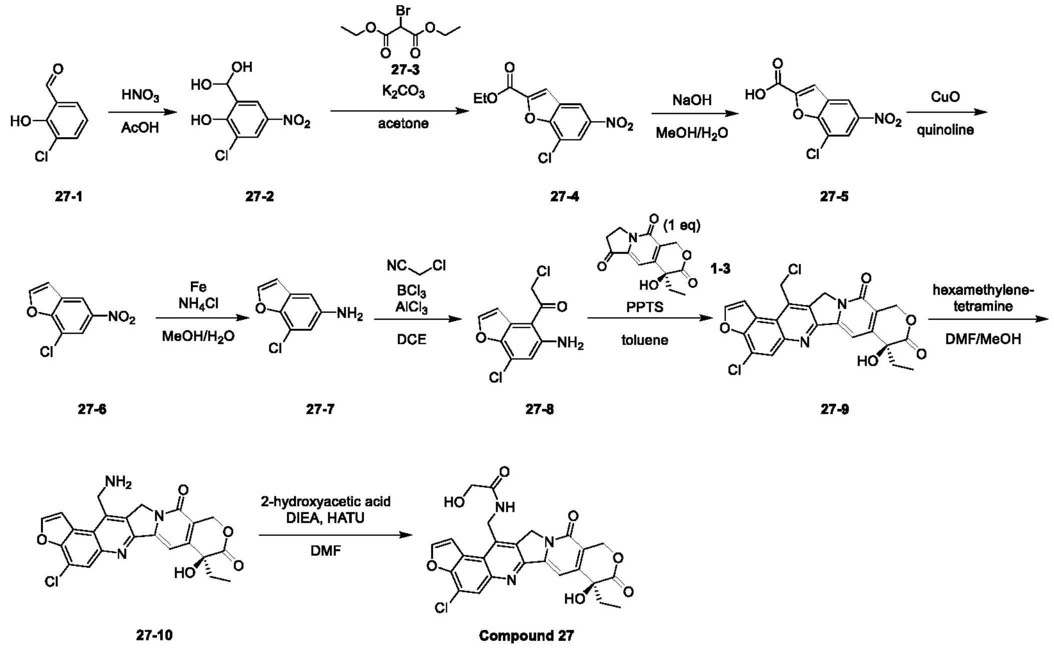


**Step 1: Synthesis of (3-chloro-2-hydroxy-5-nitrophenyl)methanediol (Intermediate 27-2)**

**Intermediate 27-2** (6 g) was dissolved in acetic acid (25 mL). The reaction solution was cooled to 0°C, and to which was slowly added nitric acid (9.64 g). The reaction solution was stirred at 25°C for 4 h. After the reaction was completed, the reaction solution was added slowly dropwise to ice water, and then the resulting mixture was extracted three times with ethyl acetate (60 mL). The organic phase was dried over anhydrous sodium sulfate. After filtration, the filtrate was concentrated to dryness under reduced pressure to obtain the title compound (5.88 g).

**$^1$H NMR (400MHz, METHANOL-*d4*)** δ = 8.16 (d, J=1.4, 2.4 Hz, 1H), 8.07-8.01 (m, 1H), 5.68 (s, 1H).

**Step 2: Synthesis of ethyl 7-chloro-5-nitrobenzofuran-2-carboxylate (Intermediate 27-4)**

**Intermediate 27-2** (5.88 g), **Intermediate 27-3** (7.69 g) and potassium carbonate (7.41g) were dissolved in acetone (60 mL), and the reaction solution was stirred at 70°C for 6 h. After the reaction was completed, to the reaction solution was added water (50 ml), and then the resulting mixture was extracted three times with ethyl acetate (50 mL). The organic phase was dried over anhydrous sodium sulfate. After filtration, the filtrate was concentrated to dryness under reduced pressure to obtain the title compound (4.5 g).

**$^1$H NMR (400MHz , Deuterated chloroform)** δ = 8 55 (d, *J* = 2.1 Hz, 1H), 8 39 (d, *J* = 2.1 Hz, 1H), 7.68 (s, 1H), 4.49 (q, *J* = 7.1 Hz, 2H), 1.46 (t, *J* = 7.1 Hz, 3H).

**Step 3: Synthesis of 7-chloro-5-nitrobenzofuran-2-carboxylic acid (Intermediate 27-5)**

**Intermediate 27-4** (4.5 g) was dissolved in methanol (50 mL), and to the mixture was added slowly dropwise an aqueous solution of sodium hydroxide (2 g in 25 ml $H_2O$). The reaction solution was stirred at 25°C for 3 h. After the reaction was completed, to the reaction solution was added water (120 ml), and then the resulting mixture was extracted twice with ethyl acetate (50 mL). The aqueous phase was adjusted to pH 1 with dilute hydrochloric acid, and then extracted three times with ethyl acetate (50 mL). The organic phase was dried over anhydrous sodium sulfate. After filtration, the filtrate was concentrated to dryness under reduced pressure to obtain the title compound (4.0 g).

**$^1$H NMR (400MHz , Deuterated chloroform)** δ = 8.45 (d, *J* = 2.1 Hz, 1H), 8.25 (d, *J* = 2.1 Hz, 1H), 7.58 (s, 1H).

**Step 4: Synthesis of 7-chloro-5-nitrobenzofuran (Intermediate 27-6)**

**Intermediate 27-5** (4 g) and cupric oxide (1.05 g) were dissolved in quinoline (28 mL), nitrogen was introduced into the reaction solution, and the mixture was stirred at 200°C for 0.5 h. After the reaction was completed, the reaction solution was cooled to 0°C, and to which was added slowly dropwise dilute hydrochloric acid (80 ml), and then water (30 ml) was added. The resulting mixture was extracted three times with ethyl acetate (30 mL). The organic phase was dried over anhydrous sodium sulfate. After filtration, the filtrate was concentrated to dryness under reduced pressure to

obtain the title compound (2.4 g).

**$^1$H NMR (400MHz , Deuterated chloroform)** δ = 8.49 (d, *J* = 2.1 Hz, 1H), 8.31 (d, *J* = 2.1 Hz, 1H), 7.88 (d, *J* = 2.3 Hz, 1H), 7.02 (d, *J* = 2.3 Hz, 1H)

**Step 5: Synthesis of 7-chlorobenzofuran-5-amine (Intermediate 27-7)**

**Intermediate 27-6** (2.4 g) and iron powder (1.55 g) were dissolved in methanol (5 mL), and to the mixture was added dropwise an aqueous solution of ammonium chloride (148.91 mg, 5 ml), nitrogen was introduced into the reaction solution, and the mixture was stirred at 80°C for 0.5 h. After the reaction was completed, the reaction solution was cooled to 25°C, and to which was added water (10 ml), and the resulting mixture was extracted three times with ethyl acetate (10 mL). The organic phase was dried over anhydrous sodium sulfate. After filtration, the filtrate was concentrated to dryness under reduced pressure to obtain the title compound (1.2 g).

MS m/z (ESI): 167.8[M+H]$^+$.

**Step 6: Synthesis of 1-(5-amino-7-chlorobenzofuran-4-yl)-2-chloroethan-1-one (Intermediate 27-8)**

Boron trichloride (671.17 mg) was dissolved in 1,2-dichloroethane (8 mL). The reaction solution was cooled to 0°C, and to which were added **Intermediate 27-7** (1.2 g) and chloroacetonitrile (702.75 mg). The reaction solution was stirred at 0°C for 10 min, and to which was added aluminum trichloride (1.24 g). The reaction solution was then warmed to 25°C under nitrogen protection and stirred for 10 min. The reaction solution was stirred at 90°C under nitrogen protection for 18 h. After the reaction was completed, ice water (5 mL) and 5% HCl (1 mL) were added successively and slowly, and stirred at 25°C for 30 min after the reaction solution was cooled to room temperature. Then dichloromethane (4 mL) was added. The organic phase was washed with water (2 mL*2), and the washed organic phase was dried over an appropriate amount of anhydrous sodium sulfate. After filtration, the filtrate was concentrated to dryness under reduced pressure. The residue was subjected to preparative thin layer chromatography (silica, petroleum ether: (3/1 mixed solvent of ethyl acetate and ethanol) = 9 : 1) to obtain the title compound (500 mg).

MS m/z (ESI): 243.8 [M+H]$^+$.

**Step 7: Synthesis of (*S*)-4-chloro-15-(chloromethyl)-8-ethyl-8-hydroxy-11,14-dihydro-12*H*-furo[3,2-*f*]pyrano[3',4':6,7]indolizino[1,2-*b*]quinoline-9,12(8*H*)-dione (Intermediate 27-9)**

**Intermediate 27-8** (450 mg) and **Intermediate 1-3** (480.35 mg) were dissolved in toluene (5 mL), and to the mixture was added pyridinium p-toluenesulfonate (23.17 mg). The reaction solution was stirred at 90°C for 18 h. After the reaction was completed, ethanol (1 mL) was added after the reaction solution was cooled to room temperature. The reaction solution was stirred at 25°C for 0.5 h. The reaction solution was filtered. The filter cake was washed with petroleum ether (2 mL*2), and drying was performed to obtain the title compound (500 mg).

MS m/z (ESI): 471.0 [M+H]$^+$.

**Step 8: Synthesis of (S)-15-(aminomethyl)-4-chloro-8-ethyl-8-hydroxy-11,14-dihydro-12*H*-furo[3,2-*f*]pyrano[3',4':6,7]indolizino[1,2-*b*]quinoline-9,12(8*H*)-dione (Intermediate 27-10)**

**Intermediate 27-9** (450 mg) was dissolved in a mixed solution of methanol (1 mL) and *N,N*-dimethylformamide (1 mL), and to the mixture was added hexamethylenetetramine (267.71 mg). The reaction solution was stirred at 50°C for 4 h. After the reaction was completed, the reaction solution was cooled to room temperature. Concentrated hydrochloric acid (2 mL) was added. The resulting mixture was stirred, and then concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.225% formic acid) and acetonitrile as the eluent; acetonitrile gradient proportion: 13%-43%, elution time: 12 min) to obtain the title compound (60.0 mg).

MS m/z (ESI): 451.9 [M+H]$^+$.

**Step 9: Synthesis of (*S*)-*N*-((4-chloro-8-ethyl-8-hydroxy-9,12-dioxo-8,9,12, 14-tetrahydro-11*H*-furo[3,2-*f*]pyrano[3',4':6,7]indolizino[1,2-*b*]quinolin-15-yl)methyl)-2-hydroxyacetamide (Compound 27)**

**Intermediate 27-10** (10 mg) and hydroxyacetic acid (5.05 mg) were dissolved in anhydrous *N,N*-dimethylformamide (0.5 mL), and to the mixture were added HATU (16.83 mg) and diisopropylethylamine (2.86 mg). The reaction solution was stirred at 25°C for 1 h. After the reaction was completed, the reaction solution was filtered. The residue was purified by preparative high performance liquid chromatography (Waters Xbridge C18 column 5 μm, 25 mm in

diameter, 100 mm in length; using a decreasingly polar mixture of water (containing 0.05% formic acid) and acetonitrile as the eluent; acetonitrile gradient proportion: 28%-48%, elution time: 12 min) to obtain the title compound (6.0 mg).

MS m/z (ESI):510.1 $[M+H]^+$.

**$^1$H NMR (400MΣIz, DMSO-$d_6$)** δ = 8.46 (s, 2H), 8.36-8.20 (m, 1H), 7.85 (s, 1H), 7.35 (s, 1H), 6.54 (s, 1H), 5.58 (t, *J* = 5.6 Hz, 1H), 5.49 (d, *J* = 2.9 Hz, 2H), 5.45 (s, 2H), 5.07 (s, 2H), 3.88 (d, *J* = 5.6 Hz, 2H), 2.02-1.75 (m, 2H), 0.89 (t, *J* = 7.3 Hz, 3H).

**Example 28. N-(((5)-4-chloro-8-ethyl-8-hydroxy-9,12-dioxo-8,9,12,14-tetrahydro-11*H*-furo[3,2-*f*]pyrano[3',4':6,7]indolizino[1,2-*b*]quinolin-15-yl)methyl)-2-cyclopropyl-2-hydroxyacetamide (Compound 28)**

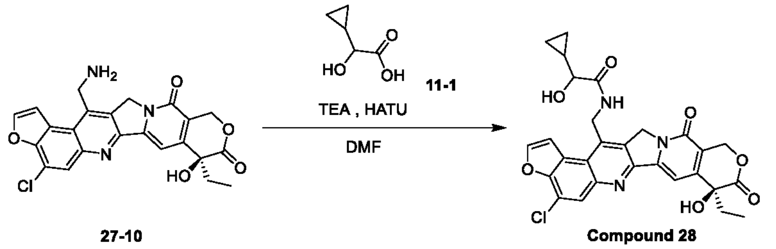


27-10 Compound 28

**Step 1: Synthesis of *N*-(((*S*)-4-chloro-8-ethyl-8-hydroxy-9,12-dioxo-8,9,12,14-tetrahydro-11*H*-furo[3,2-*f*]pyrano[3',4':6,7]indolizino[1,2-*b*]quinolin-15-yl)methyl)-2-cyclopropyl-2-hydroxyacetamide (Compound 28)**

**Intermediate 27-10** (10 mg) and **Intermediate 11-1** (8.34 mg) were dissolved in anhydrous *N,N*-dimethylformamide (0.5 mL), and to the mixture were added HATU (13.65 mg) and diisopropylethylamine (3.10 mg). The reaction solution was stirred at 25°C for 1 h. After the reaction was completed, the reaction solution was filtered. The filtrate was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 34%-54%, elution time: 12 min) to obtain the title compound (6.2 mg).

MS m/z (ESI): 550.1$[M+H]^+$.

**$^1$H NMR (400MHz, DMSO-$d_6$)** δ = 8.48-8.43 (m, 2H), 8.29 (s, 1H), 7.84 (s, 1H), 7.35 (s, 1H), 6.56 (s, 1H), 5.51 (s, 2H), 5.45 (s, 2H), 5.15-4.98 (m, 2H), 3.57 (d, *J* = 6.0 Hz, 1H), 1.95-1.80 (m, 2H), 1.10-1.00 (m, 1H), 0.89 (t, *J* =7.3 Hz, 3H), 0.41-0.32 (m, 2H), 0.32-0.24 (m, 2H).

**Example 29. 2-cyclopropyl-*N*-(((*S*)-7-ethyl-7-hydroxy-15-nitro-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 29)**

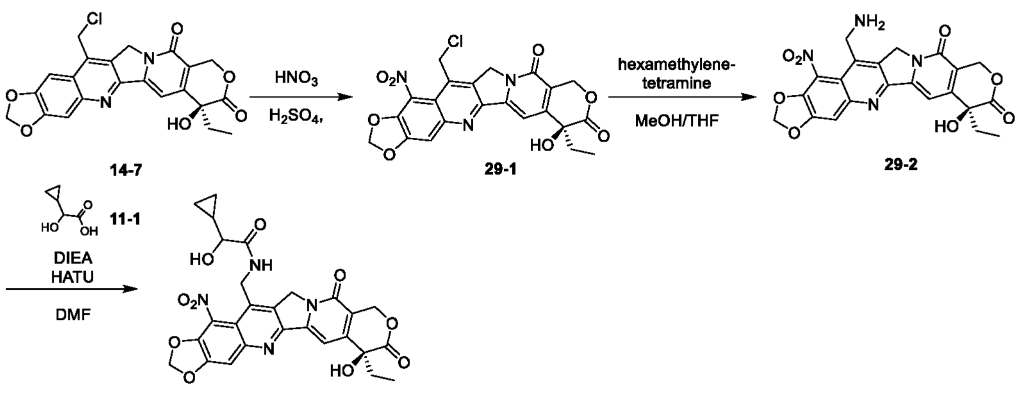


14-7 29-1 29-2

Compound 29

**Step 1: Synthesis of (*S*)-14-(chloromethyl)-7-ethyl-7-hydroxy-15-nitro-10,13-dihydro-11*H*-[1,3]dioxolo[4,5-g]pyrano[3',4':6,7]indolizino[1,2-*b*]quinoline-8,11(7*H*)-dione (Intermediate 29-1)**

**Intermediate 14-7** (500.0 mg) was dissolved in sulfuric acid (15 mL), and the reaction solution was cooled to 0°C. Then to the reaction solution was slowly added nitric acid (357.35 g, 70% purity). The reaction solution was stirred at 25°C for 1.5 h. After the reaction was completed, ice water (10 mL) and dichloromethane (30 mL) were added successively and slowly. The organic phase was washed with 40 mL of water (20 mL*2), and the washed organic phase was dried over an appropriate amount of anhydrous sodium sulfate. After filtration, the filtrate was concentrated to dryness under reduced pressure to obtain the crude of the title compound (280.0 mg).

MS m/z (ESI): 486.0 $[M+H]^+$.

**Step 2: Synthesis of (*S*)-14-(aminomethyl)-7-ethyl-7-hydroxy-15-nitro-10,13-dihydro-11*H*-[1,3]dioxolo[4,5-*g*]pyrano[3',4':6,7]indolizino[1,2-*b*]quinoline-8,11(7*H*)-dione (Intermediate 29-2)**

**Intermediate 29-1** (270.0 mg) was dissolved in methanol (2 mL) and tetrahydrofuran (2 mL), and to the mixture was added hexamethylenetetramine (233.73 mg). The reaction solution was stirred at 60°C for 16 h. After the reaction was completed, the reaction solution was concentrated to dryness under reduced pressure after the reaction solution was cooled to room temperature. The residue was purified by preparative high performance liquid chromatography (YMC-Pack CN C18 column, 5 μm silica, 30 mm in diameter, 150 mm in length; using a decreasingly polar mixture of water (containing 0.225% FA) and methanol as the eluent; methanol gradient proportion: 9%-29%, elution time: 12 min) to obtain the title compound (15.0 mg).

MS m/z (ESI): 467.1 $[M+H]^+$.

**Step 3: Synthesis of 2-cyclopropyl-*N*-(((*S*)-7-ethyl-7-hydroxy-15-nitro-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 29)**

**Intermediate 29-2** (12.00 mg) and **Intermediate 11-1** (14.94 mg) were dissolved in anhydrous *N,N*-dimethylformamide (1 mL), and to the mixture were added HATU (14.67 mg) and *N,N*-diisopropylethylamine (9.98 mg). The reaction solution was stirred at 25°C for 1.5 h. After the reaction was completed, the reaction solution was filtered. The filtrate was concentrated to dryness under reduced pressure. The residue was purified by preparative high performance liquid chromatography (Boston Prime C18 column, 5 μm silica, 30 mm in diameter, 150 mm in length; using a decreasingly polar mixture of water (containing 0.05% formic acid) and acetonitrile as the eluent; acetonitrile gradient proportion: 15%-45%, elution time: 12 min) to obtain the title compound (5.20 mg).

MS m/z (ESI): 565.2 $[M+H]^+$.

**$^1$H NMR (400MHz, DMSO-$d_6$)** δ = 8.25 (t, *J* = 5.3 Hz, 1H), 7.81 (s, 1H), 7.28 (s, 1H), 6.52 (d, *J* = 2.6 Hz, 3H), 5.55 (d, *J* = 4.9 Hz, 1H), 5.43 (s, 2H), 5.36 (s, 2H), 4.48 (d, *J* = 5.1 Hz, 2H), 3.53 (t, *J* = 5.7 Hz, 1H), 1.91-1.83 (m, 2H), 1.10-0.98 (s, 1H), 0.87 (t, *J* = 7.3 Hz, 3H), 0.42-0.28 (m, 4H).

**Example 30. *N*-(((*S*)-15-chloro-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-cyclopropyl-2-hydroxyacetamide (Compound 30)**

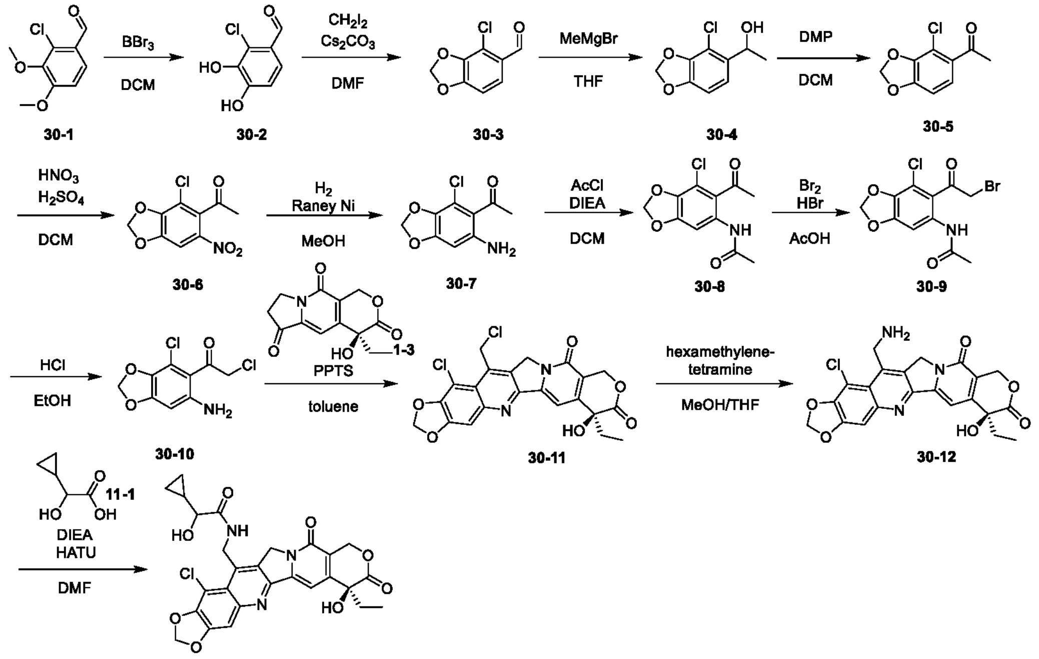


Compound 30

**Step 1: Synthesis of 2-chloro-3,4-dihydroxybenzaldehyde (Intermediate 30-2)**

**Intermediate 30-1** (20.0 g) was dissolved in anhydrous dichloromethane (100 mL). The reaction solution was cooled to 0°C, and to which was slowly added boron tribromide (87.41 g). The reaction solution was stirred at 25°C for 4 h. After the reaction was completed, the reaction solution was poured into ice water slowly, and then ethyl acetate (200 mL) was added. The organic phase was washed with water (100 mL*2), and the washed organic phase was dried over an appropriate amount of anhydrous sodium sulfate. After filtration, the filtrate was concentrated to dryness under reduced pressure, and the residue was stirred in and washed with an ethyl acetate: petroleum ether = 1: 4 solution. After filtration, the title compound (14 g) was obtained.

MS m/z (ESI): 173.1 $[M+H]^+$.

**Step 2: Synthesis of 4-chlorobenzo[*d*][1,3]dioxol-5-formaldehyde (Intermediate 30-3)**

**Intermediate 30-2** (10.0 g) was dissolved in anhydrous *N,N*-dimethylformamide (100 mL), and to the mixture were added cesium carbonate (28.32 g) and diiodomethane (23.28 g). The reaction solution was stirred at 100°C for 1 h. After the reaction was completed, water (100 mL) and ethyl acetate (150 mL) were added successively and slowly after the reaction solution was cooled to room temperature. The organic phase was washed with water (50 mL*2), and the washed organic phase was dried over an appropriate amount of anhydrous sodium sulfate. After filtration, the filtrate was concentrated to dryness under reduced pressure to obtain the title compound (5.2 g).

MS m/z (ESI): 185.4 $[M+H]^+$.

**Step 3: Synthesis of 1-(4-chlorobenzo[*d*][1,3]dioxol-5-yl)ethan-1-ol (Intermediate 30-4)**

**Intermediate 30-3** (5.0 g) was dissolved in anhydrous tetrahydrofuran (100 mL). The reaction solution was cooled to -78°C, and to which was slowly added methyl magnesium bromide (4.85 g, 3 M). The reaction solution was stirred at 25°C under nitrogen protection for 4 h. After the reaction was completed, water (50 mL) and ethyl acetate (100 mL) were added successively and slowly. The organic phase was washed with water (50 mL*2), and the washed organic phase was dried over an appropriate amount of anhydrous sodium sulfate. After filtration, the filtrate was concentrated to dryness under reduced pressure, and the residue was purified by preparative column chromatography (petroleum ether : ethyl acetate = 2 : 1) to obtain the title compound (5.3 g).

**$^1$H NMR (400MHz, Deuterated chloroform)** $\delta$ = 6.99 (d, *J* = 8.3 Hz, 1H), 6.69 (d, *J* = 8.1 Hz, 1H), 5.97 (s, 2H), 5.13 (q, *J* = 6.4 Hz, 1H), 1.41 (d, *J* = 6.4 Hz, 3H)

**Step 4: Synthesis of 1-(4-chlorobenzo[*d*][1,3]dioxol-5-yl)ethan-1-one (Intermediate 30-5)**

**Intermediate 30-4** (5.2 g) was dissolved in anhydrous dichloromethane (100 mL). The reaction solution was cooled to 0°C, and to which was slowly added Dess-Martin periodinane (DMP) (16.49 g). The reaction solution was stirred at 25°C under nitrogen protection for 2 h. After the reaction was completed, water (50 mL) and ethyl acetate (100 mL) were added successively and slowly. The organic phase was washed with water (50 mL*2), and the washed organic phase was dried over an appropriate amount of anhydrous sodium sulfate. After filtration, the filtrate was concentrated to dryness under reduced pressure, and the residue was purified by preparative column chromatography (petroleum ether:ethyl acetate = 3 : 1) to obtain the title compound (3.0 g).
MS m/z (ESI): 199.2 $[M+H]^+$.

**Step 5: Synthesis of 1-(4-chloro-6-nitrobenzo[*d*][1,3]dioxol-5-yl)ethan-1-one (Intermediate 30-6)**

**Intermediate 30-5** (2.50 g) was dissolved in anhydrous dichloromethane (20 mL), and to the mixture was slowly added concentrated sulfuric acid (1.23 g) and nitric acid (5.67 g). The reaction solution was stirred at 25°C for 3 h. After the reaction was completed, the reaction solution was poured into ice water slowly, and then ethyl acetate (150 mL) was added. The organic phase was washed with water (50 mL*2), and the washed organic phase was dried over an appropriate amount of anhydrous sodium sulfate. After filtration, the filtrate was concentrated to dryness under reduced pressure, and the residue was purified by preparative column chromatography (petroleum ether : ethyl acetate = 2 : 1) to obtain the title compound (1.8 g).
MS m/z (ESI): 244.1 $[M+H]^+$.

**Step 6: Synthesis of 1-(6-amino-4-chlorobenzo[*d*][1,3]dioxol-5-yl)ethan-1-one (Intermediate 30-7)**

**Intermediate 30-6** (0.80 g) was dissolved in absolute methanol (6 mL), and to which was added Raney Ni (400.0 mg). The reaction solution was stirred at 25°C under hydrogen atmosphere for 16 h. After the reaction was completed, the reaction solution was filtered, and the filtrate was concentrated to dryness under reduced pressure to obtain the title compound (510.0 mg).
MS m/z (ESI): 214.2 $[M+H]^+$.

**Step 7: Synthesis of *N*-(6-acetyl-7-chlorobenzo[*d*][1,3]dioxol-5-yl)acetamide (Intermediate 30-8)**

**Intermediate 30-7** (260.0 mg) was dissolved in anhydrous dichloromethane (5 mL). The reaction solution was cooled to 0°C, and to which were added *N,N*-diisopropylethylamine (235.95 mg) and acetyl chloride (143.32 mg). The reaction solution was stirred at 25°C for 1.5 h. After the reaction was completed, the reaction solution was concentrated to dryness under reduced pressure, and the residue was purified by preparative chromatography (petroleum ether : ethyl acetate = 5 : 1) to obtain the title compound (140.0 mg).
MS m/z (ESI): 256.0 $[M+H]^+$.

**Step 8: Synthesis of *N*-(6-(2-bromoacetyl)-7-chlorobenzo[*d*][1,3]dioxol-5-yl)acetamide (Intermediate 30-9)**

**Intermediate 30-8** (110.00 mg) was dissolved in acetic acid (2 mL), to the mixture was added a solution of hydrogen bromide in acetic acid (158.24 mg, 33% content), and then to which was slowly added liquid bromine (72.20 mg). The reaction solution was stirred at 25°C for 1 h. After the reaction was completed, the reaction solution was poured into ice water slowly, and then ethyl acetate (30 mL) was added. The organic phase was washed with water (20 mL*2), and the washed organic phase was dried over an appropriate amount of anhydrous sodium sulfate. The organic phase was concentrated to dryness under reduced pressure to obtain the title compound (110.0 mg).
MS m/z (ESI): 334.0 $[M+H]^+$.

**Step 9: Synthesis of 1-(6-amino-4-chlorobenzo[*d*][1,3]dioxol-5-yl)-2-chloroethan-1-one (Intermediate 30-10)**

**Intermediate 30-9** (110.00 mg) was dissolved in absolute alcohol (1 mL) and concentrated hydrochloric acid (1 mL), and the reaction solution was stirred at 60°C for 16 h. After the reaction was completed, ice water (10 mL) and saturated sodium bicarbonate (10 mL) were added successively and slowly after the reaction solution was cooled to room temperature. Then dichloromethane (30 mL) was added. The organic phase was washed with water (20 mL*2), and the washed organic phase was dried over an appropriate amount of anhydrous sodium sulfate. After filtration, the filtrate was concentrated to dryness under reduced pressure, and the residue was purified by preparative thin layer chromatography (petroleum ether:ethyl acetate = 3:1) to obtain the title compound (75 mg).

MS m/z (ESI): 248.0 $[M+H]^+$.

**Step 10: Synthesis of (*S*)-15-chloro-14-(chloromethyl)-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione (Intermediate 30-11)**

**Intermediate 30-10** (75.00 mg) and **Intermediate 1-3** (83.57 mg) were dissolved in toluene (3 mL), and to the mixture was added pyridinium p-toluenesulfonate (PPTS) (11.4 mg). The reaction solution was stirred at 90°C for 16 h. After the reaction was completed, ethanol (1 mL) was added after the reaction solution was cooled to room temperature. The reaction solution was stirred at 25°C for 0.5 h. The reaction solution was filtered. The filter cake was washed with ethanol (2 mL*2), and drying was performed to obtain the title compound (75.0 mg).
MS m/z (ESI): 475.1 $[M+H]^+$.

**Step 11: Synthesis of (*S*)-14-(aminomethyl)-15-chloro-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione (Intermediate 30-12)**

**Intermediate 30-11** (70.00 mg) was dissolved in absolute methanol (2 mL) and anhydrous tetrahydrofuran (1 mL), and to the mixture was added hexamethylenetetramine (61.94 mg). The reaction solution was stirred at 80°C for 2 h. After the reaction was completed, the reaction solution was concentrated to dryness under reduced pressure after the reaction solution was cooled to room temperature. The residue was purified by preparative high performance liquid chromatography (Boston Prime C18 column, 5 $\mu$m silica, 30 mm in diameter, 150 mm in length; using a decreasingly polar mixture of water (containing 0.225% formic acid) and acetonitrile as the eluent; acetonitrile gradient proportion: 5%-25%, elution time: 12 min) to obtain the title compound (16.0 mg).
MS m/z (ESI): 456.1 $[M+H]^+$.

**Step 12: Synthesis of *N*-(((*S*)-15-chloro-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-cyclopropyl-2-hydroxyacetamide (Compound 30)**

**Intermediate 30-12** (5.00 mg) and **Intermediate 11-1** (6.37 mg) were dissolved in anhydrous *N,N*-dimethylformamide (0.5 mL), and to the mixture were added HATU (6.26 mg) and *N,N*-diisopropylethylamine (4.25 mg). The reaction solution was stirred at 25°C for 1 h. After the reaction was completed, the reaction solution was filtered. The filtrate was concentrated to dryness under reduced pressure. The residue was purified by preparative high performance liquid chromatography (Boston Prime C18 column, 5 $\mu$m silica, 30 mm in diameter, 150 mm in length; using a decreasingly polar mixture of water (containing 0.05% formic acid) and acetonitrile as the eluent; acetonitrile gradient proportion: 31%-51%, elution time: 12 min) to obtain the title compound (2.30 mg).
MS m/z (ESI): 554.2 $[M+H]^+$.
**$^1$H NMR (400MHz, DMSO-$d_6$)** $\delta$ = 7.78 (t, *J* = 5.4 Hz, 1H), 7.35 (s, 1H), 7.01 (s, 1H), 6.30-6.25 (m, 1H), 6.16 (d, *J* = 1.5 Hz, 2H), 5.27-5.21 (m, 2H), 5.19 (s, 2H), 4.93-4.78 (m, 2H), 3.29 (dd, *J* = 2.4, 6.1 Hz, 1H), 1.68-1.56 (m, 2H), 0.81-0.72 (m, 1H), 0.63 (t, *J* = 7.3 Hz, 3H), 0.14-0.06 (m, 2H), 0.05-0.03 (m, 2H).

**Example 31. (*S*)-*N*-((15-chloro-7-ethyl-7-hydroxy-8,11-dioxo-7,8,11,13-tetrahydro-10*H*-[1,3]dioxolo[4,5-*g*] pyrano[3',4':6,7]indolizino[1,2-*b*]quinolin-14-yl)methyl)-2-hydroxyacetamide (Compound 31)**

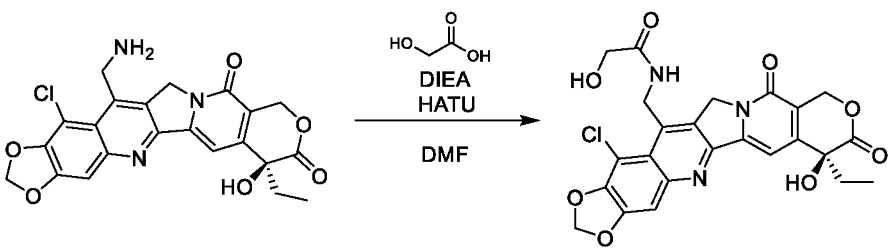


30-12 Compound 31

**Step 1: Synthesis of (*S*)-*N*-((15-chloro-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)-2-hydroxyacetamide (Compound 31)**

**Intermediate 30-12** (5 mg) and hydroxyacetic acid (4.17 mg) were dissolved in *N,N*-dimethylformamide (0.5

mL), and to the mixture were added HATU (6.26 mg) and *N,N*-diisopropylethylamine (4.25 mg). The reaction solution was stirred at 25°C for 1 h. After the reaction was completed, the reaction solution was filtered. The filtrate was concentrated to dryness under reduced pressure. The residue was purified by preparative high performance liquid chromatography (Boston Prime C18 column, 5 μm silica, 30 mm in diameter, 150 mm in length; using a decreasingly polar mixture of water (containing 0.225% formic acid) and acetonitrile as the eluent; acetonitrile gradient proportion: 20%-40%, elution time: 12 min) to obtain the title compound (2.50 mg).

MS m/z (ESI): 514.2 $[M+H]^+$.

**$^1$H NMR (400MHz, DMSO-*$d_6$*)** δ = 8.03 (t, *J* = 5.8 Hz, 1H), 7.60 (s, 1H), 7.25 (s, 1H), 6.54-6.48 (m, 1H), 6.41 (s, 2H), 5.48 (s, 2H), 5.43 (s, 2H), 5.12 (d, *J* = 6.0 Hz, 2H), 3.82 (s, 2H), 1.93-1.78 (m, 2H), 0.87 (t, *J* = 7.4 Hz, 3H).

**Example 32. (*S*)-*N*-((9-chloro-4-ethyl-8,10-difluoro-4-hydroxy-3,14-dioxo-3,4,12,14-tetrahydro-1*H*-pyrano[3',4':6,7]indolizino[1,2-*b*]quinolin-11-yl)methyl)-3-hydroxypropanamide (Compound 32)**

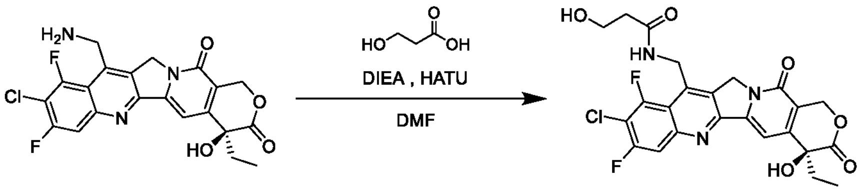


**Compound 12** **Compound 32**

**Compound 12** (10 mg) and hydroxy propanoic acid (2.21 mg) were dissolved in anhydrous *N,N*-dimethylformamide (0.5 mL), and to the mixture were added HATU (12.70 mg) and *N,N*-diisopropylethylamine (2.89 mg). The reaction solution was stirred at 25°C for 1 h. After the reaction was completed, the reaction solution was filtered. The filtrate was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 28%-48%, elution time: 12 min) to obtain the title compound (0.80 mg).

MS m/z (ESI): 520.1 $[M+H]^+$.

**$^1$H NMR (400MHz, DMSO-*$d_6$*)** δ = 8.54 (t, *J* = 5.6 Hz, 1H), 8.15 (d, *J* = 9.8 Hz, 1H), 7.35 (s, 1H), 6.57 (s, 1H), 5.54 (s, 2H), 5.45 (s, 2H), 4.85 (s, 2H), 4.54 (s, 1H), 3.57 (t, *J* = 6.5 Hz, 2H), 2.32-2.26 (m, 2H), 1.99-1.75 (m, 2H), 0.87 (t, *J* = 7.3 Hz, 3H).

**Example 33. (*S*)-*N*-((4-chloro-8-ethyl-8-hydroxy-9,12-dioxo-2,3,8,9,12,14-hexahydro-1*H*,11*H*-cyclopentadieno[*f*]pyrano[3',4':6,7]indolizino[1,2-*b*]quinolin-15-yl)methyl)-2-hydroxyacetamide (Compound 33)**

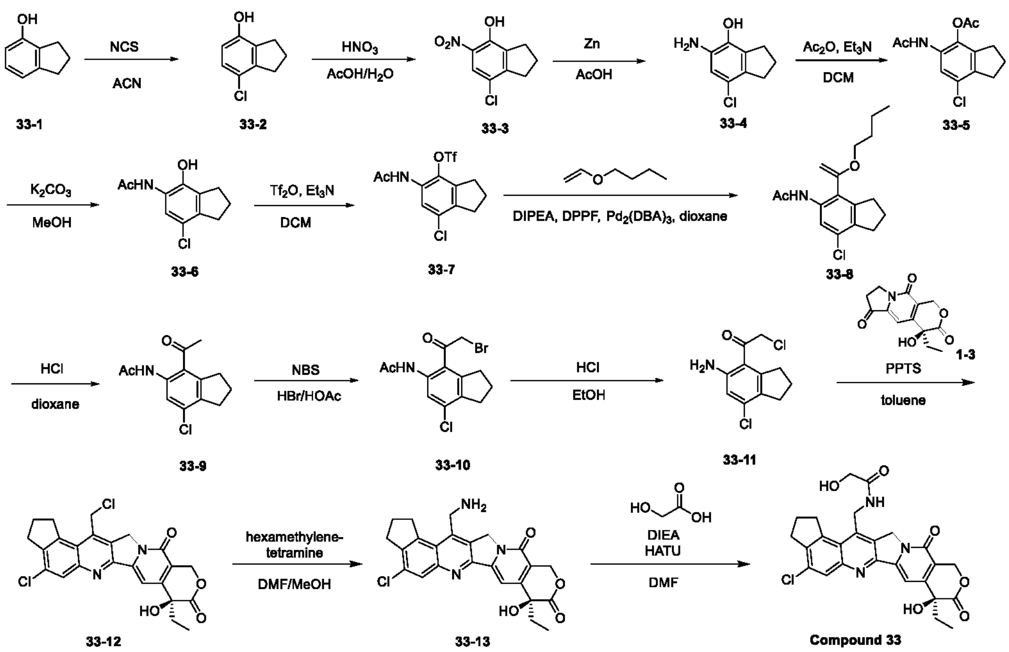


**Step 1: Synthesis of 7-chloro-2,3-dihydro-1*H*-inden-4-ol (Intermediate 33-2)**

**Intermediate 33-1** (500 mg) was dissolved in anhydrous acetonitrile (5 mL), and to the mixture was added NCS (547 mg). After the addition, stirring was performed at 25°C for 2 h. After the reaction was completed, to the reaction solution was added water (10 mL), and the resulting mixture was extracted with ethyl acetate (10 mL*3). The organic layer was washed with saturated brine (30 mL), and dried over anhydrous sodium sulfate. After filtration, the organic phase was concentrated under reduced pressure to remove the solvent, and the residue was purified by silica gel column chromatography (petroleum ether:ethyl acetate = 100 : 1 to 10 : 1) to obtain the title compound (600 mg).

MS m/z (ESI): 169.0 $[M+H]^+$.

**Step 2: Synthesis of 7-chloro-5-nitro-2,3-dihydro-1*H*-inden-4-ol (Intermediate 33-3)**

**Intermediate 33-2** (10 g) was dissolved in AcOH (50 mL) and $H_2O$ (10 mL), and to the mixture was added $HNO_3$ (8.62 g, mass fraction of 65%) at 0°C. The reaction solution was stirred at 0°C for 2 h. After the reaction was completed, the reaction solution was slowly added to ice water (200 mL). After filtration, the filtrate was concentrated under reduced pressure to remove the solvent to obtain the title compound (10 g).

MS m/z (ESI): 214.0 $[M+H]^+$.

**Step 3: Synthesis of 5-amino-7-chloro-2,3-dihydro-1*H*-inden-4-ol (Intermediate 33-4)**

**Intermediate 33-3** (5 g) was dissolved in anhydrous DCM (50 mL), and to the mixture were added AcOH (14.04 g) and Zn (7.61 g). The reaction solution was stirred at 25°C for 12 h. After the reaction was completed, water (200 mL) and ethyl acetate (200 mL) were added successively. The organic phase was washed with a saturated aqueous sodium bicarbonate solution (50 mL*2), and the washed organic phase was dried over an appropriate amount of anhydrous sodium sulfate. The organic phase was concentrated under reduced pressure to remove the solvent to obtain the title compound (4 g).

MS m/z (ESI): 184.0 $[M+H]^+$.

**Step 4: Synthesis of 5-acetamido-7-chloro-2,3-dihydro-1*H*-inden-4-yl acetate (Intermediate 33-5)**

**Intermediate 33-4** (4 g) was dissolved in anhydrous dichloromethane (40 mL), and to the mixture were added acetic anhydride (AczO) (6.67 g) and triethylamine (TEA) (6.61 g). The reaction solution was stirred at 25°C for 12 h. After the reaction was completed, water (200 mL) and ethyl acetate (200 mL) were added successively. The organic phase was washed with a saturated aqueous NaCl solution (50 mL*2), and the washed organic phase was dried over an appropriate amount of anhydrous sodium sulfate. The organic phase was concentrated under reduced pressure to remove the solvent, and the residue was purified by silica gel column chromatography (petroleum ether:ethyl acetate =

20 : 1 to 5 : 1) to obtain the title compound (4 g).

MS m/z (ESI): 268.0 $[M+H]^+$.

**Step 5: Synthesis of *N*-(7-chloro-4-hydroxy-2,3-dihydro-1*H*-inden-5-yl)acetamide (Intermediate 33-6)**

**Intermediate 33-5** (4 g) was dissolved in absolute methanol (20 mL), and to the mixture was added $K_2CO_3$ (6.19 g). The reaction solution was stirred at 25°C for 12 h. After the reaction was completed, water (200 mL) and ethyl acetate (200 mL) were added successively. The organic phase was washed with a saturated aqueous NaCl solution (50 mL*2), and the washed organic phase was dried over an appropriate amount of anhydrous sodium sulfate. The organic phase was concentrated under reduced pressure to remove the solvent, and the residue was purified by silica gel column chromatography (petroleum ether : ethyl acetate = 20 : 1 to 1 : 1) to obtain the title compound (2.8 g).

MS m/z (ESI): 226.0 $[M+H]^+$.

**Step 6: Synthesis of 5-acetamido-7-chloro-2,3-dihydro-1*H*-inden-4-yl trifluoromethanesulfonate (Intermediate 33-7)**

**Intermediate 33-6** (500 mg) was dissolved in dichloromethane (5 mL), and to the mixture were added trifluoromethanesulfonic anhydride ($Tf_2O$) (749 mg) and triethylamine (TEA) (672 mg). The reaction solution was stirred at 25°C for 12 h. After the reaction was completed, water (50 mL) and ethyl acetate (50 mL) were added successively. The organic phase was washed with a saturated aqueous NaCl solution (50 mL*2), and the washed organic phase was dried over an appropriate amount of anhydrous sodium sulfate. The organic phase was concentrated under reduced pressure to remove the solvent, and the residue was purified by silica gel column chromatography (petroleum ether:ethyl acetate = 20 : 1 to 1 : 1) to obtain the title compound (580 mg).

MS m/z (ESI): 358.0 $[M+H]^+$.

**Step 7: Synthesis of *N*-(4-(1-butoxyvinyl)-7-chloro-2,3-dihydro-1*H*-inden-5-yl)acetamide (Intermediate 33-8)**

**Intermediate 33-7** (430 mg) and vinyl n-butyl ether (360.60 mg) were dissolved in dioxane (20 mL), and diisopropylethylamine (DIEA) (466 mg), 1,1'-bis(diphenylphosphino)ferrocene (DPPF) (66 mg) and tris(dibenzylideneacetone) dipalladium ($Pd_2(dba)_3$) (110 mg) were added. The reaction solution was stirred and reacted at 80°C under nitrogen protection for 16 h. After the reaction was completed, the reaction solution was diluted with water (50 ml), and extracted with ethyl acetate (50 ml*3). The organic phases were combined, and dried over anhydrous sodium sulfate. After filtration, the organic phase was concentrated under reduced pressure to remove the solvent, and the residue was purified by silica gel column chromatography (petroleum ether : ethyl acetate = 20 : 1 to 1 : 1) to obtain the title compound (300 mg).

MS m/z (ESI): 308.0 $[M+H]^+$.

**Step 8: Synthesis of *N*-(4-acetyl-7-chloro-2,3-dihydro-1*H*-inden-5-yl)acetamide (Intermediate 33-9)**

**Intermediate 33-8** (200 mg) was dissolved in dioxane (5 mL), and 1 N HCl (5 mL) was added. The resulting mixture was stirred and reacted at 25°C for 2 h. After the reaction was completed, the organic phase was concentrated under reduced pressure to remove the solvent to obtain the title compound (150 mg).

MS m/z (ESI): 252.0 $[M+H]^+$.

**Step 9: Synthesis of *N*-(4-(2-bromoacetyl)-7-chloro-2,3-dihydro-1*H*-inden-5-yl)acetamide (Intermediate 33-10)**

**Intermediate 33-9** (300 mg) was dissolved in HBr/AcOH (4 mL, mass fraction of 33%), and NBS (318.20 mg) was added. The reaction solution was stirred and reacted at 25°C for 2 h. After the reaction was completed, the reaction solution was concentrated under reduced pressure to remove the solvent to obtain the title compound (390 mg).

MS m/z (ESI): 330.0 $[M+H]^+$.

**Step 10: Synthesis of 1-(5-amino-7-chloro-2,3-dihydro-1*H*-inden-4-yl)-2-chloroethan-1-one (Intermediate 33-11)**

**Intermediate 33-10** (300 mg) was dissolved in absolute alcohol, and HCl (12 M, 8.00 mL) was added. The reaction solution was stirred and reacted at 80°C for 2 h. After the reaction was completed, the reaction solution was concentrated under reduced pressure to remove the solvent, and the residue was purified by preparative high performance liquid chromatography (chromatographic column: Gemini NX C18 5 μm*10*150 mm; mobile phase: A: water (0.225% formic acid v/v), B: acetonitrile; B%: 30%-70%) to obtain the title compound (64 mg).

MS m/z (ESI): 244.0 [M+H]+.

**Step 11: Synthesis of (*S*)-4-chloro-15-(chloromethyl)-8-ethyl-1,2,3,8,11,14-hexahydro-9*H*,12*H*-cyclopentadieno[*f*]pyrano[3',4':6,7]indolizino[1,2-*b*]quinoline-9,12-dione (Intermediate 33-12)**

**Intermediate 33-11** (45.00 mg) and **Intermediate 1-3** (48.53 mg) were dissolved in toluene (1 mL), and to the mixture was added pyridinium p-toluenesulfonate (4.63 mg). The reaction solution was stirred at 90°C for 16 h. After the reaction was completed, the reaction solution was cooled to room temperature, and ethanol (1 mL) was added. The reaction solution was stirred at 25°C for 0.5 h. The reaction solution was filtered. The filter cake was washed with ethanol (2 mL*2), and drying was performed to obtain the title compound (80.0 mg).
MS m/z (ESI): 471.1 [M+H]+.

**Step 12: Synthesis of (*S*)-15-(aminomethyl)-4-chloro-8-ethyl-8-hydroxy-1,2,3,8,11,14-hexahydro-9*H*,12*H*-cyclopentadieno[*f*]pyrano[3',4':6,7]indolizino [1,2-*b*]quinoline-9,12-dione (Intermediate 33-13)**

**Intermediate 33-12** (40.00 mg) was dissolved in absolute methanol (1 mL) and anhydrous *N,N*-dimethylformamide (0.5 mL), and to the mixture was added hexamethylenetetramine (35.69 mg). The reaction solution was stirred at 50°C for 16 h. After the reaction was completed, the reaction solution was cooled to room temperature, and concentrated to dryness under reduced pressure. The residue was purified by preparative high performance liquid chromatography (Boston Prime C18 column, 5 μm silica, 30 mm in diameter, 150 mm in length; using a decreasingly polar mixture of water (containing 0.225% FA) and acetonitrile as the eluent; acetonitrile gradient proportion: 35%-55%, elution time: 12 min) to obtain the title compound (15.0 mg).
MS m/z (ESI): 452.1 [M+H]+.

**Step 13: Synthesis of (*S*)-*N*-((4-chloro-8-ethyl-8-hydroxy-9,12-dioxo-2,3,8,9,12,14-hexahydro-1*H*,11*H*-cyclopentadieno[*f*]pyrano[3',4':6,7]indolizino [1,2-*b*]quinolin-15-yl)methyl)-2-hydroxyacetamide (Compound 33)**

**Intermediate 33-13** (5.00 mg) and hydroxyacetic acid (4.21 mg, 55.30 μmol) were dissolved in anhydrous *N,N*-dimethylformamide (0.5 mL), and to the mixture were added HATU (6.31 mg) and *N,N*-diisopropylethylamine (4.29 mg). The reaction solution was stirred at 25°C for 1 h. After the reaction was completed, the reaction solution was filtered, and purified by preparative high performance liquid chromatography (Boston Green ODS C18 column, 5 μm silica, 30 mm in diameter, 150 mm in length; using a decreasingly polar mixture of water (containing 0.05% FA) and acetonitrile as the eluent; acetonitrile gradient proportion: 32%-52%, elution time: 12 min) to obtain the title compound (2.0 mg).
MS m/z (ESI): 510.3 [M+H]+.
**$^1$H NMR (400MHz, DMSO-*d*$_6$)** δ = 8.36-8.30 (m, 1H), 8.14 (s, 1H), 7.31 (s, 1H), 6.55 (s, 1H), 5.44 (s, 2H), 5.38 (s, 2H), 4.96 (d, *J* = 5.3 Hz, 2H), 3.88 (s, 2H), 3.74-3.66 (m, 2H), 3.14 (t, *J* = 7.6 Hz, 2H), 2.27-2.19 (m, 2H), 1.92-1.82 (m, 2H), 0.88 (t, *J* = 7.3 Hz, 3H).

**Example 34. *N*-(((*S*)-4-chloro-8-ethyl-8-hydroxy-9,12-dioxo-2,3,8,9,12,14-hexahydro-1*H*,11*H*-cyclopentadieno[*f*]pyrano[3',4':6,7]indolizino[1,2-*b*]quinolin-15-yl)methyl)-2-cyclopropyl-2-hydroxyacetamide (Compound 34)**

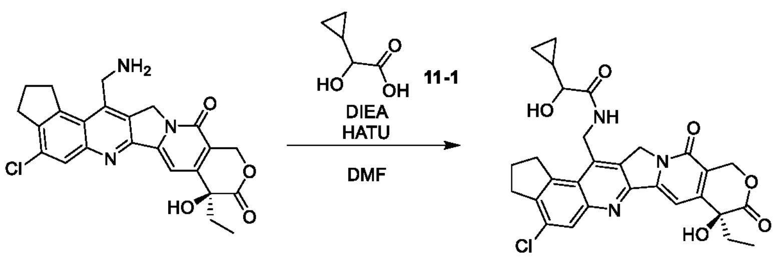


33-13

Compound 34

**Intermediate 33-13** (5.00 mg) and **Intermediate 11-1** (6.42 mg) were dissolved in anhydrous *N,N*-dimethylformamide (0.5 mL), and to the mixture were added HATU (6.31 mg) and diisopropylethylamine (4.29 mg). The reaction solution was stirred at 25°C for 1 h. After the reaction was completed, the reaction solution was filtered, and purified by preparative high performance liquid chromatography (Boston Prime C18 column, 5 μm silica, 30 mm in diameter, 150

mm in length; using a decreasingly polar mixture of water (containing 0.05% FA) and acetonitrile as the eluent; acetonitrile gradient proportion: 35%-55%, elution time: 12 min) to obtain the title compound (2.0 mg).

MS m/z (ESI): 550.3 $[M+H]^+$.

**$^1$H NMR (400MHz, DMSO-$d_6$)** δ = 8.36-8.31 (m, 1H), 8.14 (s, 1H), 7.31 (s, 1H), 6.54 (s, 1H), 5.47-5.41 (m, 3H), 5.38 (s, 2H), 4.96-4.90 (m, 2H), 3.69 (t, *J* = 7.1 Hz, 2H), 3.56 (t, *J* = 5.8 Hz, 1H), 3.14 (t, *J* = 7.4 Hz, 2H), 2.27-2.18 (m, 2H), 1.93-1.81 (m, 2H), 1.09-1.03 (m, 1H), 0.88 (t, *J* = 7.2 Hz, 3H), 0.41-0.26 (m, 4H).

**Example 35. (*S*)-14-(aminomethyl)-7-ethyl-15-fluoro-7-hydroxy-10,13-dihydro-11*H*-[1,3]dioxolo[4,5-*g*] pyrano[3',4':6,7]indolizino[1,2-*b*]quinoline-8,11(7*H*)-dione (Compound 35)**

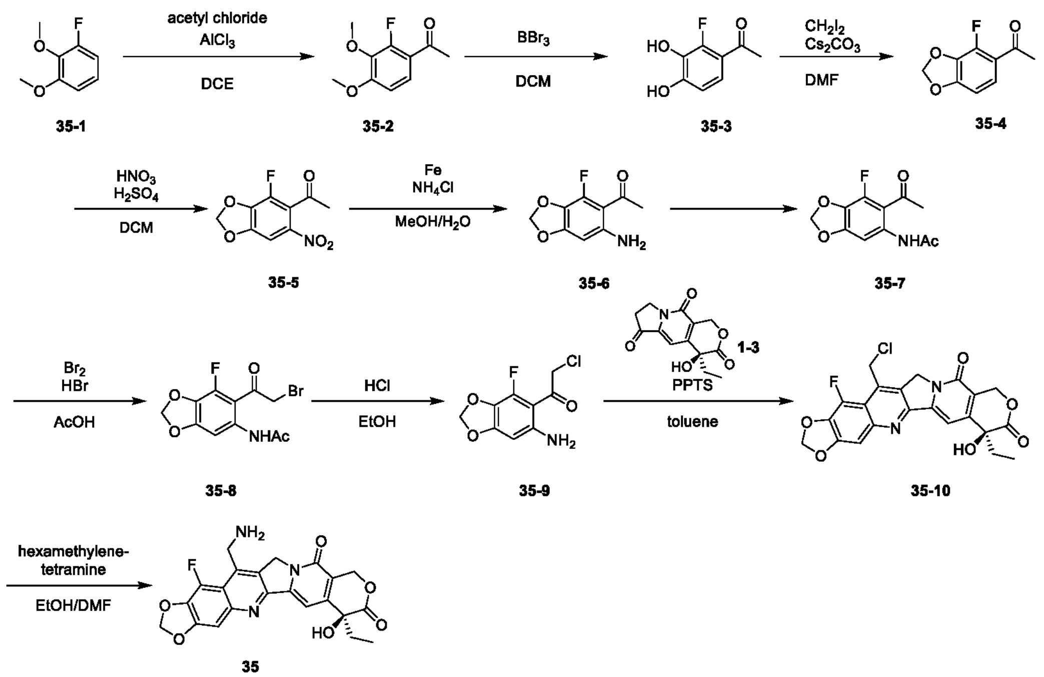


**Step 1: Synthesis of 1-(2-fluoro-3,4-dimethoxyphenyl)ethan-1-one (Intermediate 35-2)**

**Intermediate 35-1** (25.0 g) was dissolved in 1,2-dichloromethane (250 mL). The reaction solution was cooled to 0°C, and to which was slowly added aluminum trichloride (64.04 g). Then acetyl chloride (64.04 g) was added dropwise to the reaction solution. The reaction solution was stirred at 0°C under nitrogen atmosphere for 2 h. After the reaction was completed, to the reaction solution was added water (300 mL), and the mixture was extracted with ethyl acetate (150 mL*3). The organic phases were combined, and dried over anhydrous sodium sulfate. After filtration, the organic phase was concentrated under reduced pressure. The residue was purified by silica gel column chromatography (ISCO®; 120 g SepaFlash® flash silica gel column, gradient: 0% to 50% petroleum ether/ethyl acetate, flow rate: 70 mL/min) to obtain the title compound (21.0 g).

MS m/z (ESI): 199.0 $[M+H]^+$.

**Step 2: Synthesis of 1-(2-fluoro-3,4-dihydroxyphenyl)ethan-1-one (Intermediate 35-3)**

**Intermediate 35-2** (15.00 g) was dissolved in anhydrous dichloromethane (150 mL). The reaction solution was cooled to -78°C, and to which was added slowly dropwise boron tribromide (56.88 g). The reaction solution was stirred at -78°C under nitrogen atmosphere for 2 h, and then warmed to 0°C and reacted for 4 h. After the reaction was completed, the reaction solution was added slowly dropwise to ice water for quenching. After quenching was completed, the reaction solution was extracted with ethyl acetate (150 mL*3). The organic phases were combined, and dried over anhydrous sodium sulfate. After filtration, the organic phase was concentrated under reduced pressure. The residue was purified by silica gel column chromatography (ISCO®; 120 g SepaFlash® flash silica gel column, gradient: 0% to 50% petroleum ether/ethyl acetate, flow rate: 70 mL/min) to obtain the title compound (8.50 g).

MS m/z (ESI): 171.0 $[M+H]^+$.

**Step 3: Synthesis of 1-(4-fluorobenzo[*d*][1,3]dioxol-5-yl)ethan-1-one (Intermediate 35-4)**

**Intermediate 35-3** (4.0 g) was dissolved in anhydrous *N,N*-dimethylformamide (40 mL), and to the mixture were added cesium carbonate (11.49 g) and 1,2-diiodomethane (18.89 g). The reaction solution was stirred at 100°C under nitrogen atmosphere for 8 min. After the reaction was completed, the reaction solution was slowly poured into water, and extracted with ethyl acetate (50 mL*3). The organic phases were combined, and dried over anhydrous sodium sulfate. After filtration, the organic phase was concentrated under reduced pressure. The residue was purified by silica gel column chromatography (ISCO®; 24 g SepaFlash® flash silica gel column, gradient: 0% to 15% petroleum ether/ethyl acetate, flow rate: 60 mL/min) to obtain the title compound (2.0 g).

MS m/z (ESI): 183.0 $[M+H]^+$.

**Step 4: Synthesis of 1-(4-fluoro-6-nitrobenzo[*d*][1,3]dioxol-5-yl)ethan-1-one (Intermediate 35-5)**

**Intermediate 35-4** (2.0 g) was dissolved in anhydrous dichloromethane (15 mL), and to the mixture was added concentrated sulfuric acid (5.38 g, mass fraction of 98%), and the reaction solution was cooled to 0°C. Then, concentrated nitric acid (3.46 g, mass fraction of 68%) was added slowly dropwise to the reaction solution. The reaction solution was stirred at 25°C for 2 h. After the reaction was completed, the reaction solution was added slowly dropwise to ice water (50 mL), and then ethyl acetate (50 mL) was added. The organic phase was washed with water (50 mL*2), and the washed organic phase was dried over an appropriate amount of anhydrous sodium sulfate. The organic phase was concentrated to dryness under reduced pressure. The residue was purified by silica gel column chromatography (ISCO®; 24 g SepaFlash® flash silica gel column, gradient: 0% to 40% petroleum ether/ethyl acetate, flow rate: 60 mL/min) to obtain the title compound (1.8 g).

**$^1$H NMR (400MΣlz, Deuterated chloroform)** δ = 7.51 (s, 1H), 6.26 (s, 2H), 2.63 (s, 3H).

**Step 5: Synthesis of 1-(6-amino-4-fluorobenzo[*d*][1,3]dioxol-5-yl)ethan-1-one (Intermediate 35-6)**

**Intermediate 35-5** (1.8 g) was dissolved in absolute methanol (18 mL) and water (9 mL), and to the mixture was added ammonium chloride (635.83 mg) and iron powder (2.21 mg). The reaction solution was stirred at 80°C under nitrogen atmosphere for 2 h. After the reaction was completed, the reaction solution was cooled to room temperature. The reaction solution was filtered. The filtrate was diluted with ethyl acetate (50 mL). The organic phase was washed with water (50 mL*2), and the washed organic phase was dried over an appropriate amount of anhydrous sodium sulfate. The organic phase was concentrated to dryness under reduced pressure to obtain the title compound (1.5 g).

MS m/z (ESI): 198.0 $[M+H]^+$.

**Step 6: Synthesis of *N*-(6-acetyl-7-fluorobenzo[*d*][1,3]dioxol-5-yl)acetamide (Intermediate 35-7)**

**Intermediate 35-6** (500.0 mg) was dissolved in anhydrous dichloromethane (5 mL), and to the mixture was added pyridine (601.79 mg). Acetyl chloride (398.14 mg) was added dropwise to the reaction solution under nitrogen atmosphere. The reaction solution was stirred at 25°C under nitrogen atmosphere for 1.5 h. After the reaction was completed, the organic phase was concentrated to dryness under reduced pressure. The residue was purified by silica gel column chromatography (ISCO®; 12 g SepaFlash® flash silica gel column, gradient: 0% to 40% petroleum ether/ethyl acetate, flow rate: 60 mL/min) to obtain the title compound (380.0 mg).

**$^1$H NMR (400MΣlz, Deuterated chloroform)** δ = 11 72 (s, 1H), 8.18 (d, *J* = 1.0 Hz, 1H), 6.10 (s, 2H), 2.64 (d, *J* = 8.4 Hz, 3H), 2.22 (s, 3H).

**Step 7: Synthesis of *N*-(6-(2-bromoacetyl)-7-fluorobenzo[*d*][1,3]dioxol-5-yl) acetamide (Intermediate 35-8)**

**Intermediate 35-7** (380.0 mg) was dissolved in acetic acid (3 mL), to the mixture was added a solution of hydrogen bromide in acetic acid (1.95 g, 33% content), and then to the reaction solution was added slowly dropwise liquid bromine (256.42 mg). The reaction solution was stirred at 25°C for 1 h. After the reaction was completed, the reaction solution was slowly poured into ice water and stirred for 0.5 h, and filtered. The filter cake was washed with water (20 mL*2), and dried to obtain the title compound (400.0 mg).

MS m/z (ESI): 317.8 $[M+H]^+$.

**Step 8: Synthesis of 1-(6-amino-4-fluorobenzo[*d*][1,3]dioxol-5-yl)-2-chloroethan-1-one (Intermediate 35-9)**

**Intermediate 35-8** (400.0 mg) was dissolved in absolute alcohol (2 mL) and concentrated hydrochloric acid (2 mL), and the reaction solution was stirred at 60°C for 3 h. After the reaction was completed, ice water (20 mL) was added

successively and slowly, saturated sodium bicarbonate was used to adjust pH to 8. Then ethyl acetate (40 mL) was added. The organic phase was washed with water (20 mL*2), and the washed organic phase was dried over an appropriate amount of anhydrous sodium sulfate. The organic phase was concentrated to dryness under reduced pressure to obtain the title compound (220.0 mg).

MS m/z (ESI): 232.0 $[M+H]^+$.

**Step 9: Synthesis of (*S*)-14-(chloromethyl)-7-ethyl-15-fluoro-7-hydroxy-10,13-dihydro-11*H*-[1,3]dioxolo[4,5-*g*] pyrano[3',4':6,7]indolizino[1,2-*b*]quinoline-8,11(7*H*)-dione (Compound 35-10)**

**Intermediate 35-9** (200.0 mg) and **Intermediate 1-3** (227.32 mg) were dissolved in toluene (3 mL), and to the mixture was added pyridinium p-toluenesulfonate (21.70 mg). The reaction solution was stirred at 90°C for 16 h. After the reaction was completed, ethanol (1 mL) was added after the reaction solution was cooled to room temperature. The reaction solution was stirred at 25°C for 0.5 h. The reaction solution was filtered. The filter cake was washed with ethanol (5 mL*2) to obtain the title compound (320.0 mg).

MS m/z (ESI): 459.0 $[M+H]^+$.

**Step 10: Synthesis of (*S*)-14-(aminomethyl)-7-ethyl-15-fluoro-7-hydroxy-10,13-dihydro-11*H*-[1,3]dioxolo[4,5-*g*] pyrano[3',4':6,7]indolizino[1,2-*b*]quinoline-8,11(7*H*)-dione (Compound 35)**

**Intermediate 35-10** (260.00 mg) was dissolved in absolute methanol (1 mL) and anhydrous *N,N*-dimethylformamide (1 mL), and to the mixture was added hexamethylenetetramine (238.32 mg). The reaction solution was stirred at 50°C for 3 h. After the reaction was completed, the reaction solution was concentrated to dryness under reduced pressure after the reaction solution was cooled to room temperature, and purified by preparative high performance liquid chromatography (Boston Prime C18 column, 5 μm silica, 30 mm in diameter, 150 mm in length; using a decreasingly polar mixture of water (containing 0.225% formic acid) and acetonitrile as the eluent; acetonitrile gradient proportion: 0%-30%, elution time: 14 min) to obtain the title compound (85.0 mg).

MS m/z (ESI): 440.0 $[M+H]^+$.

**$^1$H NMR (400MHz, DMSO-$d_6$)** δ = 7.49 (s, 1H), 7.26 (s, 1H), 6.53 (s, 1H), 6.38 (s, 2H), 5.44 (s, 4H), 4.27 (s, 2H), 1.94-1.79 (m, 2H), 0.88 (t, *J* = 7.3 Hz, 3H).

**Example 36. (*S*)-15-(aminomethyl)-8-ethyl-16-fluoro-8-hydroxy-2,3,11,14-tetrahydro-12*H*-[1,4]dioxino[2,3-*g*] pyrano[3',4':6,7]indolizino[1,2-*b*]quinoline-9,12(8*H*)-dione (Compound 36)**

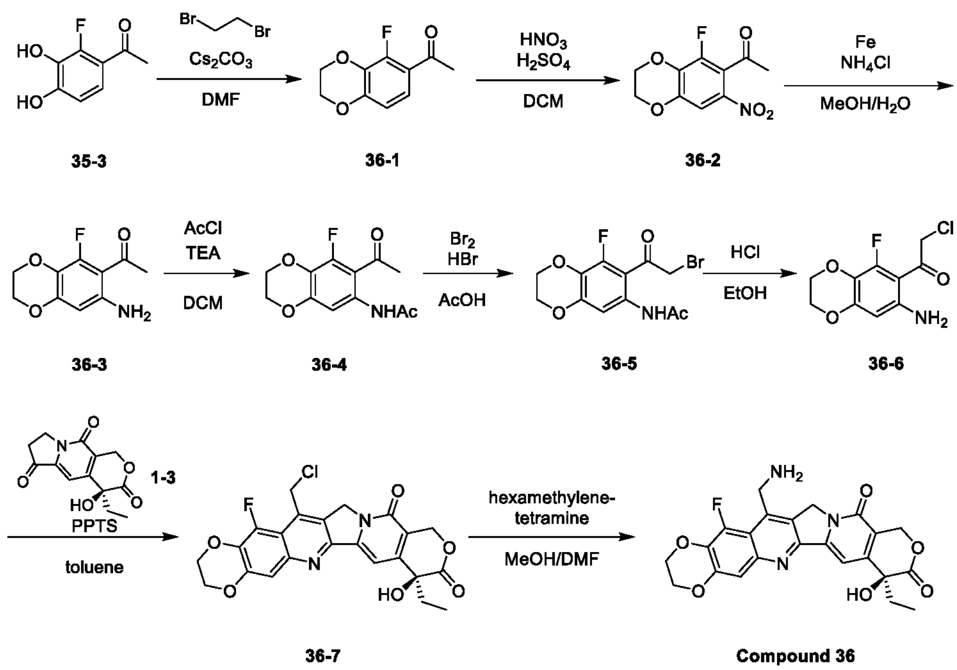


35-3 36-1 36-2

36-3 36-4 36-5 36-6

36-7 Compound 36

**Step 1: Synthesis of 1-(5-fluoro-2,3-dihydrobenzo[*b*][1,4]dioxine-6-yl)ethan-1-one (Intermediate 36-1)**

**Intermediate 35-3** (0.5 g) and 1,2-dibromoethane were dissolved in anhydrous *N,N*-dimethylformamide (5 mL), and to the mixture was added potassium carbonate (1.44 g). The reaction solution was stirred at 100°C for 10 min. After the reaction was completed, the reaction solution was slowly poured into ice water, and then ethyl acetate (50 mL) was added. The organic phase was washed with water (30 mL*2), and the washed organic phase was dried over an appropriate amount of anhydrous sodium sulfate. The organic phase was concentrated under reduced pressure, and the residue was purified by silica gel column chromatography (ISCO®; 12 g SepaFlash® flash silica gel column, gradient 0% to 40% petroleum ether/ethyl acetate, flow rate 60 mL/min) to obtain the title compound (0.2 g).
MS m/z (ESI): 197.0 $[M+H]^+$.

**Step 2: Synthesis of 1-(5-fluoro-7-nitro-2,3-dihydrobenzo[*b*][1,4]dioxine-6-yl)ethan-1-one (Intermediate 36-2)**

**Intermediate 36-1** (0.7 g) was dissolved in anhydrous dichloromethane (6 mL), and to the mixture was added concentrated sulfuric acid (1.75 g, mass fraction of 98%), and the reaction solution was cooled to 0°C. Then, concentrated nitric acid (1.12 g, mass fraction of 68%) was added slowly dropwise to the reaction solution. The reaction solution was stirred at 25°C for 2 h. After the reaction was completed, the reaction solution was added slowly dropwise to ice water (20 mL), and then ethyl acetate (50 mL) was added. The organic phase was washed with water (30 mL*2), and the washed organic phase was dried over an appropriate amount of anhydrous sodium sulfate. The organic phase was concentrated to dryness under reduced pressure. The residue was purified by silica gel column chromatography (ISCO®; 12 g SepaFlash® flash silica gel column, gradient: 0% to 50% petroleum ether/ethyl acetate, flow rate: 60 mL/min) to obtain the title compound (0.3 g).
**$^1$H NMR (400MΣIz, Deuterated chloroform)** δ = 7.61 (d, *J* = 1.9 Hz, 1H), 4.48-4.44 (m, 2H), 4.43-4.38 (m, 2H), 2.63 (s, 3H).

**Step 3: Synthesis of 1-(7-amino-5-fluoro-2,3-dihydrobenzo[*b*][1,4]dioxine-6-yl)ethan-1-one (Intermediate 36-3)**

**Intermediate 36-2** (300.0 mg) was dissolved in absolute methanol (3 mL) and water (0.5 mL), and to the mixture was added ammonium chloride (79.85 mg) and iron powder (347.33 mg). The reaction solution was stirred at 80°C under nitrogen atmosphere for 1.5 h. After the reaction was completed, the reaction solution was cooled to room temperature. The reaction solution was filtered. The filtrate was diluted with ethyl acetate (50 mL). The organic phase was washed with water (50 mL*2), and the washed organic phase was dried over an appropriate amount of anhydrous sodium sulfate. The organic phase was concentrated to dryness under reduced pressure to obtain the title compound (250.0 mg).
MS m/z (ESI): 212.0 $[M+H]^+$.

**Step 4: Synthesis of *N*-(7-acetyl-8-fluoro-2,3-dihydrobenzo[*b*][1,4]dioxin-6-yl)acetamide (Intermediate 36-4)**

**Intermediate 36-3** (250.0 mg) was dissolved in anhydrous dichloromethane (5 mL), and to the mixture was added triethylamine (598.93 mg). Acetyl chloride (278.77 mg) was added dropwise to the reaction solution under nitrogen atmosphere. The reaction solution was stirred at 25°C under nitrogen atmosphere for 0.5 h. After the reaction was completed, the organic phase was concentrated to dryness under reduced pressure. The residue was purified by silica gel column chromatography (ISCO®; 12 g SepaFlash® flash silica gel column, gradient: 0% to 70% petroleum ether/ethyl acetate, flow rate: 60 mL/min) to obtain the title compound (0.1 g).
MS m/z (ESI): 254.0 $[M+H]^+$.

**Step 5: Synthesis of *N*-(7-(2-bromoacetyl)-8-fluoro-2,3-dihydrobenzo [*b*][1,4]dioxin-6-yl)acetamide (Intermediate 36-5)**

**Intermediate 36-4** (100.0 mg) was dissolved in acetic acid (2 mL), to the mixture was added a solution of hydrogen bromide in acetic acid (290.48 mg, 33% content), and then to the reaction solution was added slowly dropwise liquid bromine (75.73 mg). The reaction solution was stirred at 25°C for 0.5 h. After the reaction was completed, the reaction solution was slowly poured into ice water and stirred for 0.5 h, and filtered. The filter cake was dried to obtain the title compound (80.0 mg).
**$^1$H NMR (400MΣIz, Deuterated chloroform)** δ = 11.04 (s, 1H), 8.15 (d, *J* = 2.1 Hz, 1H), 4.56 (d, *J* = 4.3 Hz, 2H), 4.43-4.38 (m, 2H), 4.37-4.32 (m, 2H), 2.22 (s, 3H).

**Step 6: Synthesis of 1-(7-amino-5-fluoro-2,3-dihydrobenzo[*b*][1,4]dioxin-6-yl)-2-chloroethan-1-one (Intermediate 36-6)**

**Intermediate 36-5** (80.0 mg) was dissolved in absolute alcohol (0.5 mL) and concentrated hydrochloric acid (0.5 mL), and the reaction solution was stirred at 60°C for 2 h. After the reaction was completed, ice water (10 mL) and saturated sodium bicarbonate (10 mL) were added successively and slowly after the reaction solution was cooled to room temperature. Then dichloromethane (30 mL) was added. The organic phase was washed with water (20 mL*2), and the washed organic phase was dried over an appropriate amount of anhydrous sodium sulfate. The organic phase was concentrated to dryness under reduced pressure to obtain the title compound (60.0 mg).

MS m/z (ESI): 246.0 $[M+H]^+$.

**Step 7: Synthesis of (*S*)-15-(chloromethyl)-8-ethyl-16-fluoro-8-hydroxy-2,3,11,14-tetrahydro-12*H*-[1,4]dioxolo[2,3-*g*]pyrano[3',4':6,7]indolizino[1,2-*b*]quinoline-9,12(8*H*)-dione (Intermediate 36-7)**

**Intermediate 36-6** (60.0 mg) and **Intermediate 1-3** (64.3 mg) were dissolved in toluene (3 mL), and to the mixture was added pyridinium p-toluenesulfonate (12.28 mg). The reaction solution was stirred at 95°C for 16 h. After the reaction was completed, ethanol (1 mL) was added after the reaction solution was cooled to room temperature. The reaction solution was stirred at 25°C for 0.5 h. The reaction solution was filtered. The filter cake was washed with ethanol (2 mL*2) to obtain the title compound (60.0 mg).

MS m/z (ESI): 473.0 $[M+H]^+$.

**Step 8: Synthesis of (*S*)-15-(aminomethyl)-8-ethyl-16-fluoro-8-hydroxy-2,3,11,14-tetrahydro-12*H*-[1,4]dioxolo[2,3-g]pyrano[3',4':6,7]indolizino[1,2-*b*]quinoline-9,12(8*H*)-dione (Compound 36)**

**Intermediate 36-7** (60.00 mg) was dissolved in absolute methanol (1 mL) and anhydrous *N,N*-dimethylformamide (0.5 mL), and to the mixture was added hexamethylenetetramine (53.36 mg). The reaction solution was stirred at 50°C for 3 h. After the reaction was completed, the reaction solution was concentrated to dryness under reduced pressure after the reaction solution was cooled to room temperature. The residue was purified by preparative high performance liquid chromatography (Boston Prime C18 column, 5 μm silica, 30 mm in diameter, 150 mm in length; using a decreasingly polar mixture of water (containing 0.225% formic acid) and acetonitrile as the eluent; acetonitrile gradient proportion: 0%-25%, elution time: 12 min) to obtain the title compound (24.0 mg).

MS m/z (ESI): 454.1 $[M+H]^+$.

**$^1$H NMR (400MHz, DMSO-$d_6$)** 7.60 (s, 1H), 7.31 (s, 1H), 6.55 (s, 1H), 5.49 (s, 2H), 5.45 (s, 2H), 4.60 (s, 2H), 4.54 (s, 4H), 1.92-1.84 (m, 2H), 0.88 (t, *J* = 7.3 Hz, 3H).

**Example 37. 2-cyclopropyl-*N*-(((*S*)-7-ethyl-15-fluoro-7-hydroxy-8,11-dioxo-7,8,11,13-tetrahydro-10*H*-[1,3]dioxolo[4,5-g]pyrano[3',4':6,7]indolizino[1,2-*b*]quinolin-14-yl)methyl)-2-hydroxyacetamide (Compound 37)**

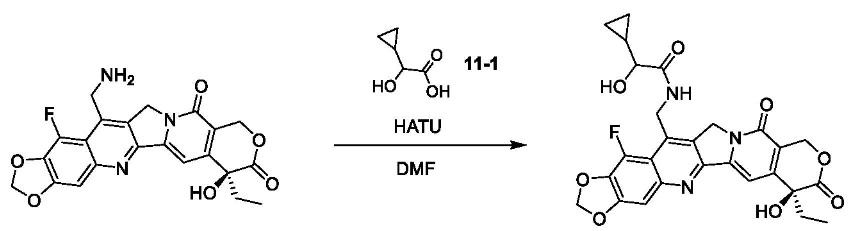

Compound 35

Compound 37

**Compound 35** (6 mg) and **Intermediate 11-1** (7.93 mg) were dissolved in *N,N*-dimethylformamide (0.5 mL), and to the mixture were added 2-(7-azabenzotriazol-1-yl)-*N,N,N',N'*-tetramethyluronium hexafluorophosphate (7.79 mg) and *N,N*-dimethyldiisopropylamine (5.29 mg). The reaction solution was stirred at 25°C for 1 h. After the reaction was completed, the reaction solution was concentrated to dryness under reduced pressure. The residue was purified by preparative high performance liquid chromatography (Boston Prime C18 column, 5 μm silica, 30 mm in diameter, 150 mm in length; using a decreasingly polar mixture of water (containing 0.225% formic acid) and acetonitrile as the eluent; acetonitrile gradient proportion: 15%-45%, elution time: 12 min) to obtain the title compound (6.50 mg).

MS m/z (ESI): 538.1 $[M+H]^+$.

**$^1$H NMR (400MHz, DMSO-$d_6$)** $\delta$ = 8.21 (t, *J* = 6.0 Hz, 1H), 7.51 (s, 1H), 7.26 (s, 1H), 6.53 (s, 1H), 6.40 (s, 2H), 5.47 (s, 2H), 5.43 (s, 2H), 4.84 (d, *J* = 3.8 Hz, 2H), 3.53 (d, *J* = 6.3 Hz, 1H), 1.90-1.81 (m, 2H), 1.01 (d, *J* = 5.5 Hz, 1H), 0.89-0.85 (m, 3H), 0.33 (d, *J* = 6.0 Hz, 2H), 0.30-0.25 (m, 2H).

**Synthesis of Compound 37-P1 and Compound 37-P2**

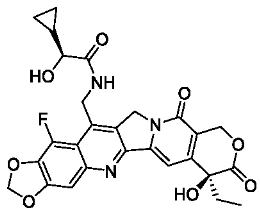

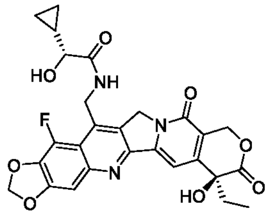

**Compound 35** (6 mg) and **Intermediate 14-10-P1** (7.93 mg) were dissolved in *N,N*-dimethylformamide (1 mL), and to the mixture were added 2-(7-azabenzotriazol-1-yl)-*N,N,N',N'*-tetramethyluronium hexafluorophosphate (7.79 mg) and *N,N*-dimethyldiisopropylamine (5.29 mg). The reaction solution was stirred at 25°C for 1 h. After the reaction was completed, the reaction solution was concentrated to dryness under reduced pressure. The residue was purified by preparative high performance liquid chromatography (Boston Green ODS C18 column, 5 μm silica, 30 mm in diameter, 150 mm in length; using a decreasingly polar mixture of water (containing 0.225% formic acid) and acetonitrile as the eluent; acetonitrile gradient proportion: 17%-47%, elution time: 12 min) to obtain Compound 37-P1 (2.87 mg).

MS m/z (ESI): 538.1 $[M+H]^+$.

**$^1$H NMR (400MHz, DMSO-$d_6$)** $\delta$ = 8.19 (t, *J* = 5.9 Hz, 1H), 7.51 (s, 1H), 7.26 (s, 1H), 6.51 (s, 1H), 6.40 (s, 2H), 5.47 (s, 2H), 5.43 (s, 2H), 4.84 (d, *J* = 4.6 Hz, 2H), 3.53 (d, *J* = 6.4 Hz, 1H), 1.91-1.80 (m, 2H), 1.06-0.97 (m, 1H), 0.87 (t, *J* = 7.3 Hz, 3H), 0.38-0.31 (m, 2H), 0.31-0.24 (m, 2H).

**Compound 35** (6 mg) and **Intermediate 14-10-P2** (4.76 mg) were dissolved in *N,N*-dimethylformamide (1 mL), and to the mixture were added 2-(7-azabenzotriazol-1-yl)-*N,N,N',N'*-tetramethyluronium hexafluorophosphate (10.38 mg) and *N,N*-dimethyldiisopropylamine (1.76 mg). The reaction solution was stirred at 25°C for 1 h. After the reaction was completed, the reaction solution was concentrated to dryness under reduced pressure. The residue was purified by preparative high performance liquid chromatography (Boston Green ODS C18 column, 5 μm silica, 30 mm in diameter, 150 mm in length; using a decreasingly polar mixture of water (containing 0.225% formic acid) and acetonitrile as the eluent; acetonitrile gradient proportion: 17%-47%, elution time: 12 min) to obtain Compound 37-P2 (2.01 mg).

MS m/z (ESI): 538.1 $[M+H]^+$.

**$^1$H NMR (400MHz, DMSO-$d_6$)** $\delta$ = 8.26-8.16 (m, 1H), 7.51 (s, 1H), 7.26 (s, 1H), 6.51 (s, 1H), 6.40 (s, 2H), 5.46 (s, 2H), 5.43 (s, 2H), 4.87-4.82 (m, 2H), 3.53 (d, *J* = 6.2 Hz, 1H), 1.94-1.80 (m, 2H), 1.05-0.96 (m, 1H), 0.87 (t, *J* = 7.3 Hz, 3H), 0.39-0.30 (m, 2H), 0.31-0.20 (m, 2H).

The two isomers were further analyzed separately by the following chiral supercritical fluid chromatography.

| Column | Chiralcel OJ-3 100A 4.6 mm I.D., 3 μm |
|---|---|
| Mobile phase | A: Carbon dioxide |
| | B: Ethanol (0.05% diethylamine) |
| Flow rate | 2.8 mL/min |
| Wavelength | PDA 220 nm |
| Column temperature | 35°C |
| Column pressure | 1500 Psi |
| Model of instrument | Waters UPCC with PDA Detector |

**Compound 37-P1:**
Under the above chiral supercritical fluid chromatography conditions, the retention time was 3.519 min;
**Compound 37-P2:**
Under the above chiral supercritical fluid chromatography conditions, the retention time was 3.573 min.

**Example 38. (*S*)-*N*-((7-ethyl-15-fluoro-7-hydroxy-8,11-dioxo-7,8,11,13-tetrahydro-10*H*-[1,3]dioxolo[4,5-*g*] pyrano[3',4':6,7]indolizino[1,2-*b*]quinolin-14-yl)methyl)-2-hydroxyacetamide (Compound 38)**

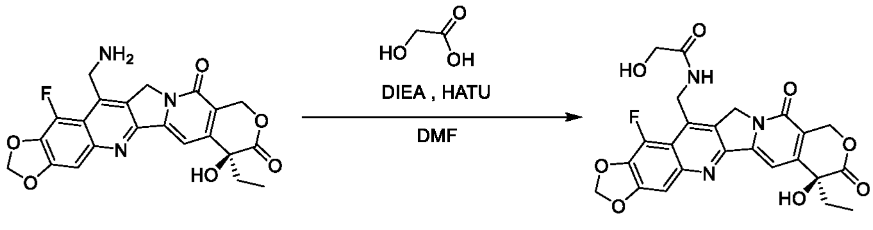


Compound 35 Compound 38

**Compound 35** (6 mg) and hydroxyacetic acid (3.21 mg) were dissolved in anhydrous *N,N*-dimethylformamide (0.5 mL), and to the mixture were added 2-(7-azabenzotriazol-1-yl)-*N,N,N',N'*-tetramethyluronium hexafluorophosphate (10.38 mg) and diisopropylethylamine (1.76 mg). The reaction solution was stirred at 25°C for 1 h. After the reaction was completed, the reaction solution was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 18%-48%, elution time: 12 min) to obtain the title compound (2.40 mg).

MS m/z (ESI): 498.1[M+H]$^+$.

**$^1$H NMR (400MHz, DMSO-*d*6)** δ = 8.20 (t, *J* = 5.9 Hz, 1H), 7.51 (s, 1H), 7.26 (s, 1H), 6.39 (s, 2H), 5.46 (s, 2H), 5.43 (s, 2H), 4.85 (d, *J* = 4.3 Hz, 2H), 3.83 (s, 2H), 1.92-1.80 (m, 2H), 0.87 (t, *J* = 7.3 Hz, 3H).

**Example 39. 2-cyclopropyl-*N*-(((*S*)-7-ethyl-7-hydroxy-8,11-dioxo-7,8,11,13-tetrahydro-10*H*-[1,3]dioxolo[4,5-*g*] pyrano[3',4':6,7]indolizino[1,2-*b*]quinolin-14-yl-2,2-*d*$_2$)methyl)-2-hydroxyacetamide (Compound 39, Compound 39-P1/P2)**

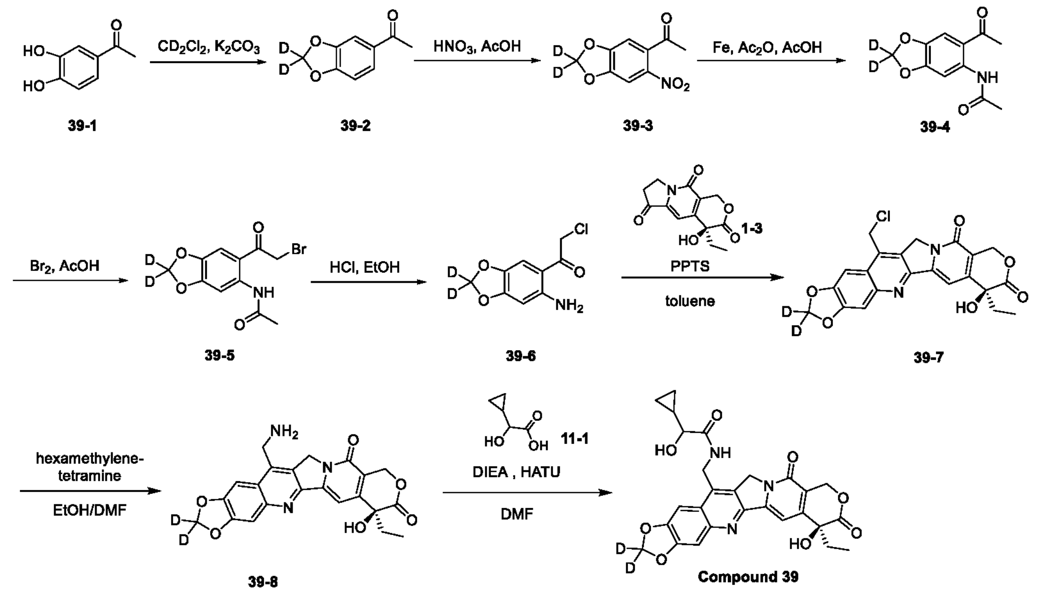


**Step 1: Synthesis of 1-(benzo[*d*][1,3]dioxol-5-yl-2,2-*d*$_2$)ethan-1-one (Intermediate 39-2)**

**Intermediate 39-1** (3 g) was dissolved in an anhydrous DMF solution (25 mL), and deuterodichloromethane (8.57 g) and potassium carbonate (8.18 g) were added. After the addition, the resulting mixture was warmed to 90°C and stirred for 16 h. Then the reaction solution was added to water (100 mL), and the resulting mixture was extracted with ethyl acetate (200 mL*2). The organic phases were combined and washed with saturated brine (100 mL), and dried

over anhydrous sodium sulfate. After filtration, the filtrate was concentrated to dryness under reduced pressure, and the residue was purified by column chromatography (ethyl acetate/petroleum ether = 5 : 1) to obtain the title compound (2.4 g).
MS m/z (ESI): 167.1[M+H]+.

**Step 2: Synthesis of 1-(6-nitrobenzo[*d*][1,3]dioxol-5-yl-2,2-$d_2$)ethan-1-one (Intermediate 39-3)**

**Intermediate 39-2** (2.4 g) was dissolved in anhydrous acetic acid (10 mL), and concentrated nitric acid (32.50 g, 70% content) was added dropwise at 0°C. After the addition, the resulting mixture was stirred at 0°C for 10 min, and then warmed to room temperature and stirred for 1 h. After the reaction was completed, the reaction solution was added dropwise to ice water (200 mL). After filtration, the filter cake was dried to obtain the title compound (1.9 g).
MS m/z (ESI): 212.0 [M+H]+.
**$^1$H NMR (400 MHz, DMSO-$d_6$)** δ 7.69 (s, 1H), 7.30 (s, 1H), 2.49 (s, 3H).

**Step 3: Synthesis of *N*-(6-acetylbenzo[*d*][1,3]dioxol-5-yl-2,2-$d_2$)acetamide (Intermediate 39-4)**

**Intermediate 39-3** (1.8 g) was dissolved in acetic acid (25 mL), and acetic anhydride (1.84 g) and reduced iron powder (4.76 g) were added. The resulting mixture was stirred at room temperature for 1 h. After the reaction was completed, filtration was performed, the filtrate was concentrated to dryness under reduced pressure, and the residue was purified by column chromatography (ethyl acetate/petroleum ether = 5 : 1) to obtain the title compound (1.5 g).
MS m/z (ESI): 224.1 [M+H]+.

**Step 4: Synthesis of *N*-(6-(2-bromoacetyl)benzo[*d*][1,3]dioxol-5-yl-2,2-$d_2$)acetamide (Intermediate 39-5)**

A solution of HBr in acetic acid (2.39 g, 33% content) was added dropwise to a solution of **Intermediate 39-4** (1.45 g) in anhydrous acetic acid (25 mL), and then $Br_2$ (1.07 g) was added dropwise. After the dropwise addition, the resulting mixture was stirred at room temperature for 1 h. After the reaction was completed, the reaction solution was concentrated to dryness under reduced pressure. The residue was added to water (50 mL), and the resulting mixture was extracted with ethyl acetate (50 mL*2). The organic phase was washed with saturated brine (50 mL), and dried over anhydrous sodium sulfate. After filtration, the residue was purified by column chromatography (ethyl acetate/petroleum ether = 5 : 1) to obtain the title compound (1.3 g).
MS m/z (ESI): 302.1 [M+H]+.

**Step 5: Synthesis of 1-(6-aminobenzo[*d*][1,3]dioxol-5-yl-2,2-$d_2$)-2-chloroethan-1-one (Intermediate 39-6)**

**Intermediate 39-5** (1.2 g) and concentrated hydrochloric acid (144.82 mg) were dissolved in ethanol (15 mL), and the reaction solution was stirred at 60°C for 16 h. After the reaction was completed, the reaction solution was concentrated to dryness under reduced pressure. The residue was purified by high performance liquid chromatography (YMC-Actus Triart C18 column 5 μm silica, 30 mm in diameter, 150 mm in length; using a decreasingly polar mixture of water (containing 0.05% $NH_4HCO_3$) and acetonitrile as the eluent; acetonitrile gradient proportion: 40%-50%) to obtain the title compound (577 mg).
MS m/z (ESI): 216.0 [M+H]+.

**Step 6: Synthesis of (*S*)-14-(chloromethyl)-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione-2,2-$d_2$ (Intermediate 39-7)**

**Intermediate 39-6** (100.0 mg) and **Intermediate 1-3** (109.87 mg) were dissolved in toluene (1 mL) and acetic acid (1 mL), and to the mixture was added pyridinium p-toluenesulfonate (5.24 mg). The reaction solution was stirred at 100°C for 16 h. After the reaction was completed, the reaction solution was directly concentrated to dryness under reduced pressure after the reaction solution was cooled to room temperature. Ethanol (5 mL) was added, and the reaction solution was stirred at 25°C for 0.5 h. The reaction solution was filtered. The filter cake was washed with ethanol (5 mL*2) to obtain the title compound (100.0 mg).
MS m/z (ESI): 443.0 [M+H]+.

**Step 7: Synthesis of (*S*)-14-(aminomethyl)-7-ethyl-7-hydroxy-10,13-dihydro-11*H*-[1,3]dioxolo[4,5-*g*] pyrano[3',4':6,7]indolizino[1,2-*b*]quinoline-8,11(7*H*)-dione-2,2-$d_2$ (Intermediate 39-8)**

**Intermediate 39-7** (100.00 mg) was dissolved in absolute alcohol (1.5 mL) and anhydrous *N,N*-dimethylformamide (1.5 mL), and to the mixture was added hexamethylenetetramine (94.97 mg). The reaction solution was stirred

at 50°C for 6 h. After the reaction was completed, the reaction solution was concentrated to dryness under reduced pressure, and the residue was purified by high performance liquid chromatography (column: Boston Green ODS 150*30 mm*5 μm; mobile phase: [A: water (formic acid), B: acetonitrile]; B%: 0%-30%, 12 min) to obtain the title compound (25.0 mg).

MS m/z (ESI): 424.0 [M+H]+.

**Step 8: Synthesis of 2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl-2,2-$d_2$)methyl)-2-hydroxyacetamide (Compound 39)**

**Intermediate 39-8** (7 mg) and **Intermediate 11-1** (5.76 mg) were dissolved in anhydrous *N,N*-dimethylformamide (0.5 mL), and to the mixture were added 2-(7-azabenzotriazol-1-yl)-*N,N,N',N'*-tetramethyluronium hexafluorophosphate (12.57 mg) and diisopropylethylamine (4.27 mg). The reaction solution was stirred at 25°C for 1 h. After the reaction was completed, the reaction solution was filtered, and the filtrate was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 20%-50%, elution time: 12 min) to obtain the title compound (2.60 mg).

MS m/z (ESI): 522.1[M+H]+.

**$^1$H NMR (400MHz, DMSO-$d_6$)** δ = 8.62 (t, *J* = 5.9 Hz, 1H), 7.84 (s, 1H), 7.51 (s, 1H), 7.24 (s, 1H), 6.49 (s, 1H), 5.48-5.41 (m, 5H), 4.72 (d, *J* = 5.5 Hz, 2H), 3.59-3.52 (m, 1H), 2.00-1.76 (m, 2H), 1.05-0.96 (m, 1H), 0.88 (t, *J* = 7.4 Hz, 3H), 0.37-0.30 (m, 2H), 0.29-0.19 (m, 2H).

**Step 9: Synthesis of 2-cyclopropyl-*N*-(((*S*)-7-ethyl-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl-2,2-$d_2$)methyl)-2-hydroxyacetamide (Compound 39-P1/P2)**

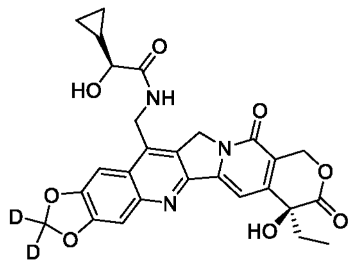 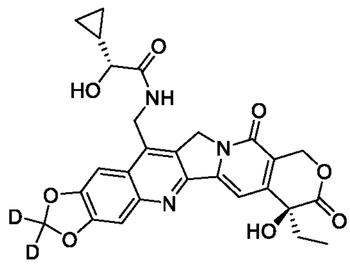

**Intermediate 39-8** (7 mg) and **Intermediate 14-10-P1** (5.76 mg) were dissolved in anhydrous *N,N*-dimethylformamide (0.5 mL), and to the mixture were added 2-(7-azabenzotriazol-1-yl)-*N,N,N',N'*-tetramethyluronium hexafluorophosphate (12.57 mg) and diisopropylethylamine (4.27 mg). The reaction solution was stirred at 25°C for 1 h. After the reaction was completed, the reaction solution was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 15%-45%, elution time: 12 min) to obtain **Compound 39-P1** (3.30 mg).

MS m/z (ESI): 522.1[M+H]+.

**$^1$H NMR (400MHz, DMSO-$d_6$)** δ = 8.62 (t, *J* = 6.0 Hz, 1H), 7.86 (s, 1H), 7.52 (s, 1H), 7.25 (s, 1H), 6.51 (s, 1H), 5.54-5.51 (m, 1H), 5.47 (s, 2H), 5.43 (s, 2H), 4.72 (d, *J* = 6.0 Hz, 2H), 3.55-3.53 (m, 1H), 1.94-1.78 (m, 2H), 1.05-0.96 (m, 1H), 0.88 (t, *J* = 7.3 Hz, 3H), 0.40-0.30 (m, 2H), 0.29-0.19 (m, 2H).

**Intermediate 39-8** (7 mg) and **Intermediate 14-10-P2** (5.76 mg) were dissolved in anhydrous *N,N*-dimethylformamide (0.5 mL), and to the mixture were added 2-(7-azabenzotriazol-1-yl)-*N,N,N',N'*-tetramethyluronium hexafluorophosphate (12.57 mg) and diisopropylethylamine (4.27 mg). The reaction solution was stirred at 25°C for 1 h. After the reaction was completed, the reaction solution was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 15%-45%, elution time: 12 min) to obtain **Compound 39-P2** (4.0 mg).

MS m/z (ESI): 522.1[M+H]+.

**$^1$H NMR (400MHz, DMSO-$d_6$)** δ = 8.62 (t, *J* = 6.1 Hz, 1H), 7.86 (s, 1H), 7.52 (s, 1H), 7.25 (s, 1H), 6.50 (s, 1H), 5.54-5.51 (m, 1H), 5.46 (s, 2H), 5.43 (s, 2H), 4.72 (d, *J* = 5.8 Hz, 2H), 3.55-3.52 (m, 1H), 1.94-1.80 (m, 2H), 1.04-0.95

(m, 1H), 0.88 (t, $J$ = 7.4 Hz, 3H), 0.39-0.30 (m, 2H), 0.29-0.21 (m, 2H).

The two isomers were further analyzed separately by the following chiral supercritical fluid chromatography.

| | |
|---|---|
| Column | Chiralcel OD-3 50A 4.6 mm I.D., 3 μm |
| Mobile phase | A: Carbon dioxide |
| | B: Ethanol (0.05% diethylamine) |
| Flow rate | 4 mL/min |
| Wavelength | PDA 254 nm |
| Column temperature | 35°C |
| Column pressure | 1500 Psi |
| Model of instrument | Waters UPCC with PDA Detector and QDa Detector |

**Compound 39-P1:**
Under the above chiral supercritical fluid chromatography conditions, the retention time was 2.877 min;
**Compound 39-P2:**
Under the above chiral supercritical fluid chromatography conditions, the retention time was 2.690 min.

**Example 40. (*S*)-2-amino-*N*-((7-ethyl-15-fluoro-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)acetamide (Compound 40)**

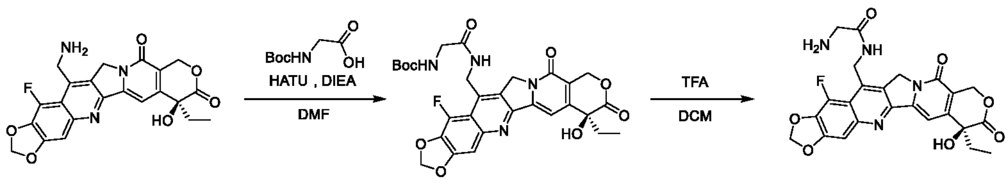


Compound 35 40-1 Compound 40

**Step 1: Synthesis of tert-butyl (*S*)-(2-(((7-ethyl-15-fluoro-7-hydroxy-8,11-dioxo-8,10,11,13-tetrahydro-10*H*-[1,3]dioxolo[4,5-*g*]pyrano[3',4':6,7] indolizino[1,2-*b*]quinolin-14-yl)methyl)amino)-2-oxoethyl)carbamate (Intermediate 40-1)**

**Compound 35** (7 mg) and 2-((tert-butoxycarbonyl)amino)acetic acid (5.58 mg) were dissolved in anhydrous *N,N*-dimethylformamide (1 mL), and to the mixture were added 2-(7-azabenzotriazol-1-yl)-*N,N,N',N'*-tetramethyluronium hexafluorophosphate (12.11 mg) and diisopropylethylamine (2.06 mg). The reaction solution was stirred at 25°C for 1 h. After the reaction was completed, the reaction solution was concentrated to dryness to obtain the title compound (8.00 mg).

MS m/z (ESI): 597.3 $[M+H]^+$.

**Step 2: Synthesis of (*S*)-2-amino-*N*-((7-ethyl-15-fluoro-7-hydroxy-8,11-dioxo-7,8,11,13-tetrahydro-10*H*-[1,3]dioxolo[4,5-*g*]pyrano[3',4':6,7]indolizino[1,2-*b*]quinolin-14-yl)methyl)acetamide (Compound 40)**

**Intermediate 40-1** (6 mg) was dissolved in dichloromethane (0.5 mL), and to the mixture was added trifluoroacetic acid (902.27 mg). The reaction solution was stirred at 25°C for 1 h. After the reaction was completed, the reaction solution was concentrated to dryness under reduced pressure. The residue was purified by preparative high performance liquid chromatography (Waters Xbridge C18 column 5 μm, 25 mm in diameter, 100 mm in length; using a decreasingly polar mixture of water (containing 0.05% formic acid) and acetonitrile as the eluent; acetonitrile gradient proportion: 2%-32%, elution time: 12 min) to obtain the title compound (1.3 mg).

MS m/z (ESI): 497.1 $[M+H]^+$.

**$^1$H NMR (400MHz, DMSO-$d_6$)** $\delta$ = 8.80-8.58 (m, 1H), 7.51 (s, 1H), 7.26 (s, 1H), 6.52 (s, 1H), 6.40 (s, 2H), 5.50 (s, 2H), 5.43 (s, 2H), 4.85 (s, 2H), 3.09-2.75 (m, 2H), 1.92-1.79 (m, 2H), 0.87 (t, $J$ = 7.1 Hz, 3H).

Compounds other than those synthesized in examples 1-40 can also be synthesized by referring to the synthetic pathways and source materials in examples 1-40.

**Test Examples of Biological Activity and Related Properties**

The compounds in the following test examples were all prepared according to the methods of the above examples of the present disclosure.

**Test example 1. Test 1 of anti-proliferative activity against tumor cells**

**Cells and materials:** Human colorectal cancer cell line HCT116 purchased from KYinno Biotechnology Co., Ltd., human breast cancer cell line SKBR3 purchased from ATCC, human ovarian cancer cell line OVCAR3 purchased from ATCC, bovine serum (Gibco#10099-141C#2186958), McCoy's 5a medium (Gibco#16600-082#2192439), 1640 medium (Gibco#A10491-01#2193156), penicillin-streptomycin (Gibco#15140-122#2211091) and 0.25% Trypsin-EDTA (Gibco#25200-056#2186958) purchased from Gibco (USA), bovine insulin (Solarbio#I8040) purchased from Solarbio, 96-well plate (Greiner Bio-one#655098#E20103H8) purchased from Corning (USA), Cell-Titer Glo reagent (Promega#G7568#0000411325) purchased from Promega (USA).

**Cell culture:** HCT116 cells and SKBR3 cells were cultured in McCoy's 5a medium containing 10% fetal bovine serum + 1% penicillin-streptomycin at 37°C and 5% $CO_2$, and OVCAR3 cells were cultured in 1640 medium containing 20% fetal bovine serum + 2 μg/mL bovine insulin + 1% penicillin-streptomycin at 37°C and 5% $CO_2$. Cells in logarithmic growth phase were suitable for the experiment.

**Detection of cell proliferation activity:** The inhibitory activity of the compounds on the proliferation of the three cell lines HCT116, SKBR3 and OVCAR3 was detected using Cell-Titer Glo reagent. HCT116 cells (1500 cells per well), SKBR3 cells (3000 cells per well) and OVCAR3 cells (5000 cells per well) were seeded in 96-well plates and cultured at 37°C and 5% $CO_2$ for 24 h. The solution of the compound to be tested (the compound was dissolved in DMSO with the concentration of the compound being 1 mM, then the compound was diluted to 3 μM with DMSO, with 3-fold dilution and a total of 9 concentrations, and 10 μL of the prepared compound solution was transferred to a 96-well plate with the final concentration of 0-300 nM) was added. The cells were placed at 37°C and 5% $CO_2$ for further culture. HCT116 cells were cultured for 3 days, and SKBR3 cells and OVCAR3 cells were cultured for 5 days. The cell viability was detected by adding Cell-Titer Glo reagent.

Additionally, a negative control group and a positive control group were provided as Bottom and Top, respectively. In the negative control group, the cells were not added, only the same volume of medium was added, and other operations were consistent with those in the experimental group; in the positive control group, the test compound was not added, only the same volume of DMSO was added, and other operations were consistent with those in the experimental group.

**Data analysis:** The % compound inhibition was calculated, and fitting was performed to obtain $IC_{50}$ of the compound.

$$\% \text{ Compound inhibition} = 1\text{-}100\% * (\text{Signal-Bottom})/(\text{Top-Bottom}).$$

Signal represents the signal value of the experimental group, Bottom represents the average signal value of the negative control group, and Top represents the average signal value of the positive control group.

**Experimental results:**

The compounds of the present disclosure exhibited strong proliferation inhibitory activity against HCT116 cells, SKBR3 cells and OVCAR3 cells under the present experimental conditions. The corresponding anti-cell proliferation activities of the compounds of the present disclosure are specifically as shown in Table 1.

**Table 1** Anti-cell proliferation activity of compounds of the present disclosure

| Compound | HCT116 Anti-proliferative activity $IC_{50}$ (nM) | SKBR3 Anti-proliferative activity $IC_{50}$ (nM) | OVCAR3 Anti-proliferative activity $IC_{50}$ (nM) |
|---|---|---|---|
| Compound 1 | 36.8 | 8.5 | N/A |
| Compound 2 | 3.6 | 2.5 | N/A |

(continued)

| Compound | HCT116 Anti-proliferative activity $IC_{50}$ (nM) | SKBR3 Anti-proliferative activity $IC_{50}$ (nM) | OVCAR3 Anti-proliferative activity $IC_{50}$ (nM) |
|---|---|---|---|
| Compound 3 | 18.2 | 3.6 | N/A |
| Compound 4 | 3.6 | 2.7 | N/A |
| Compound 5 | 16.9 | 3.3 | N/A |
| Compound 6 | 12.6 | 4.8 | 2.1 |
| Compound 7 | 22.8 | N/A | N/A |
| Compound 8 | 289.0 | 55.1 | N/A |
| Compound 9 | 4.6 | 2.1 | N/A |
| Compound 10 | 5.6 | 2.3 | N/A |
| Compound 11 | 10.4 | 3.2 | N/A |
| Compound 12 | 13.7 | 4.8 | N/A |
| Compound 13 | 8.0 | N/A | 0.7 |
| Compound 14 | 8.4 | 2.2 | 0.9 |
| Compound 14-P1 | 9.8 | 2.1 | 0.6 |
| Compound 14-P2 | 12.4 | 3.5 | 1.0 |
| Compound 15 | 81.3 | N/A | N/A |
| Compound 16 | 3.0 | 2.3 | N/A |
| Compound 17 | 6.0 | 2.7 | N/A |
| Compound 18 | 10.5 | 5.1 | N/A |
| Compound 19 | 5.0 | 4.0 | N/A |
| Compound 20 | 20.7 | 10.5 | N/A |
| Compound 21 | 7.1 | 4.0 | 1.7 |
| Compound 22 | 4.0 | 3.5 | 1.4 |
| Compound 23 | 5.6 | 5.3 | 3.9 |
| Compound 23-P1 | 7.9 | 5.8 | 1.9 |
| Compound 23-P2 | 9.5 | 4.5 | 1.5 |
| Compound 25 | 7.9 | 3.7 | 2.0 |
| Compound 26 | 9.5 | 4.2 | 1.6 |
| Compound 27 | 24.3 | N/A | N/A |
| Compound 28 | 8.8 | N/A | N/A |
| Compound 29 | 55.1 | N/A | N/A |
| Compound 30 | 4.6 | N/A | 0.4 |
| Compound 31 | 1.2 | N/A | 0.2 |
| Compound 32 | 9.8 | N/A | 1.0 |
| Compound 33 | 5.5 | N/A | 0.8 |
| Compound 34 | 2.3 | N/A | 0.6 |
| Compound 35 | N/A | N/A | 0.9 |

(continued)

| Compound | HCT116 Anti-proliferative activity $IC_{50}$ (nM) | SKBR3 Anti-proliferative activity $IC_{50}$ (nM) | OVCAR3 Anti-proliferative activity $IC_{50}$ (nM) |
|---|---|---|---|
| Compound 36 | N/A | N/A | N/A |
| Compound 37 | 8.1 | 3.5 | 1.2 |
| Compound 37-P1 | 4.8 | 2.1 | 0.5 |
| Compound 37-P2 | N/A | 3.6 | 1.8 |
| Compound 38 | N/A | N/A | 2.9 |
| Compound 39 | 9.5 | 2.9 | 1.0 |
| Compound 39-P1 | 4.6 | 1.9 | 0.6 |
| Compound 39-P2 | 6.5 | 2.9 | 1.0 |
| Compound 40 | N/A | N/A | 11.3 |
| "N/A" means not tested. | | | |

**Test example 2. Test 2 of anti-proliferative activity against tumor cells**

**Cells and materials:** Human ovarian cancer cell line SK-OV-3 purchased from ATCC, human ovarian cancer cell line PA-1 purchased from ATCC, human small cell lung cancer cell line NCI-H82 purchased from ATCC, human breast cancer cell line MDA-MB-231 purchased from ATCC, human non-small cell lung cancer cell line A549 purchased from ATCC, bovine serum (Gibco#10099-141C), McCoy's 5a medium (Gibco#16600-082), MEM medium (Gibco#11095-080), 1640 medium (Gibco#A10491-01), DMEM medium (Gibco#11995-065), MEM NEAA (Gibco#11140-050), sodium pyruvate (Gibco#11360-070), penicillin-streptomycin (Gibco#15140-122) and 0.25% Trypsin-EDTA (Gibco#25200-056) purchased from Gibco (USA), bovine insulin (Solarbio#I8040) purchased from Solarbio, 96-well plate (Greiner Bio-one#655098) purchased from Corning (USA), Cell-Titer Glo reagent (Promega#G7568) purchased from Promega (USA).

**Cell culture:** SK-OV-3 cells were cultured in McCoy's 5a medium containing 10% fetal bovine serum + 1% penicillin-streptomycin, PA-1 cells were cultured in MEM medium containing 10% fetal bovine serum + 1% MEM NEAA + 1% sodium pyruvate+1% penicillin-streptomycin, NCI-H82 cells and MDA-MB-231 cells were cultured in 1640 medium containing 10% fetal bovine serum + 1% penicillin-streptomycin, A549 cells were cultured in DMEM medium containing 10% fetal bovine serum + 1% penicillin-streptomycin, all under conditions of 37°C and 5% $CO_2$. Cells in logarithmic growth phase were suitable for the experiment.

**Detection of cell proliferation activity:** The inhibitory activity of the compounds on the proliferation of the five cell lines SK-OV-3, PA-1, NCI-H82, MDA-MB-231 and A549 was detected using Cell-Titer Glo reagent. SK-OV-3 cell (1000 cells per well), PA-1 cells (800 cells per well), NCI-H82 cells (5000 cells per well), MDA-MB-231 cells (3000 cells per well) and A549 cells (400 cells per well) were seeded in 96-well plates and cultured at 37°C and 5% $CO_2$ for 24 h. The compound to be tested was dissolved in DMSO to a concentration of 1 mM, then subjected to gradient dilution with DMSO and corresponding medium, and transferred to 96-well cell plates to a final concentration of 300 nM (initial concentration), with 3-fold dilution and a total of 9 concentration points. The cells were cultured at 37°C and 5% $CO_2$ for additional 5 days. The cell viability was detected by adding Cell-Titer Glo reagent.

Additionally, a negative control group and a positive control group were provided as Bottom and Top, respectively. In the negative control group, the cells were not added, only the same volume of medium was added, and other operations were consistent with those in the experimental group; in the positive control group, the test compound was not added, only the same volume of DMSO was added, and other operations were consistent with those in the experimental group.

**Data analysis:** The % compound inhibition was calculated, and fitting was performed to obtain $IC_{50}$ of the compound.

$$\text{\% Compound inhibition} = 1\text{-}100\% * (\text{Signal-Bottom})/(\text{Top-Bottom}).$$

Signal represents the signal value of the experimental group, Bottom represents the average signal value of

the negative control group, and Top represents the average signal value of the positive control group.

**Experimental results:**

The compounds of the present disclosure exhibited strong proliferation inhibitory activity against the five cell lines PA-1, SK-OV-3, NCI-H82, MDA-MB-231 and A549 under the present experimental conditions. The corresponding anti-cell proliferation activities of the compounds of the present disclosure are specifically as shown in Table 2. Table 1 and Table 2 show that the compounds of the present disclosure exhibit strong proliferation inhibitory activity against tumor cells such as human colorectal cancer cell line HCT116, human breast cancer cell lines SKBR3 and MDA-MB-231, human small cell lung cancer cell line NCI-H82, human non-small cell lung cancer cell line A549 and human ovarian cancer cell lines OVCAR3, PA-1, and SK-OV-3 at the same time, and have good therapeutic potential against various refractory tumors with high incidence, such as intestinal cancer, breast cancer and lung cancer.

**Table 2** Anti-cell proliferation activity of compounds of the present disclosure

| Compound | PA-1 Anti-proliferative activity $IC_{50}$ (nM) | SK-OV-3 Anti-proliferative activity $IC_{50}$ (nM) | NCI-H82 Anti-proliferative activity $IC_{50}$ (nM) | MDA-MB-231 Anti-proliferative activity $IC_{50}$ (nM) | A549 Anti-proliferative activity $IC_{50}$ (nM) |
|---|---|---|---|---|---|
| Compound 14 | 1.0 | 3.0 | 3.9 | 10.4 | 9.7 |
| Compound 14-P1 | 0.7 | 2.2 | 2.2 | 7.5 | 10.2 |
| Compound 14-P2 | 0.9 | 3.2 | 4.7 | 8.8 | 14.3 |
| Compound 31 | 1.7 | 12.3 | 4.0 | 13.5 | 15.9 |
| Compound 35 | 0.8 | N/A | N/A | N/A | N/A |
| Compound 37 | 0.9 | 3.5 | N/A | 23.9 | 19.6 |
| Compound 37-P1 | 0.9 | 2.1 | N/A | 13.2 | 9.1 |
| Compound 37-P2 | 1.1 | 3.0 | N/A | 25.9 | 16.1 |
| Compound 39 | 1.5 | 2.6 | N/A | 26.4 | 19.1 |
| Compound 39-P1 | 0.9 | 1.9 | N/A | 15.6 | 13.9 |
| Compound 39-P2 | 1.2 | 2.1 | N/A | 23.2 | 15.7 |
| "N/A" means not tested. | | | | | |

**Test example 3. Determination of metabolic stability of compounds of the present disclosure in liver microsomes**

The metabolic stability of the compounds of the present disclosure in liver microsomes was determined by the following experimental method.

I. Experimental materials and instruments

1. Human liver microsomes (Corning 452117), Beagle dog liver microsomes (XENOTECH D1000), SD rat liver microsomes (XENOTECH R1000) and CD-1 mouse liver microsomes (XENOTECH M1000)
2. $Na_2HPO_4$ (Tianjin Guangfu Fine Chemical Research Institute 20180130)
3. $KH_2PO_4$ (Tianjin Guangfu Fine Chemical Research Institute 20180920)
4. MgCh (Tianjin Guangfu Fine Chemical Research Institute 20191216)
5. NADPH (Solarbio 1216C022)
6. Positive control compound verapamil (Sigma MKBV4993V)
7. AB Sciex API4000 liquid chromatography-mass spectrometry

II. Experimental steps

1. Preparation of 100 mM phosphate buffered saline (PBS): 7.098 g of $Na_2HPO_4$ was weighed, 500 mL of pure water was added and subjected to ultrasonic dissolution to obtain solution A. 3.400 g of $KH_2PO_4$ was weighed, 250 mL of pure water was added and subjected to ultrasonic dissolution to obtain solution B. Solution A was placed on a stirrer and

solution B was slowly added until the pH value reached 7.4 to prepare 100 mM PBS buffer.
2. Preparation of reaction system
The reaction system was prepared according to the following table:

| Reagent | Stock solution concentration | Volume | Final concentration |
|---|---|---|---|
| Liver microsomes | 20 mg/mL | 10 μL | 0.5 mg/mL |
| Phosphate buffer | 100 mM | 346 μL | 100 mM |

3. The reaction system was placed in a 37°C water bath and pre-incubated for 10 min. 40 μL of 10 mM NADPH solution (NADPH was dissolved in 100 mM phosphate buffer) was added to the reaction system with the final concentration of NADPH being 1 mM. 40 μL of phosphate buffer was used instead of NADPH solution as negative control. The role of the negative control was to exclude the effect of the chemical stability of the compounds themselves.
4. 4 μL of 100 μM compound of the present disclosure and the positive control compound verapamil were added to the reaction system to initiate the reaction, with the final concentration of the compound being 1 μM.
5. After thorough mixing on a vortex oscillator, 50 μL of the incubated sample was taken out at 0.5 min, 15 min, 30 min, 45 min and 60 min, respectively, and the reaction was stopped with 200 μL of glacial acetonitrile containing internal standard. The sample was centrifuged at 3220 g for 45 min. After the completion of the centrifugation, 90 μL of the supernatant was transferred to a loading plate, and 90 μL of ultrapure water was added and mixed uniformly for LC-MS/MS analysis.

All the data was calculated by the Microsoft Excel software. The peak area was detected by extracting the ion chromatogram. The *in vitro* half-life ($t_{1/2}$) of the compound was determined by performing linear fitting of the natural logarithm of elimination percent of the compound and time.

The *in vitro* half-life ($t_{1/2}$) was calculated by means of the slope *k:*

$$\textit{in vitro}\ t_{1/2} = 0.693/k$$

The *in vitro* intrinsic clearance (unit: μL/min/mg protein) was calculated using the following equation:

$$\textit{in vitro}\ CL_{int} = k \times \text{volume of incubation (μL)/amount of proteins (mg)}$$

wherein $CL_{int}$ is the inherent clearance rate; *k* is the elimination rate constant; volume of incubation is the incubation volume (μL); and amount of proteins is the protein amount (mg)

The compounds of the present disclosure had good stability in liver microsomes, see Table 3 for details.

**Table 3**

| Compound | Stability in human liver microsomes $t_{1/2}$ (min) | Stability in mouse liver microsomes $t_{1/2}$ (min) |
|---|---|---|
| Compound 14-P1 | 66.3 | 72.8 |
| Compound 14-P2 | 50.1 | 65.0 |
| Compound 37-P1 | 105.1 | 133.8 |
| Compound 39 | 240.9 | 268.4 |

**Test example 4. Determination of membrane permeability and transport characteristics of compounds of the present disclosure**

The membrane permeability and transport characteristics of the compounds of the present disclosure were determined by the following experimental method.

I. Experimental materials and instruments

1. Caco-2 cells (ATCC)
2. HEPES (Solarbio 804D049), penicillin/streptomycin (Solarbio 20200109) and PBS (Solarbio 20200620)

3. Fetal bovine serum (FBS) (Sigma WXBD0055V), fluorescein (Sigma MKCJ3738) and $NaHCO_3$ (Sigma SLBZ4647)
4. Hank's balanced salt solution (HBSS) (Gibco 2085528), non-essential amino acid (NEAA) (Gibco 2211548) and Trypsin/EDTA (Gibco 2120732)
5. High glucose DMEM (Corning 20319014)
6. HTS Transwell-96 Well Permeable (Corning, 3391)
7. Resistance tester (Millipore, Millicell® ERS-2)
8. Cellometer® Vision (Nexcelom Bioscience)
9. Infinite 200 PRO microplate reader (Tecan, Infinite M200PRO)
10. Positive control compounds metoprolol (Sinopharm 100084-201403), erythromycin (MCE 84550) and cimetidine (Sinopharm 100158-201406)
11. ABI QTrap 5500 liquid chromatography-mass spectrometry

II. Experimental steps

1. Caco-2 cell culture

1) Transport buffer (HBSS containing 25 mM HEPES, pH 7.4) was prepared: 5.958 g of HEPES and 0.35 g of NaHCOs were accurately weighed, 900 mL of pure water was added to dissolve same, then 100 mL of 10×HBSS was added, uniform stirring was performed, the pH was adjust to 7.4, and filtering was performed.
2) Caco-2 cell culture medium was prepared: FBS, penicillin/streptomycin, kanamycin and NEAA were added to high glucose DMEM (containing L-glutamine) medium to prepare a cell culture medium containing 10% FBS, 100 U/mL penicillin/0.1 mg/mL streptomycin, 0.6 μg/mL kanamycin and 1×NEAA.
3) Cells were cultured in T-75 culture flasks in an incubator at 37°C and 5% $CO_2$, and the medium was discarded when the cell growth density reached 80-90%. The cells were rinsed with 5 mL of PBS, 1.5 mL of Trypsin/EDTA was added, then the cells were incubated in an incubator at 37°C for 5-10 min until the cells fell off in the form of quicksand, and finally Trypsin/EDTA was neutralized with an FBS-containing medium.
4) The cell suspension was centrifuged at 120 g for 10 min, and the supernatant was discarded.
5) The cell culture medium was added to re-suspend the cells to obtain a cell suspension with a density of $6.86 \times 10^5$ cells/mL.

2. Caco-2 cell seeding

1) 50 μL of medium was added to each well of Transwell chamber, and 25 mL of medium was added to the lower layer. The Transwell chamber was preheated in an incubator at 37°C and 5% $CO_2$ for 1 h.
2) 50 μL of the cell suspension was added to each well of the preheated Transwell chamber to a final seeding density of $2.4 \times 10^5$ cells/cm.
3) The cells were cultured for 14-18 days, the medium was changed every other day, and the medium was changed within 48 h after the initial seeding in the plate. The medium must be changed one day before the experiment.

3. Evaluation of monolayer cell membrane integrity

1) After 14 days of cell culture, the cells were fused and differentiated, ready for transport experiment.
2) The resistance of monolayer cell membrane was measured by the resistance tester, and the resistance of each well was recorded.
3) After the measurement was completed, the Transwell culture plate was re-incubated.
4) The TEER value was calculated:

$$\text{TEER value} = \text{measured TEER value } (\Omega) \times \text{membrane area } (\text{cm}^2)$$

The resistance of monolayer cell membrane< 230 $\Omega\cdot cm^2$, indicating that the monolayer cell membrane had poor density and could not be used in the experiment.

4. Transport experiment

1) The 10 mM stock solution of the compound of the present disclosure or the positive control compound was diluted with DMSO to obtain a 2 mM stock solution, and then the 2 mM stock solution was diluted with the transport buffer to obtain a 10 μM working solution of the compound of the present disclosure or the positive control compound.

2) The Caco-2 cell plate was taken out of the incubator, and then the Transwell plate was washed twice with the preheated transport buffer, and incubated in the incubator at 37°C for 30 min.

3) To determine the rate of transporting the compound from the apical end to the basal end (A→B), 108 μL of the working solution of the compound was added to the Transwell chamber (apical end); at the same time, 8 μL of the sample was immediately taken from the apical end and transferred to 72 μL of transport buffer, and 240 μL of stop solution containing internal standard was added to stop the transport, so as to serve as the initial apical sample. At the same time, 300 μL of the transport buffer was added to the acceptor end (basal end). Double samples were set for the experiment.

4) To determine the rate of transporting the compound from the basal end to the apical end (B→A), 308 μL of the working solution of the compound was added to the basal end; at the same time, 8 μL of the sample was immediately taken from the basal end and transferred to 72 μL of transport buffer, and 240 μL of stop solution containing internal standard was added to stop the transport, so as to serve as the initial basal sample. At the same time, 100 μL of the transport buffer was added to the Transwell chamber (apical end). Double samples were set for the experiment.

5) The cell culture plate was incubated in an incubator at 37°C and 5% $CO_2$ for 2 h.

6) After the completion of the transport experiment, 8 μL of the sample was taken from the administration end (i.e., the apical end in the A→B direction and the basal end in the B→A direction) and transferred to 72 μL of transport buffer, and then 240 μL of stop solution containing internal standard was added to stop the transport. 80 μL of the sample was taken from the acceptor end (i.e., the basal end in the A→B direction and the apical end in the B→A direction) and transferred to 240 μL of stop solution containing internal standard, vortexed at 1000 rpm for 10 min, and centrifuged at 3220 g for 30 min. 100 μL of the supernatant was taken into a loading plate, and 100 μL of ultrapure water was added and mixed uniformly for LC-MS/MS analysis.

7) After the completion of the transport experiment, the fluorescence value was measured, and a 10 mM fluorescein stock solution was prepared with water, and then diluted to 100 μm with transport buffer. 100 μL of fluorescein solution was added to the Transwell chamber (apical end) and 300 μL of transport buffer was added to the basal end, and the Transwell chamber was incubated in an incubator at 37°C and 5% $CO_2$ for 30 min. 80 μL of the solution was taken from the apical end and the basal end and transferred to a 96-well plate, and the fluorescence value of the cells was measured by a microplate reader at the excitation wavelength of 485 nm and the emission wavelength of 530 nm (to detect the membrane integrity).

Percentage leakage (%) or LY (%) was calculated by the following equation:

$$\text{Percentage Leakage} = \{I_{\text{acceptor}} \times 0.3/(I_{\text{acceptor}} \times 0.3+I_{\text{donor}}\times 0.1)\} \times 100\%$$

$I_{\text{acceptor}}$ refers to the fluorescence density on the acceptor side (0.3 mL), $I_{\text{donor}}$ refers to the fluorescence density on the administration side (0.1 mL). LY > 1.0% indicates poor density of the monolayer cell membrane, and the corresponding results will be excluded from the evaluation.

The peak areas of the compound on the administration side and the acceptor side were determined, and the apparent permeability coefficient ($P_{\text{app}}$, unit: cm/s) and Efflux ratio of the compound were calculated:

$$P_{\text{app}} = \{V_{\text{A}} \times [drug]_{acceptor}/(Area \times incubation\ time \times [drug]_{initial\ donor}\}$$

$V_A$ is the volume of solution at the acceptor end (A→B is 0.3 mL, B→A is 0.1 mL), Area (membrane area) is the membrane area of Transwell-96-well plate (0.143 $cm^2$); incubation time is the time for incubation (unit: s); $[drug]_{acceptor}$ is the concentration of the drug at the acceptor end; $[drug]_{initial\ donor}$ is the initial concentration of the drug on the administration side.

$$Efflux\ Ratio = \frac{P_{app\ (B-A)}}{P_{app\ (A-B)}}$$

$P_{app\ (B-A)}$ is the apparent permeability coefficient from the basal end to the apical end; $P_{app\ (A-B)}$ is the apparent permeability coefficient from the apical end to the basal end.

The compounds of the present disclosure had good membrane permeability and transport properties, see Table 4 for details.

**Table 4**

| Compound | $P_{app}$ (A-B) ($10^{-6}$ cm/s) | $P_{app}$ (B-A) ($10^{-6}$ cm/s) | Efflux ratio |
|---|---|---|---|
| Compound 14 | 0.26 | 3.95 | 15.2 |
| Compound 39 | 0.71 | 4.21 | 5.9 |

**Test example 5. Determination of plasma protein binding rate of compounds of the present disclosure**

The protein binding rates of the compounds of the present disclosure in human and mouse plasma were determined by the following experimental method.

I. Experimental materials and instruments

1. Human plasma (BioIVT), CD-1 mouse plasma (BioIVT)
2. $Na_2HPO_4$ (Sigma S5136-500G)
3. $NaH_2PO_4$ (Sigma S3139-500G)
4. NaCl (Sigma S5886-IKG)
5. 96-well equilibrium dialysis plate (HTDialysis LLC, Gales Ferry, CT, HTD96B) and equilibrium dialysis membrane (MWCO 12-14K, 1101)
6. Positive control compound warfarin
7. ABI QTrap 5500 liquid chromatography-mass spectrometry

II. Experimental steps

1. Preparation of a buffer with a concentration of 100 mM sodium phosphate and 150 mM NaCl: An alkaline solution with a concentration of 14.2 g/L $Na_2HPO_4$ and 8.77 g/L NaCl was prepared with ultrapure water, and an acidic solution with a concentration of 12.0 g/L $NaH_2PO_4$ and 8.77 g/L NaCl was prepared with ultrapure water. Then the alkaline solution was titrated to pH 7.4 with the acidic solution to prepare a buffer with a concentration of 100 mM sodium phosphate and 150 mM NaCl.
2. Preparation of dialysis membrane: The dialysis membrane was soaked in ultrapure water for 60 min to separate the membrane into two pieces, then soaked in 20% ethanol for 20 min, and finally soaked in a buffer used for dialysis for 20 min.
3. Preparation of plasma: The frozen plasma was quickly thawed at room temperature, and then centrifuged at 3220 g for 10 min at 4°C to remove clots, and the supernatant was collected into a new centrifugal tube. The pH of the plasma was determined and recorded, and the plasma with pH of 7-8 was used.
4. Preparation of compound-containing plasma samples: The 10 mM stock solution of the compound of the present invention or the positive control compound was diluted with DMSO to obtain a 200 μM working solution. To 597 μl of human or mouse plasma was added 3 μl of 200 μM compound working solution to obtain a plasma sample with a final concentration of 1 μM.
5. Equilibrium dialysis steps: The dialysis devices were assembled according to the operating instructions. 120 μL of plasma sample containing 1 μM compound was added to one side of the dialysis membrane, and an equal volume of dialysate (phosphate buffer) was added to the other side thereof. Double samples were set for the experiment. The dialysis plate was sealed, placed in the incubation device, and incubated at 37°C, 5% $CO_2$ and about 100 rpm for 6 h. After the completion of the incubation, the sealing film was removed and 50 μl of sample was pipetted from the buffer and plasma sides in each well, respectively, and transferred into separate wells of a new plate. 50 μL of blank plasma was added to the phosphate buffer sample, an equal volume of blank phosphate buffer was added to the plasma sample, and then 300 μL of acetonitrile containing the internal standard was added to precipitate the protein. The mixture was vortexed for 5 min and centrifuged at 3220 g for 30 min at 4°C. 100 μL of the supernatant was taken into a loading plate, and 100 μL of ultrapure water was added and mixed uniformly for LC-MS/MS analysis.

The peak areas of the compound on the buffer side and the plasma side were determined. The equation for calculating the plasma protein binding rate of the compound is as follows:

% Free rate = (ratio of compound peak area to internal standard peak area$_{\text{buffer side}}$/ratio of compound peak area to internal standard peak area$_{\text{plasma side}}$) × 100%.

$$\% \text{ Binding rate} = 100\% - \% \text{ free rate.}$$

**Table 5**

| Compound | % Human plasma protein binding rate | % Mouse plasma protein binding rate |
|---|---|---|
| Compound 14-P1 | 89.4 | 63.7 |
| Compound 37-P1 | 87.2 | 67.3 |
| Compound 39 | 91.9 | 65.8 |

**Test example 6. Inhibitory effect of compounds of the present disclosure on CYP2C9, CYP2D6 and CYP3A4 enzymatic activities**

The inhibition of CYP2C9, CYP2D6 and CYP3A4 enzymatic activities by the compounds of the present disclosure was determined by the following experimental method.

I. Experimental materials and instruments

1. Human liver microsomes (Corning 452117)
2. NADPH (Solarbio 705Y021)
3. Positive substrates diclofenac (Sigma SLBV3438), dextromethorphan (TRC 3-EDO-175-1) and midazolam (Cerilliant FE01161704)
4. Positive inhibitors sulfaphenazole (D. Ehrenstorfer GmbH 109012), quinidine (TCI WEODL-RE) and ketoconazole (Sigma 100M1091V)
5. AB Sciex Triple Quad 5500 liquid chromatography-mass spectrometry

II. Experimental steps

1. Preparation of 100 mM phosphate buffered saline (PBS): 7.098 g of $Na_2HPO_4$ was weighed, 500 mL of pure water was added and subjected to ultrasonic dissolution to obtain solution A. 3.400 g of $KH_2PO_4$ was weighed, 250 mL of pure water was added and subjected to ultrasonic dissolution to obtain solution B. Solution A was placed on a stirrer and solution B was slowly added until the pH value reached 7.4 to prepare 100 mM PBS buffer.
2. Preparation of 10 mM NADPH solution with 100 mM PBS buffer. The 10 mM stock solution of the compound of the present disclosure was diluted with DMSO to obtain a 200× concentration of compound working solution (6000, 2000, 600, 200, 60, 20, 0 μM). The stock solution of positive inhibitors was diluted with DMSO to obtain a 200× concentration of positive inhibitor working solution (sulfaphenazole, 1000, 300, 100, 30, 10, 3, 0 μM; quinidine/ketoconazole, 100, 30, 10, 3, 1, 0.3, 0 μM). A 200× concentration of substrate working solution (120 μM diclofenac, 400 μM dextromethorphan, and 200 μM midazolam) was prepared with water, acetonitrile, or acetonitrile/methanol.
3. 2 μL of 20 mg/ml liver microsome solution, 1 μL of substrate working solution, 1 μL of compound working solution and 176 μL of PBS buffer were taken, mixed uniformly, and pre-incubated in a 37°C water bath for 15 min. To the positive control group was added 1 μL of sulfaphenazole, quinidine or ketoconazole working solution instead of compound working solution. A 10 mM NADPH solution was also pre-incubated in the 37°C water bath for 15 min. After 15 min, 20 μL of NADPH was taken and added to each well to initiate the reaction, and the reaction was incubated at 37°C for 5 min (CYP2C9), 20 min (CYP2D6) or 5 min (CYP3A4). Double samples were set for all incubation samples. After the corresponding time of incubation, 400 μL of glacial methanol containing internal standard was added to all samples to stop the reaction. The mixture was mixed uniformly

by vortex and centrifuged at 3220 g for 40 min at 4°C. After the completion of the centrifugation, 100 μL of the supernatant was transferred to a loading plate, and 100 μL of ultrapure water was added and mixed uniformly for LC-MS/MS analysis.

The reduction in metabolite production in the administration group compared to the control group was compared by the ratio of sample peak area to internal standard peak area, and $IC_{50}$ value was calculated by Excel XLfit 5.3.1.3.

The percent remaining activity was calculated by the following equation:

Percent remaining activity = ratio of metabolite peak area to internal standard peak area$_{\text{test substance}}$ / ratio of metabolite peak area to internal standard peak area$_{\text{blank solvent}}$ × 100%.

Drug-drug interaction (DDI) refers to physical or chemical changes caused by two or more drugs and changes in efficacy due to these changes. Understanding drug-drug interaction can provide patients with better pharmaceutical service, promote rational use of drugs and maximumly avoid adverse reactions. Drug-drug interactions are dominated by metabolic interactions, which are mainly related to CYP450 enzymes involved in drug metabolism. The experimental results in Table 6 show that the compounds of the present disclosure had a poor ability to inhibit CYP450, indicating a lower potential risk of developing DDI for the compounds of the present disclosure.

**Table 6**

| Compound | Inhibition on CYP2C9 $IC_{50}$ (μM) | Inhibition on CYP2D6 $IC_{50}$ (μM) | Inhibition on CYP3A4 $IC_{50}$ (μM) |
|---|---|---|---|
| Compound 14-P1 | >50 | >50 | >50 |
| Compound 14-P2 | >50 | >50 | >50 |
| Compound 37-P1 | >50 | >50 | >50 |
| Compound 39 | >50 | >50 | >50 |

**Test example 7. Determination of hERG inhibitory activity of compounds of the present disclosure**

The inhibition of hERG activity by the compounds of the present disclosure was determined by the following experimental method.

I. Experimental materials and instruments

| Raw materials/Instruments | | Manufacturer (catalog number) |
|---|---|---|
| 1. | F-12 medium | Gibco (11765054) |
| 2. | Bovine serum albumin | Hyclone (SH30070) |
| 3. | Dimethyl sulfoxide | Merck (102952) |
| 4. | Phosphate buffer (without calcium and magnesium ions) | Gibco (14190) |
| 5. | Penicillin-streptomycin (10000 U/mL) | Gibco (15140122) |
| 6. | TrypLE™ Express cell digestion solution | Gibco (12604) |
| 7. | Hygromycin | Invivogen (ant-hg-5) |
| 8. | Geneticin | Gibco (11811031) |
| 9. | StemPro™ Accutase™ cell digestion solution | Gibco (A1110501) |
| 10. | 1 M HEPES buffer (100 mL) | Gibco (15630-080) |
| 11. | UltraPure™ 0.5 M EDTA | Invitrogen (15575020) |
| 12. | Escin | Sigma (C4740) |
| 13. | 384 well microplate | Greiner (781201) |

(continued)

| Raw materials/Instruments | | Manufacturer (catalog number) |
|---|---|---|
| 14. | 10 cm cell culture dish | NEST (704001) |
| 15. | 5 mL pipette | BIOFIL (GSP110005) |
| 16. | 10 mL pipette | BIOFIL (GSP110010) |
| 17. | 175 $cm^2$ cell culture flask | CORNING (431082) |
| 18. | iSeries, 50 $\mu$L tip | Beckman coulter (B85753) |
| 19. | NPC-384 patch clamp chip | Nanion (221401, 4xhigh) |
| 20. | Thermostatic carbon dioxide incubator | Thermo (371) |
| 21. | Apricot high-throughput automated pipetting workstation | Apricot Designs |
| 22. | Echo | LABCYTE (550) |
| 23. | Microplate shaker | IKA (MS3 digital) |
| 24. | Countess fully automated Cell Counter | Invitrogen (Countess II) |
| 25. | SyncroPatch 384i | Nanion (384i) |

II. Cell line and cell culture

A CHO cell line stably expressing hERG ion channel was purchased from B'SYS GmbH (Switzerland). The cells were cultured in F-12 (HAM) medium containing 10% FBS buffer, 100 U/mL penicillin-streptomycin, 100 $\mu$g/mL hygromycin and 100 $\mu$g/mL G418. Trypsin substitute TrypLE™ Express was used for digestion and passage, passaging three times per week and maintaining about 80% confluence.

III. Preparation of intracellular/extracellular fluid and preparation of solution of compound to be tested

Extracellular fluid

1) NMDG 60 standard extracellular fluid: 80 mM sodium chloride, 60 mM NMDG, 4 mM potassium chloride, 2 mM calcium chloride, 1 mM magnesium chloride, 5 mM polyglucose, 10 mM HEPES, pH adjusted to 7.4 with HCl, osmotic pressure 289 mOsm/kg;
2) NMDG 60 cell sealing solution: 80 mM sodium chloride, 60 mM NMDG, 4 mM potassium chloride, 10 mM calcium chloride, 1 mM magnesium chloride, 5 mM polyglucose, 10 mM HEPES, pH adjusted to 7.4 with HCl, osmotic pressure 313 mOsm/kg;
3) Chip filling solution: 140 mM sodium chloride, 4 mM potassium chloride, 5 mM polyglucose, 10 mM HEPES, pH adjusted to 7.4 with NaOH, osmotic pressure 289 mOsm/kg;
4) Standard extracellular fluid: 140 mM sodium chloride, 4 mM potassium chloride, 2 mM calcium chloride, 1 mM magnesium chloride, 5 mM polyglucose, 10 mM HEPES, pH adjusted to 7.4 with NaOH, osmotic pressure 298 mOsm/kg.

Intracellular fluid

KF110 intracellular fluid: 10 mM EGTA, 10 mM HEPES, 10 mM potassium chloride, 10 mM sodium chloride, 110 mM potassium fluoride, pH adjusted to 7.2 with KOH, osmotic pressure greater than 280 mOsm/kg.

Solution of compound to be tested

1) The compound to be tested was dissolved in DMSO and prepared into a stock solution with a final concentration of 10 mM.
2) The stock solution was subjected to gradient dilution with DMSO as a solvent at a ratio of 1 : 3 into solutions with other three intermediate concentrations (mM): 3.33, 1.11 and 0.37, respectively.
3) Before the start of the experiment, the gradient solution of the compound to be tested was diluted again with the extracellular fluid according to a ratio of 1 : 500 into a series of working solutions with final concentrations of: 20,

6.66, 2.22 and 0.74 (μM), respectively, while the 60 μM working solution was diluted from 10 mM stock solution according to a ratio of 3 : 500. In the experiment, 40 μL of working solution was added to 40 μL of cell solution to obtain the compound working solution with 2 times the experimental concentration.

4) The potential inhibitory effects on the hERG channel at 5 different concentration points 30, 10, 3.33, 1.11 and 0.37 μM in the given concentration gradient were tested, the concentration-effect curve was fitted, and the corresponding $IC_{50}$ values were calculated.

IV. Experimental steps

Preparation before experiment

1) "Home All Axes" was run on SyncroPatch 384i system.
2) The "LH_Startup" method was run, cleaning the instrument before the start of the experiment.
3) No.1 tube was placed into a bottle filled with an internal solution, placed at No. 1 position, and pre-filled with the intracellular fluid.
4) The prepared extracellular fluid and working concentration of compound plate were placed in the corresponding plate positions in the instrument, with the experimental preparation done.

Cell treatment

1) Adherent cells were taken from two T175 culture flasks, and the supernatant of the medium was discarded.
2) At room temperature, 8 mL of DPBS-2 mM EDTA was pipetted by a 10 mL pipette to rinse the cells 2 times, removing excess medium.
3) 3 mL of TrypLE™ Express was added into the culture flask, and gently shaken so that the solution covered the entire cell plane.
4) Half the volume of digestion solution was removed so that only a thin layer of the digestion solution spread on the cell surface.
5) The cells were incubated at 37°C for 8 to 10 min, and gently shaken under a microscope to observe the floating situation of the cells.
6) 10 mL of F-12 medium containing 15 mM HEPES was prepared in a centrifugal tube, and 10 mL standard extracellular fluid was added. 3 mL of mixed solution was added to each culture flask and incubated in a refrigerator at 4°C to 8°C for 5 min.
7) The cells were pipetted gently 3 to 5 times by a pipette to disperse the cells and transferred into a 10 cm cell culture dish.
8) The cells were counted, and diluted with a cold standard extracellular fluid to ensure the final density of 0.5-2*$10^6$ cells/mL.
9) The diluted cell suspension was transferred into a 10 cm low adsorption cell culture dish and incubated at 4°C to 10°C for 10 min.
10) The cells were pipetted gently to mix same uniformly, and transferred into a special Teflon plate for SyncroPatch 384i system. The plate was placed into the cell incubation tank of an automated patch clamp system, and incubated at 15°C.

The electrophysiological signals were recorded by SyncroPatch 384i system.

1) The pipette tip was loaded and cleaned.
2) The chip was filled with the chip filling solution, and the binding potential was compensated.
3) The cell suspension was added to the chip.
4) The cell sealing solution was added, and the holding potential was set to -90 mV.
5) The cells were rinsed 4 times with the extracellular fluid.
6) 5 μM Escin in the internal solution was perforated into the cells to obtain the intact cell structure.
7) Analog Cslow and Digital Cslow were compensated.
8) The holding potential was set to 500 ms, -90 mV; the current sampling frequency was set to 500 Hz, and the filtering frequency was set to 3k Hz. The detection condition of leakage current was -90 mV, and the time course was 500 ms.

9) The membrane potential was depolarized from -90 mV to +30 mV by applying depolarization voltage for 4.8 s, followed by an instantaneous application of a repolarization voltage for 5.2 s to make the membrane potential down to -50 mV to remove channel inactivation, so that hERG tail current was observed. The peak value of the tail current is the magnitude of the hERG current. The sampling interval for this stimulation pattern was 15 s.
10) The hERG currents used to detect the compounds to be tested were continuously recorded for 120 s before administration to evaluate the stability of the hERG currents produced by the test cells. Subsequent detection results were confident only for stable cells within the acceptance range of the evaluation criteria.
11) A stable hERG current was determined and used as a baseline for detection. After the hERG current remained stable for at least 5 min, the solution containing the compound to be tested was perfused around the cells. After the current tended to be stable, 5 stable hERG current values were read. If the current did not reach the steady state within 10 min, the last 5 current peaks recorded were taken as the readings. Cisapride was used as a positive control in the experiment to verify the stability of experimental cells and the accuracy of experimental results. In the present experiment, the inhibitory effects of the sample at 5 different concentrations on the hERG current were detected in 2 independent experimental hole positions (n = 2) to fit the $IC_{50}$ curve.

V. Data acceptance criteria

The following criteria will be used to determine the acceptability of data:

1) The initial seal resistance was greater than 100 MS2;
2) The series resistance was less than 25 MΩ;
3) The leakage current at the detection voltage was less than 50% of the current value under this condition;
4) The tail current was greater than the plateau current magnitude of the pre-pulse, and the initial tail current value was greater than 150 pA;
5) The decay rate of the tail current was lower than 30%.

VI. Data analysis

Data meeting the above hERG current quality standards would be further analyzed by the following specific steps:

1) The current inhibition rate was calculated by the following equation.
Notes: Data was output by Data control 384 software.

$$\text{\% Tail current rejection rate} = \frac{\text{Tail current magnitude}_{\text{compound}} - \text{tail current magnitude}_{\text{positive control}}}{\text{Tail current magnitude}_{\text{blank}} - \text{tail current magnitude}_{\text{positive control}}}\ ) \times 100\%$$

2) The dose-effect curve was fitted by Graphpad Prism 8.0 software, and $IC_{50}$ was calculated.

None of the compounds of the present disclosure significantly inhibited the hERG potassium ion channel, indicating that the compounds had a low risk of cardiotoxicity due to the inhibition of the hERG potassium ion channel. The hERG potassium ion channel inhibitory activity of the compounds of the present disclosure are specifically as shown in Table 7.

**Table 7**

| Compound | $IC_{50}$ (μM) |
|---|---|
| Compound 14-P1 | >30 |
| Compound 14-P2 | >30 |
| Compound 37-P1 | >30 |
| Compound 39 | >30 |

**Claims**

1. A compound of formula (I), or a stereoisomer or pharmaceutically acceptable salt thereof:

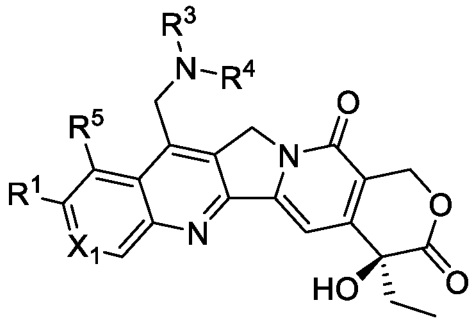

(I)

wherein

$R^1$ is selected from halogen, CN, $C_1$-$C_6$ alkyl, $C_3$-$C_6$ cycloalkyl or $C_2$-$C_6$ alkynyl, and the $C_1$-$C_6$ alkyl, $C_3$-$C_6$ cycloalkyl or $C_2$-$C_6$ alkynyl is optionally substituted with $R^{a1}$,
$X_1$ is selected from $CR^2$ or N;
$R^2$ is selected from H, halogen or CN, or $R^1$ and $R^2$ together with the atoms to which they are attached form a 5- to 6-membered heterocyclyl, and the 5- to 6-membered heterocyclyl is optionally substituted with $R^{a2}$;
$R^5$ is selected from H, halogen, CN, $NH_2$ or $NO_2$, or $R^1$ and $R^5$ together with the atoms to which they are attached form a 5- to 6-membered heterocyclyl, a 5- to 6-membered heteroaryl or $C_5$-$C_7$ cycloalkenyl, and the 5- to 6-membered heterocyclyl, 5-to 6-membered heteroaryl or $C_5$-$C_7$ cycloalkenyl is optionally substituted with $R^{a5}$;
$R^3$ is selected from H,

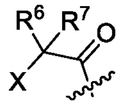 , 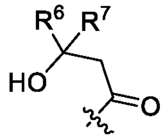 or 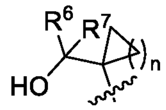 ,

X is selected from $NH_2$ or OH, $R^6$ is selected from H or $C_1$-$C_3$ alkyl, $R^7$ is selected from H, $C_1$-$C_3$ alkyl or $C_3$-$C_6$ cycloalkyl, or $R^6$ and $R^7$ together with the C atom to which they are attached form $C_3$-$C_6$ cycloalkyl, and the $C_3$-$C_6$ cycloalkyl is optionally substituted with $R^{a3}$,
n is selected from 1, 2, 3 or 4;
$R^4$ is selected from H, or $R^4$ and $R^7$ together with the atoms to which they are attached form a 5- to 6-membered heterocyclyl, and the 5- to 6-membered heterocyclyl is optionally substituted with $R^{a4}$;
each of $R^{a1}$, $R^{a2}$, $R^{a3}$, $R^{a4}$, and $R^{a5}$ is independently selected from D, halogen, CN, =O, OH, $NH_2$, $C_1$-$C_3$ alkyl, $C_3$-$C_6$ cycloalkyl or 4- to 7-membered heterocyclyl, and the OH, $NH_2$, $C_1$-$C_3$ alkyl, $C_3$-$C_6$ cycloalkyl or 4- to 7-membered heterocyclyl is optionally substituted with $R^b$;
each $R^b$ is independently selected from halogen, CN, =O, $C_1$-$C_3$ alkyl, OH, O($C_1$-$C_3$ alkyl), $NH_2$, NH($C_1$-$C_3$ alkyl) or N($C_1$-$C_3$ alkyl)$_2$;
provided that: i) when $R^1$ is selected from methyl and $R^2$ is selected from F, $R^3$ is selected from

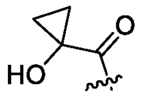 , 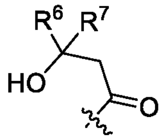 or 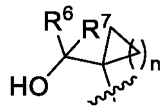 ,

wherein $R^6$ is selected from H or $C_1$-$C_3$ alkyl, $R^7$ is selected from H, $C_1$-$C_3$ alkyl or $C_3$-$C_6$ cycloalkyl, and n is selected from 1, 2, 3 or 4; ii) when X is selected from $NH_2$, $R^5$ is not selected from H; and iii) the compound of formula (I) does not comprise the following compounds:

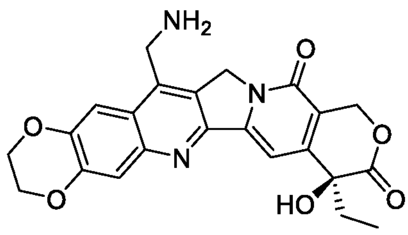,
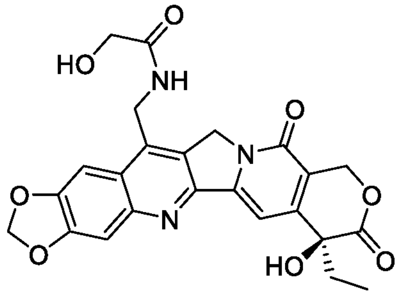,

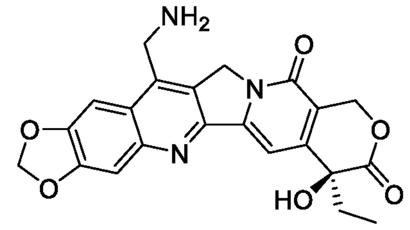.

2. The compound of formula (I), or the stereoisomer or pharmaceutically acceptable salt thereof according to claim 1, wherein, $R^1$ is selected from halogen, $C_1$-$C_3$ alkyl, $C_3$-$C_6$ cycloalkyl or $C_2$-$C_3$ alkynyl; or
$R^1$ is selected from Cl, Br, methyl, cyclopropyl or ethynyl.

3. The compound of formula (I), or the stereoisomer or pharmaceutically acceptable salt thereof according to claim 1 or 2, wherein, $R^2$ is selected from H, halogen or CN, or $R^1$ and $R^2$ together with the atoms to which they are attached form a 5- to 6-membered heterocyclyl, the 5- to 6-membered heterocyclyl contains 1 or 2 oxygen atoms as ring atoms, and the 5- to 6-membered heterocyclyl is optionally substituted with D atom; or
$R^2$ is selected from H or halogen, or $R^1$ and $R^2$ together with the atoms to which they are attached form a 5- to 6-membered heterocyclyl, the 5- to 6-membered heterocyclyl contains 1 or 2 oxygen atoms as ring atoms, and the 5- to 6-membered heterocyclyl is optionally substituted with D atom.

4. The compound of formula (I), or the stereoisomer or pharmaceutically acceptable salt thereof according to any one of claims 1 to 3, wherein, $R^2$ is selected from H, F or Cl, or $R^1$ and $R^2$ together with the atoms to which they are attached form

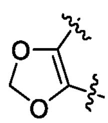

, 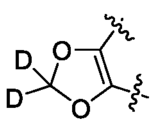 or 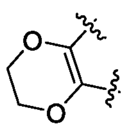.

5. The compound of formula (I), or the stereoisomer or pharmaceutically acceptable salt thereof according to any one of claims 1 to 4, wherein, $R^3$ is selected from

H, 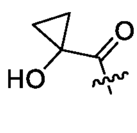, 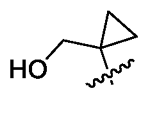, 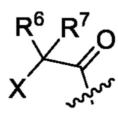 or 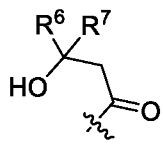,

wherein X is selected from $NH_2$ or

OH, $R^6$ is selected from H or methyl, and $R^7$ is selected from H, methyl, isopropyl, or cyclopropyl optionally substituted with D atom; $R^4$ is selected from H, or $R^4$ and $R^7$ together with the atoms to which they are each attached form a 5-membered heterocyclyl; or

$R^3$ is selected from

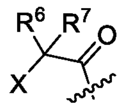 or 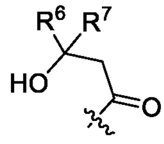,

X is selected from $NH_2$ or OH, $R^6$ is selected from H or methyl, and $R^7$ is selected from H, methyl, isopropyl, or cyclopropyl optionally substituted with D atom; or
$R^3$ is selected from

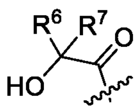 or 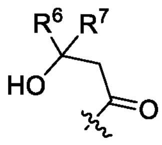,

$R^6$ is selected from H, $R^7$ is selected from H or cyclopropyl, or $R^6$ and $R^7$ together with the C atom to which they are attached form $C_3$-$C_6$ cycloalkyl.

**6.** The compound of formula (I), or the stereoisomer or pharmaceutically acceptable salt thereof according to any one of claims 1 to 4, wherein,

$R^1$ and $R^2$ together with the atom to which they are each attached form

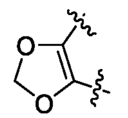

or

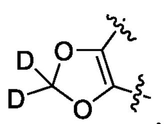,

$R^3$ is selected from H,

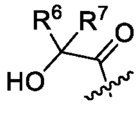 or 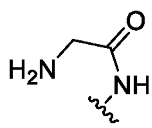,

$R^4$ is selected from H, $R^6$ is selected from H, $R^7$ is selected from H, or cyclopropyl optionally substituted with D atom, or $R^6$ and $R^7$ together with the C atom to which they are attached form $C_3$-$C_6$ cycloalkyl; or
$R^1$ and $R^2$ together with the atoms to which they are each attached form

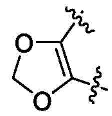

or

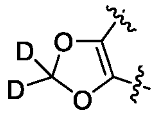,

$R^3$ is selected from

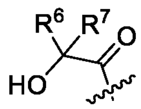,

$R^4$ is selected from H, $R^6$ is selected from H, $R^7$ is selected from cyclopropyl optionally substituted with D atom, or $R^6$ and $R^7$ together with the C atom to which they are attached form cyclopropyl.

**7.** The compound of formula (I), or the stereoisomer or pharmaceutically acceptable salt thereof according to any one of claims 1 to 4, wherein,

$R^1$ and $R^2$ together with the atoms to which they are each attached form

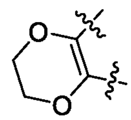,

$R^3$ is selected from H,

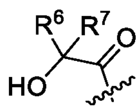 or 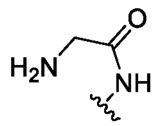,

$R^4$ is selected from H, $R^6$ is selected from H, $R^7$ is selected from H or cyclopropyl, or $R^6$ and $R^7$ together with the C atom to which they are attached form $C_3$-$C_6$ cycloalkyl; or
$R^1$ and $R^2$ together with the atoms to which they are attached form

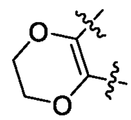,

$R^3$ is selected from

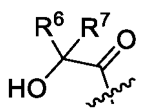,

$R^4$ is selected from H, $R^6$ is selected from H, $R^7$ is selected from H or cyclopropyl, or $R^6$ and $R^7$ together with the C atom to which they are attached form $C_3$-$C_6$ cycloalkyl.

**8.** The compound of formula (I), or the stereoisomer or pharmaceutically acceptable salt thereof according to any one of claims 1 to 7, wherein, $R^5$ is selected from H, halogen, $NH_2$ or $NO_2$, or $R^1$ and $R^5$ together with the atoms to which they are attached form a 5- to 6-membered heteroaryl or $C_5$-$C_6$ cycloalkenyl, and the 5- to 6-membered heteroaryl or $C_5$-$C_6$ cycloalkenyl is optionally substituted with $R^{a5}$; or
$R^5$ is selected from H, Cl, F, $NH_2$ or $NO_2$, or $R^1$ and $R^5$ together with the atoms to which they are attached form

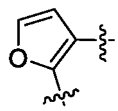 or 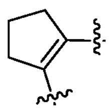 .

**9.** The compound of formula (I), or the stereoisomer or pharmaceutically acceptable salt thereof according to any one of claims 1 to 4 and 8, wherein, the structural unit

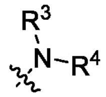

is selected from

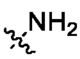, 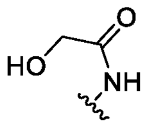, 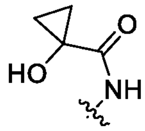, 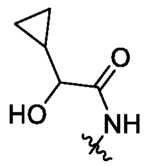, 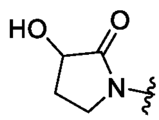

, 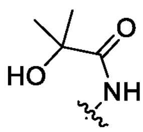, 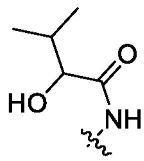, 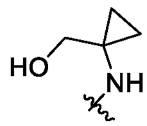, 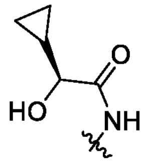, 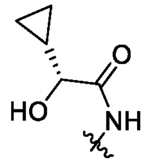, 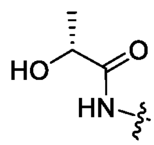,

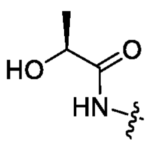, 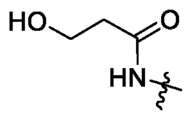, 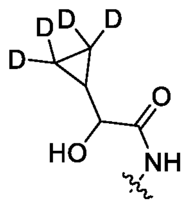 or 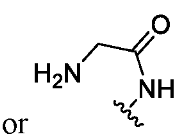 .

**10.** The compound of formula (I), or the stereoisomer or pharmaceutically acceptable salt thereof according to any one of claims 1 to 9, wherein, the compound of formula (I), or the stereoisomer or pharmaceutically acceptable salt thereof is selected from a compound of formula (Ia), or a stereoisomer or pharmaceutically acceptable salt thereof:

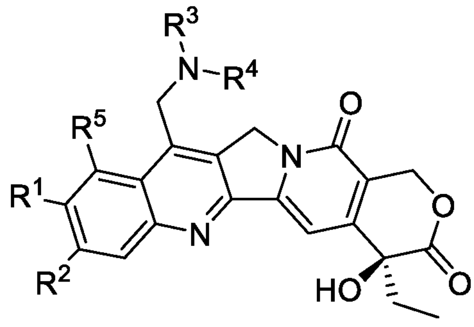

(Ia)

wherein $R^1$, $R^2$, $R^3$, $R^4$ and $R^5$ are as defined in claims 1 to 9.

**11.** The compound of formula (I), or the stereoisomer or pharmaceutically acceptable salt thereof according to any one of claims 1-2, 5 and 8-9, wherein, the compound of formula (I), or the stereoisomer or pharmaceutically acceptable salt thereof is selected from a compound of formula (Ib), or a stereoisomer or pharmaceutically acceptable salt thereof:

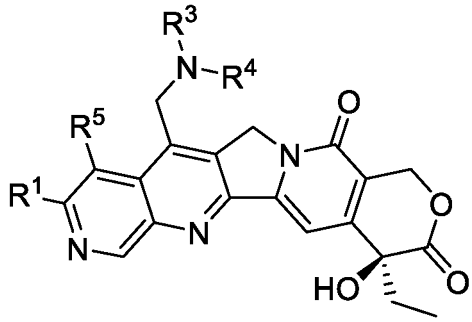

(Ib)

wherein $R^1$, $R^3$, $R^4$ and $R^5$ are as defined in claims 1-2, 5 and 8-9.

**12.** A compound of formula (II), or a stereoisomer or pharmaceutically acceptable salt thereof:

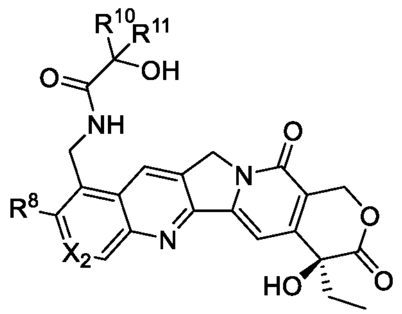

(II)

wherein

$R^8$ is selected from hydroxyl, halogen, CN, $C_1$-$C_6$ alkyl, $C_3$-$C_6$ cycloalkyl or $C_2$-$C_6$ alkynyl;
$X_2$ is selected from $CR^9$ or N;
$R^9$ is selected from H, halogen or CN, or $R^8$ and $R^9$ together with the atoms to which they are each attached form a 5- to 6-membered heterocyclyl;
$R^{10}$ and $R^{11}$ are independently selected from H, $C_3$-$C_6$ cycloalkyl, or $R^{10}$ and $R^{11}$ together with the C atom to

which they are attached form $C_3$-$C_6$ cycloalkyl.

**13.** A compound, or a stereoisomer or pharmaceutically acceptable salt thereof, wherein the compound has a structure selected from one of the following:

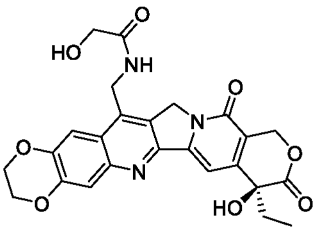

1

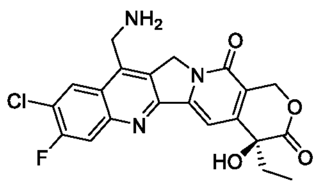

2

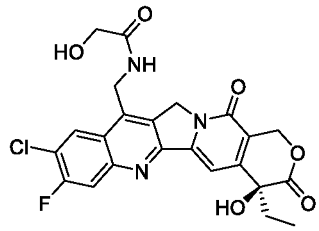

3

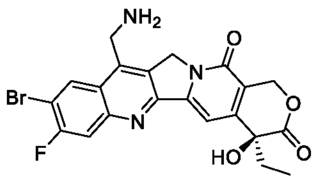

4

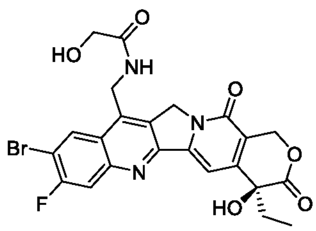

5

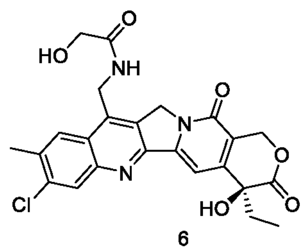

6

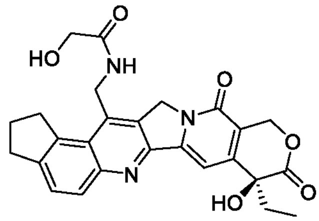

7

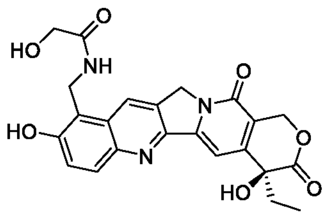

8

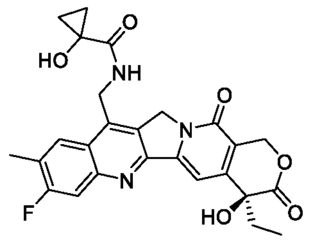

9

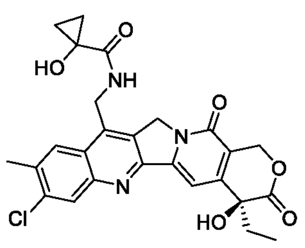

10

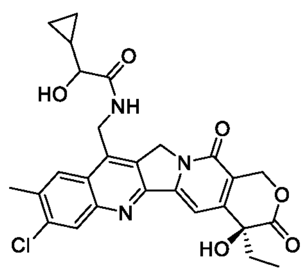

11

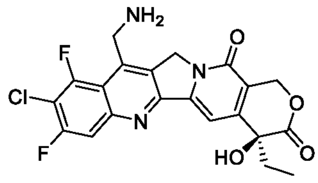

12

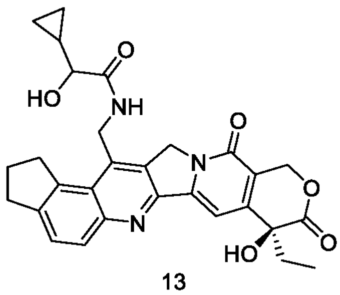

13

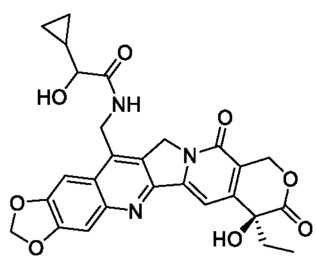

14

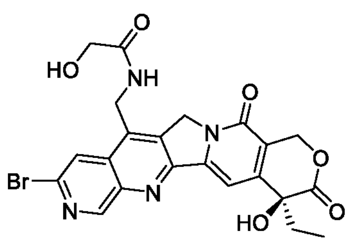

15

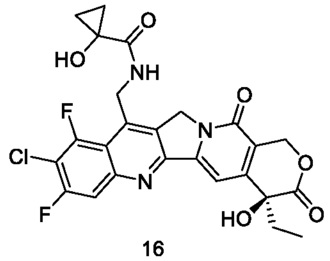
16

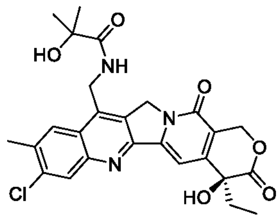
17

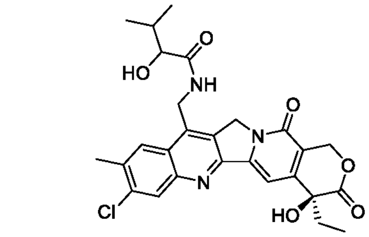
18

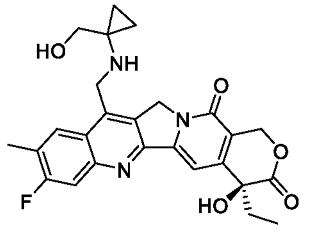
19

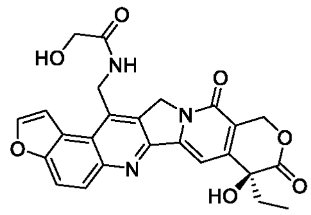
20

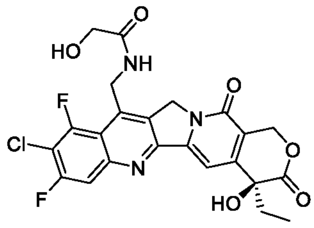
21

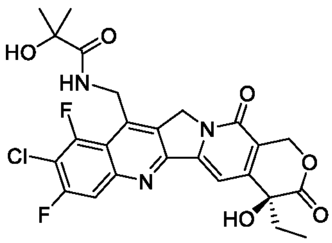
22

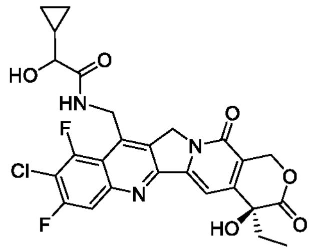
23

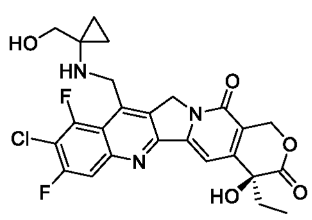
24

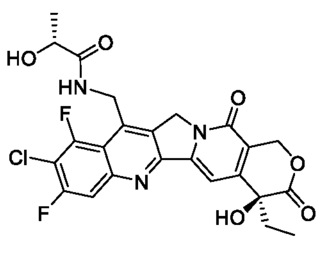
25

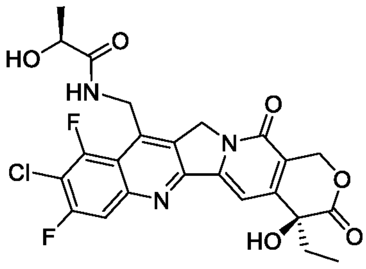
26

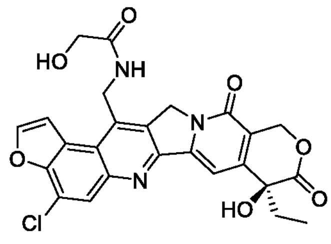
27

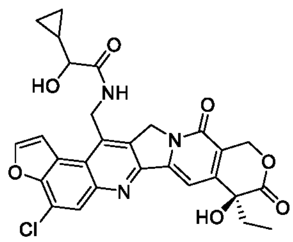
28

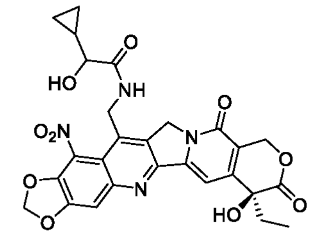
29

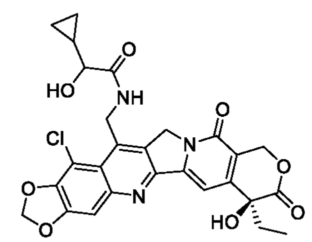
30

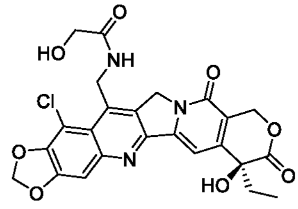

31

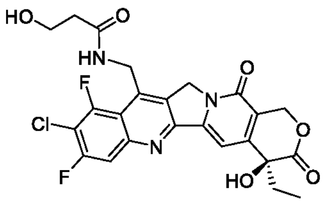

32

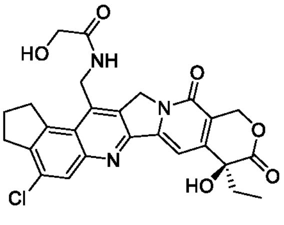

33

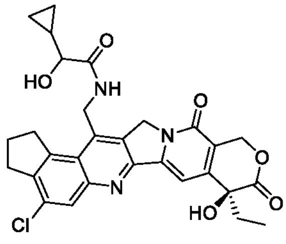

34

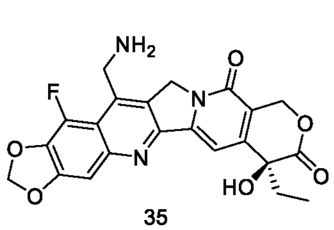

35

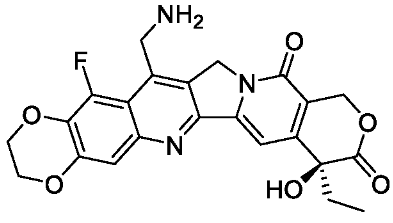

36

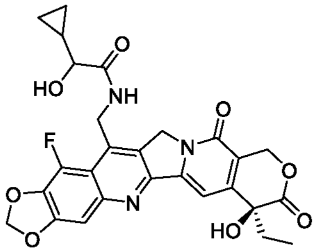

37

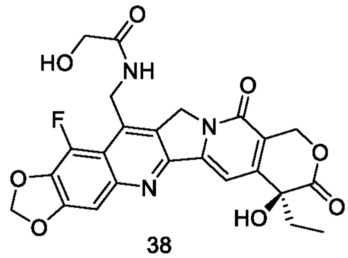

38

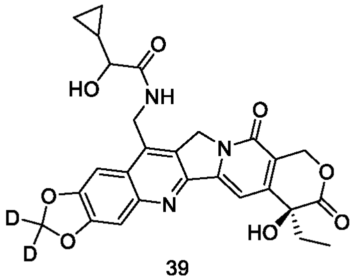

39

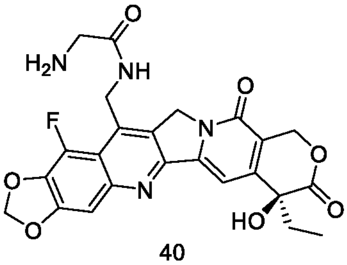

40

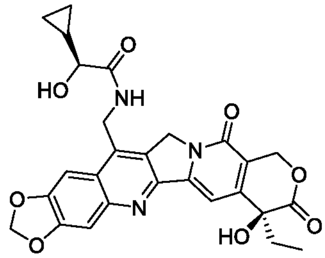

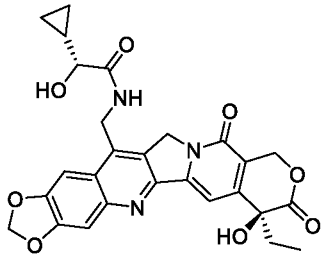

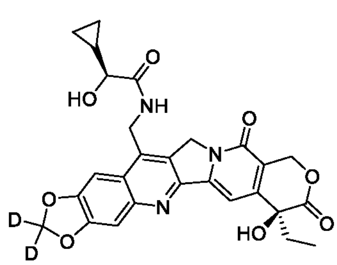

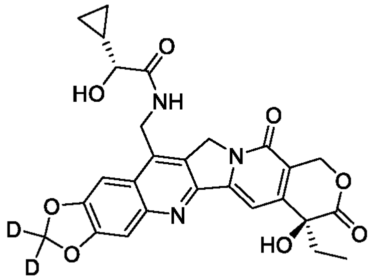

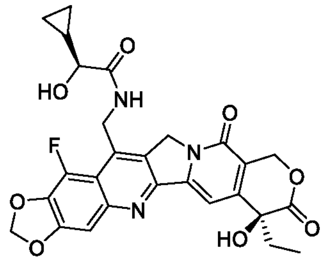

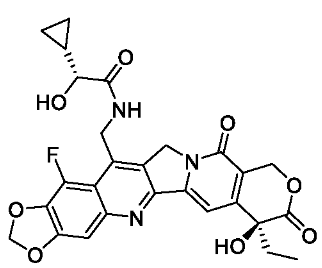
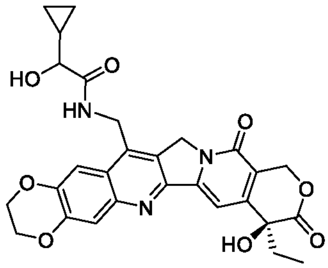
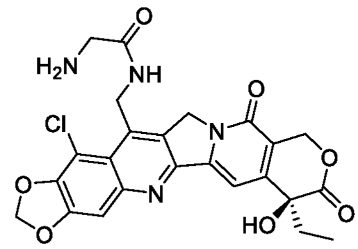
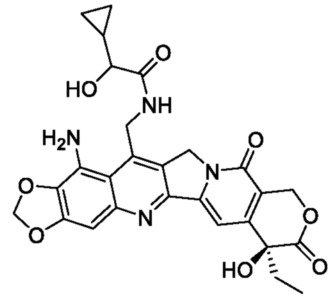
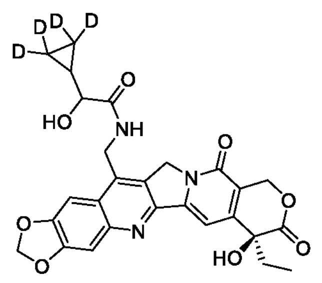
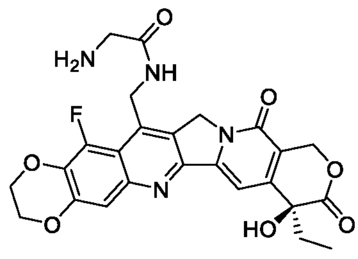
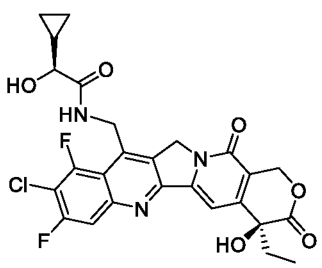
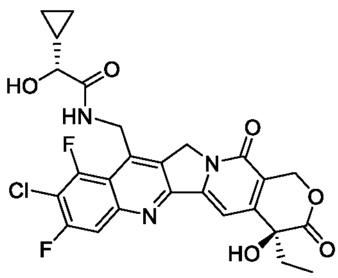
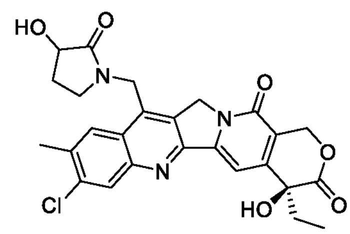
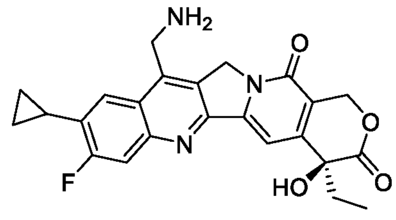
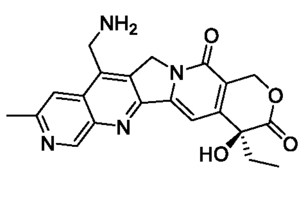
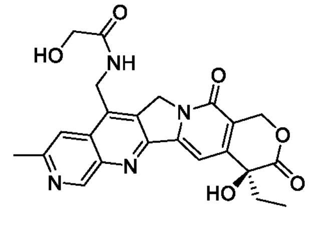
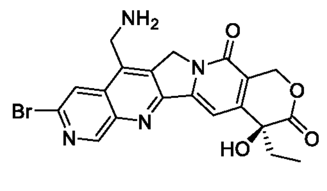
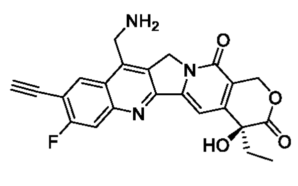
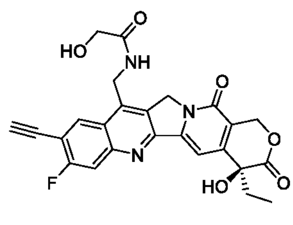
or
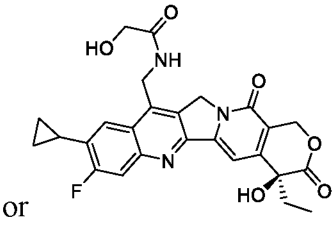
.

**14.** A pharmaceutical composition, comprising the compound, or the stereoisomer or pharmaceutically acceptable salt thereof according to any one of claims 1 to 13, and a pharmaceutically acceptable adjuvant.

15. Use of the compound, or the stereoisomer or pharmaceutically acceptable salt thereof according to any one of claims 1 to 13, or the pharmaceutical composition according to claim 14, in the preparation of an anti-tumor drug.

## INTERNATIONAL SEARCH REPORT

International application No.
**PCT/CN2022/113499**

**A. CLASSIFICATION OF SUBJECT MATTER**

C07D 491/147(2006.01)i; C07D 491/22(2006.01)i; A61K 31/436(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI; SIPOABS; CNKI; CNABS; STNext; Web of Science: 江苏先声药业有限公司, 喜树碱, 肿瘤, 癌症, camptothecin, tumor, cancer, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2022166762 A1 (SICHUAN KELUN-BIOTECH BIOPHARMACEUTICA CO., LTD.) 11 August 2022 (2022-08-11)<br>description, abstract, and claims 1-14 | 1-15 |
| PX | WO 2022170971 A1 (MEDILINK THERAPEUTICS (SUZHOU) CO., LTD.) 18 August 2022 (2022-08-18)<br>description, abstract, and description, pages 82-83 and 89 | 1-15 |
| X | WO 2020219287 A1 (IMMUNOGEN INC.) 29 October 2020 (2020-10-29)<br>claims 1, 25 and 114-117, and description, table 1a | 1-15 |
| X | CN 1074908 A (GLAXO INC.) 04 August 1993 (1993-08-04)<br>description, abstract, and claims 1-20 | 1, 3-5, 7-10, 13-15 |
| X | JP H11-71280 A (TANABE SEIYAKU CO.) 16 March 1999 (1999-03-16)<br>description, abstract, and claims 1-15, manufacturing example 1 | 1, 3-5, 7-10, 13-15 |
| X | CN 1072683 A (GLAXO INC.) 02 June 1993 (1993-06-02)<br>description, abstract, and claims 1-12 | 1, 3-5, 7-10, 13-15 |
| X | WO 2019236954 A1 (SEATTLE GENETICS INC.) 12 December 2019 (2019-12-12)<br>description, embodiment 6 | 1, 3-5, 7-10, 13-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 November 2022** | **17 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/113499**

**C. DOCUMENTS CONSIDERED TO BE RELEVANT**

| Category* | Citation of document, with indication, where appropriate, of the relevant passages | Relevant to claim No. |
|---|---|---|
| X | WO 2019195665 A1 (SEATTLE GENETICS INC.) 10 October 2019 (2019-10-10)<br>description, embodiment 4 | 1, 3-5, 7-10, 13-15 |

Form PCT/ISA/210 (second sheet) (January 2015)

**INTERNATIONAL SEARCH REPORT**
**Information on patent family members**

International application No.
**PCT/CN2022/113499**

| Patent document cited in search report | Publication date (day/month/year) | Patent family member(s) | Publication date (day/month/year) |
|---|---|---|---|
| WO 2022166762 A1 | 11 August 2022 | None | |
| WO 2022170971 A1 | 18 August 2022 | None | |
| WO 2020219287 A1 | 29 October 2020 | TW 202106691 A | 16 February 2021 |
| | | AU 2020263231 A1 | 18 November 2021 |
| | | JP 2022529854 A | 24 June 2022 |
| | | US 2021077482 A1 | 18 March 2021 |
| | | KR 20220027828 A | 08 March 2022 |
| | | SG 11202110922 Q A | 28 October 2021 |
| | | CA 3137125 A1 | 29 October 2020 |
| | | US 2022133711 A1 | 05 May 2022 |
| | | EP 3958977 A1 | 02 March 2022 |
| | | IL 286846 A | 31 October 2021 |
| | | CN 113766954 A | 07 December 2021 |
| CN 1074908 A | 04 August 1993 | None | |
| JP 特开平11-71280 A | 16 March 1999 | JP H1171280 A | 16 March 1999 |
| CN 1072683 A | 02 June 1993 | GR 3020120 T3 | 31 August 1996 |
| | | TW 221994 B | 01 April 1994 |
| | | DK 0540099 T3 | 17 June 1996 |
| | | HU 9203384 D0 | 28 January 1993 |
| | | DE 69209969 D1 | 23 May 1996 |
| | | RU 2119921 C1 | 10 October 1998 |
| | | FI 924878 A0 | 28 October 1992 |
| | | MX 9206211 A | 01 May 1993 |
| | | FI 982185 A0 | 08 October 1998 |
| | | CA 2081580 A1 | 30 April 1993 |
| | | EP 0540099 A1 | 05 May 1993 |
| | | ES 2086643 T3 | 01 July 1996 |
| | | NZ 244914 A | 27 January 1995 |
| | | MY 108261 A | 30 September 1996 |
| | | NO 924158 D0 | 28 October 1992 |
| | | JP H05222048 A | 31 August 1993 |
| | | AP 9200439 A0 | 31 October 1992 |
| | | AU 2738592 A | 06 May 1993 |
| | | FI 20021090 A | 07 June 2002 |
| | | KR 100246154 B1 | 01 April 2000 |
| | | IL 103571 D0 | 15 March 1993 |
| | | OA 9767 A | 30 November 1993 |
| | | AT 136898 T | 15 May 1996 |
| | | SK 279298 B6 | 09 September 1998 |
| | | IS 3936 A | 30 April 1993 |
| | | CZ 323792 A3 | 13 October 1993 |
| | | JP 2000109475 A | 18 April 2000 |
| | | HU 211349 A9 | 28 November 1995 |
| WO 2019236954 A1 | 12 December 2019 | JP 2021527040 A | 11 October 2021 |
| | | EP 3801633 A1 | 14 April 2021 |
| | | IL 278990 A | 31 January 2021 |
| | | CN 112512592 A | 16 March 2021 |
| | | CA 3101866 A1 | 12 December 2019 |
| | | AU 2019282767 A1 | 07 January 2021 |

Form PCT/ISA/210 (patent family annex) (January 2015)

**INTERNATIONAL SEARCH REPORT**
**Information on patent family members**

International application No.
**PCT/CN2022/113499**

| Patent document cited in search report | Publication date (day/month/year) | Patent family member(s) | Publication date (day/month/year) |
|---|---|---|---|
| | | TW 202015740 A | 01 May 2020 |
| | | BR 112020024915 A2 | 09 March 2021 |
| | | SG 11202011915 T A | 30 December 2020 |
| | | KR 20210053871 A | 12 May 2021 |
| WO 2019195665 A1 | 10 October 2019 | SG 11202009527 P A | 29 October 2020 |
| | | BR 112020020466 A2 | 12 January 2021 |
| | | TW 202010498 A | 16 March 2020 |
| | | US 2022193069 A1 | 23 June 2022 |
| | | CA 3094313 A1 | 10 October 2019 |
| | | AR 114473 A1 | 09 September 2020 |
| | | AU 2019247434 A1 | 08 October 2020 |
| | | IL 277748 A | 30 November 2020 |
| | | MA 52669 A | 17 February 2021 |
| | | EA 202092410 A1 | 09 February 2021 |
| | | KR 20210006362 A | 18 January 2021 |
| | | EP 3773736 A1 | 17 February 2021 |
| | | JP 2021521111 A | 26 August 2021 |
| | | CN 111936169 A | 13 November 2020 |
| | | US 2019343828 A1 | 14 November 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110955364
- CN 202111515247
- CN 202210515797
- WO 2020219287 A


### Non-patent literature cited in the description


- **MAEHR.** *J.Chem.Ed.,* 1985, vol. 62, 114-120